(19) Europäisches Patentamt
European Patent Office
Office européen des brevets

(11) Veröffentlichungsnummer: **0 310 918 A2**

(12) # EUROPÄISCHE PATENTANMELDUNG

(21) Anmeldenummer: **88115878.6**

(22) Anmeldetag: **27.09.88**

(51) Int. Cl.[4]: **C07D 233/64 , C07K 5/06 , C07K 5/08 , C07K 5/10 , C07C 103/50 , C07C 91/02 , C07C 21/24 , A61K 31/41 , A61K 37/02 , C07D 403/12 , C07D 401/12**

Patentansprüche für folgende Vertragsstaaten: ES + GR.

(30) Priorität: **06.10.87 CH 3903/87**

(43) Veröffentlichungstag der Anmeldung:
**12.04.89 Patentblatt 89/15**

(84) Benannte Vertragsstaaten:
**AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Branca, Quirico, Dr.**
**Lenzgasse 2**
**CH-4056 Basel(CH)**
Erfinder: **Edenhofer, Albrecht, Dr.**
**Rudolf Wackernagelstrasse 35**
**CH-4125 Riehen(CH)**
Erfinder: **Gutknecht, Eva-Maria, Dr.**
**Im Feldele 23**
**D-7845 Buggingen-Seefelden(DE)**
Erfinder: **Neidhart, Werner, Dr.**
**Oltmannstrasse 14**
**D-7800 Freiburg im Breisgau(DE)**
Erfinder: **Ramuz, Henri, Prof. Dr.**
**Rheinparkstrasse 3**
**CH-4127 Birsfelden(CH)**
Erfinder: **Wostl, Wolfgang, Dr.**
**Im Strick 2**
**D-7889 Grenzach-Wyhlen(DE)**

(74) Vertreter: **Kellenberger, Marcus, Dr. et al**
**Grenzacherstrasse 124 Postfach 3255**
**CH-4002 Basel(CH)**

(54) **Peptidartige Aminosäurederivate.**

(57) Verbindung der Formel

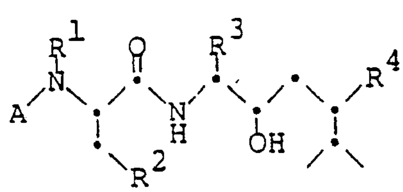

I

EP 0 310 918 A2

worin $R^1$, $R^2$, $R^3$, $R^4$ und A die in Anspruch 1 angegebene Bedeutung haben,
sowie pharmazeutisch verwendbare Salze davon besitzen Renin-inhibierende Eigenschaften und können demnach in Form pharmazeutischer Präparate als Arzneimittel verwendet werden. Sie können nach an sich bekannten Methoden hergestellt werden.

## Peptidartige Aminosäurederivate

Die vorliegende Erfindung betrifft Aminosäurederivate. Im speziellen betrifft sie Aminosäurederivate der allgemeinen Formel

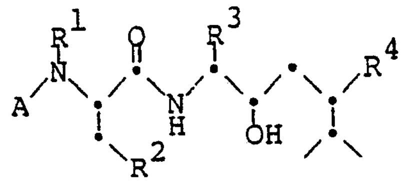

I

worin $R^1$ Wasserstoff oder Methyl, $R^2$ Aethyl, Propyl oder Imidazol-4-yl, $R^3$ Isobutyl, Cyclohexylmethyl oder Benzyl, $R^4$ Phenyl, Furyl, Vinyl, Aethyl oder 1,2-Dihydroxyäthyl und A über die Carboxylgruppe gebundene Dibenzsuberanessigsäure, 2-(2-Pyridyl)benzosäure, $\beta$ -Naphthylbernsteinsäuremonoäthylester, 2-Indolylessigsäure oder Dihydrozimtsäure oder eine der Gruppen

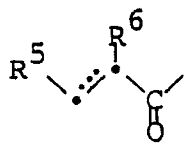

und

-Y-Z

(a) (b)

bedeuten, worin die gestrichelte Linie eine zusätzliche Bindung bedeuten kann, $R^5$ Phenyl, Naphthyl, Indolyl, Pyrazolyl, Imidazolyl oder Pyridylmethyl und $R^6$ Wasserstoff, Alkyl, Arylalkyl, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminocarbonyl, substituiertes Aminocarbonyl, Alkoxycarbonyl, Alkylcarbonyloxy, Arylalkylcarbonyloxy, Alkylaminocarbonyloxy, Alkoxycarbonylamino, Cyan oder 2,5-Dimethylpyrrol-1-yl bedeuten, mit der Massgabe dass $R^4$ nicht Alkoxycarbonylamino bedeuten kann, wenn $R^3$ Phenyl oder $\alpha$-Naphthyl bedeutet, und Y den N-terminal mit Z verbundenen, bivalenten Rest von gegebenenfalls N- und/oder $\alpha$-methyliertem Phenylglycin, Cyclohexylglycin, Phenylalanin, Cyclohexylalanin, 4-Fluorphenylalanin, 4-Chlorphenylalanin, Tyrosin, $\alpha$-Naphthylalanin, Homophenylalanin, Asparaginsäureäthylester, Glutaminsäure-t-butylester oder Glutaminsäurebenzylester und Z Wasserstoff, Acyl oder eine der Gruppen

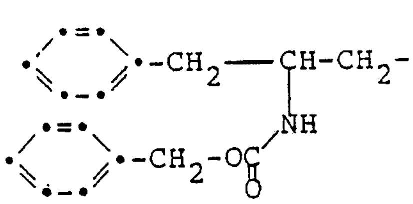

,

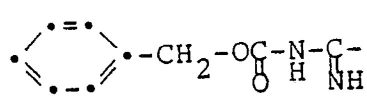

und

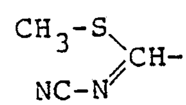

(c) (d) (e)

bedeuten,

in Form von optisch reinen Diastereomeren, Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die

Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Der in der vorliegenden Beschreibung verwendete Ausdruck "Alkyl" - allein oder in Kombination - bedeutet geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Isobutoxy, sec.-Butoxy, t-Butoxy und dergleichen. Der Ausdruck "Cycloalkyl" bedeutet gesättigte, cyclische Kohlenwasserstoffreste mit 3-8, vorzugsweise 3-6, Kohlenstoffatomen, wie Cyclopropyl, Cyclobutyl, Cyclopentyl, Cyclohexyl und dergleichen. Der Ausdruck "Heterocycloalkyl" betrifft in ähnlicher Weise gesättigte , 3-8-gliedrige, vorzugsweise 5- oder 6-gliedrige, cyclische Kohlenwasserstoffreste, in welchen eine oder zwei Methylengruppen durch ein oder zwei Sauerstoff- , Schwefel- oder gegebenenfalls durch Alkyl, Phenylalkyl, Alkanoyl oder Alkanoyloxy substi tuierte Stickstoffatome ersetzt sind, wie Piperidinyl, Pyrazinyl, N-Benzylpyrazinyl, Morpholinyl, N-Methylpiperidinyl, N-Benzylmorpholinyl und dergleichen. Der Ausdruck "Aryl" bezeichnet einen gegebenenfalls durch Alkyl, Alkoxy, Alkanoyloxy, Amino, Alkylamino, Dialkylamino, Alkanoylamino, Hydroxy, Halogen, Trifluormethyl oder Nitro ein- oder mehrfach substituierten mono- oder bicyclischen aromatischen Kohlenwasserstoffrest mit 6-14 Kohlenstoffatomen, wie Phenyl, α- oder β-Naphthyl, Indenyl, Anthryl oder Phenanthryl und dergleichen. Der Ausdruck "Arylalkyl" bezeichnet geradkettige oder verzweigte Alkylgruppen, worin ein oder mehrere Wasserstoffatome durch Arylgruppen ersetzt sind, wie Benzyl, Diphenylmethyl, Trityl, α- oder β-Naphthylmethyl, 2-Phenyläthyl, 3-Phenyl-2-propyl, 4-Phenyl-3-butyl, 2-(α- oder β-Naphthyl)äthyl, 3-α-Naphthyl-2-propyl, 4-α-Naphthyl-3-butyl und dergleichen, wobei der aromatische Rest jeweils wie oben angegeben ein- oder mehrfach substituiert sein kann. Der Ausdruck "substituiertes Amino" bedeutet eine durch Alkyl, Arylalkyl, Alkanoyl, Alkoxycarbonyl oder Arylalkoxycarbonyl mono- oder disubstituierte oder eine gegebenenfalls durch ein Sauerstoff-, Schwefel-oder gegebenenfalls Alkyl-, Phenylalkyl-, Alkanoyl- oder Alkanoyloxy-substituiertes Stickstoffatom unterbrochene $C_3$-$C_6$-Alkylen disubstituierte Aminogruppe. Der Ausdruck "Acyl" betrifft die Acylgruppe einer Carbonsäure, eines Halbesters der Kohlensäure, einer gegebenenfalls N-substituierten Carbamin- oder Thiocarbaminsäure, eines gegebenenfalls N-substituierten Oxalamids, einer Sulfonsäure oder einer gegebenenfalls N-substituierten Amidosulfonsäure, insbesondere solche mit den Teilformeln $R^b$-CO-, $R^a$-O--CO-, ($R^b$) ($R^b$)N-CO-, ($R^b$)($R^b$)N-CS-, ($R^b$)-($R^b$)N--CO-CO-, $R^b$-$SO_2$-, oder ($R^b$)($R^b$)N-$SO_2$-, worin $R^a$ einen unsubstituierten oder substituierten, gesättigten oder ungesättigten, aliphatischen, cycloaliphatischen, cycloaliphatisch-aliphatischen Kohlenwasserstoffrest mit bis zu 18, vorzugsweise 10, Kohlenstoffatomen, einen unsubstituierten oder substituierten aromatischen, heteroaromatischen, aroma tisch-aliphatischen oder heteroaromatisch-aliphatischen Kohlenwasserstoffrest mit bis zu 18, vorzugsweise 10, Kohlenstoffatomen oder einen unsubstituierten oder substituierten, gesättigten 5- oder 6-gliedrigen Heterocyclus bedeutet und $R^b$ Wasserstoff bedeutet oder die Bedeutung von $R^a$ besitzt. Der Ausdruck "Acyl" betrifft auch den einwertigen, über die Carboxylgruppe gebundenen Rest einer Aminosäure, eines Dipeptids oder eines Tripeptids.

Ein unsubstituierter oder substituierter, gesättigter oder ungesättigter, aliphatischer, cycloaliphatischer oder cycloaliphatisch-aliphatischer Kohlenwasserstoffrest $R^a$ oder $R^b$ ist beispielsweise unsubstituiertes oder substituiertes Alkyl, Alkenyl, Alkinyl, Mono-, Bi- oder Tricycloalkyl, Monocycloalkenyl, Bicycloalkenyl, Cycloalkylalkyl, Cycloalkylalkenyl oder Cycloalkenylalkyl. "Substituiertes Alkyl" bedeutet einen Alkylrest, in welchem ein oder mehrere Wasserstoffatome durch Hydroxy, Alkoxy, Aryloxy, Alkanoyloxy, Halogen, Hydroxysulfonyloxy, Carboxy, Alkoxycarbonyl, Carbamoyl, Alkylcarbamoyl, Dialkylcarbamoyl, Cyan, Phosphono, verestertes Phosphono, Amino oder Oxo substituiert sein können, wobei die Substituenten nur dann in 1-Stellung des Alkylrestes stehen, wenn dieser in der Teilformel $R^b$-CO- an die Carbonylgruppe gebunden ist.

Beispiele von substituiertem Alkyl sind 2-Hydroxyäthyl, Methoxymethyl, 2-Methoxyäthyl, Phenoxymethyl, α- oder β-Naphthoxymethyl, Acetoxymethyl, 2-Acetoxyäthyl, Chlormethyl, Brommethyl, 2-Chlor- oder 2-Bromäthyl, Hydroxysulfonyloxymethyl, 2-Hydroxysulfonyloxyäthyl, Carboxymethyl, 2-Carboxyäthyl, methoxycarbonylmethyl, 2-Methoxycarbonyläthyl, Aethoxycarbonylmethyl, 2-Aethoxycarbonyläthyl, Carbamoylmethyl, 2-Carbamoyläthyl, Methylcarbamoylmethyl, Dimethylcarbamoylmethyl, Cyanomethyl, 2-Cyanoäthyl, 2-Oxopropyl, 2-Oxobutyl, Hydroxycarboxymethyl, 1-Hydroxy-2-carboxyäthyl, Hydroxyäthoxycarbonyläthyl, Hydroxymethoxycarbonyläthyl, Acetoxymethoxycarbonylmethyl, 1,2-Dihydroxy-2-car boxyäthyl, 1,2-Dihydroxy-2-äthoxycarbonyläthyl, 1,2-Dihydroxy-2-methoxycarbonyläthyl, 1,2-Diacetoxy-2-äthoxycarbonyläthyl, 1,2-Diacetoxy-2-methoxycarbonyläthyl, 1-α-Naphthoxy-3-carboxypropyl, 1-α-Naphthoxy-2-äthoxycarbonyläthyl, 1-α-Naphthoxy-3-t-butyoxycarbonylpropyl, 1-α-Naphthoxy-2-benzyloxycarbonyläthyl, 1-α-Naphthoxy-3-carbamoylpropyl, α-Naphthoxycyanomethyl, 1-α-Naphthoxy-3-cyanopropyl, 1-α-Naphthoxy-4-

dimethylaminobutyl oder 1-α-Naphthoxy-3-oxobutyl.

Der Ausdruck "Alkenyl" betrifft geradkettige oder verzweigte, ungesättigte Kohlenwasserstoffreste mit 2-8, vorzugsweise 2-4, Kohlenstoffatomen, wobei die Doppelbindung nur dann in 1-Stellung des Alkenylrestes steht, wenn dieser in der Teilformel $R^b$-CO- an die Carbonylgruppe gebunden ist. Beispiele solcher Alkenylreste sind Vinyl, Allyl, 2-Butenyl oder 3-Butenyl. Die Alkenylreste können durch die gleichen Substituenten substituiert ein wie die Alkylreste.

Der Ausdruck "Akinyl" betrifft Kohlenwasserstoffreste mit 2-8, vorzugsweise 2-4, Kohlenstoffatomen, welche eine Dreifachbindung enthalten, wie Aethinyl, 1-Propinyl oder 2-Propinyl. Der Ausdruck "Bicycloalkyl" betrifft bicyclische gesättigte Kohlenwasserstoffreste mit 5-10, vorzugsweise 6-9, Kohlenstoffatomen, wie Bicyclo[3.1.0]hex-1-yl, Bicyclo[3.1.0]hex-2-yl, Bicyclo[3.1.0]hex-3-yl, Bicyclo[4.1.0]hept-1-yl, Bicyclo[4.1.0]hept-4-yl, Bicyclo[2.2.1]hept-2-yl, Bicyclo[3.2.1]oct-2-yl, Bicyclo[3.3.0]oct-3-yl, Bicyclo[3.3.1]-non-9-yl, α- oder β-Decahydronaphthyl und dergleichen.

Der Ausdruck "Tricycloalkyl" betrifft einen tricyclischen gesättigten Kohlenwasserstoffrest mit 8-10 Kohlenstoffatomen, wie 1-Adamantyl.

Der Ausdruck "Cycloalkenyl" betrifft einen ungesättigten cyclischen Kohlenwasserstoffrest mit 3-8, vorzugsweise 3-6, Kohlenstoffatomen, wie 1-Cyclohexenyl, 1,4-Cyclohexadienyl und dergleichen.

Der Ausdruck "Bicycloalkenyl" betrifft bicyclischen ungesättigten Kohlenwasserstoffrest mit 5-10, vorzugsweise 7-10, Kohlenstoffatomen, wie 5-Norbornen-2-yl, Bicyclo[2.2.2]octen-2-yl, Hexahydro-4,7-methanoind-1-en-6-yl und dergleichen.

Beispiele für Cycloalkylalkyl sind Cyclopropylmethyl, Cyclobutylmethyl, Cyclopentylmethyl, Cyclohexylmethyl und dergleichen. Als Beispiele für Cycloalkylalkenyl können Cyclohexylvinyl und Cyclohexylallyl und dergleichen genannt werden. Beispiele für Cycloalkenylalkyl sind 1-Cyclohexenylmethyl, 1,4-Cyclohexadienylmethyl und dergleichen.

Die genannten cycloaliphatischen und cycloaliphatisch-aliphatischen Reste können durch die gleichen Substituenten wie Alkyl substituiert sein .

Ein gegebenenfalls substituierter aromatischer oder aromatisch-aliphatischer Kohlenwasserstoffrest ist beispielsweise unsubstituiertes oder substituiertes Aryl, Arylalkyl oder Arylalkenyl. Beispiele für Arylalkenyl sind Styryl, 3-Phenylallyl, 2-(α-Naphthyl)vinyl, 2-(β-Naphthyl)vinyl und dergleichen.

In einem heteroaromatischen oder heteroaromatisch-aliphatischen Kohlenwasserstoffrest ist der Heterocyclus mono-, bi- oder tricyclisch und enthält ein bis zwei Stickstoffatome und/oder ein Sauerstoff- oder Schwefelatom und ist mit einem seiner Ring-Kohlenstoffatome mit der Gruppe -CO-, -O-CO-, >N-CO-, >N-CS-, >N-CO-CO-, $-SO_2$ oder >N-$SO_2$-verknüpft. Beispiele solcher heteroaromatischer Kohlenwasserstoffreste sind Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, -Carbolinyl oder ein benzanneliertes, cyclopenta-, cyclohexa- oder cyclohepta-änneliertes Derivat dieser Reste. Der heteroaromatische Rest kann an einem Stickstoffatom durch Alkyl, Phenyl oder Phenylalkyl, z.B. Benzyl und/oder an einem oder mehreren Kohlenstoffatomen durch Alkyl, Phenyl, Phenylalkyl, Halogen, Hydroxy, Alkoxy, Phenylalkoxy oder Oxo substituiert und teilweise gesättigt sein. Beispiele solcher heteroaromatischer Reste sind 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, -Carbolin-3-yl und dergleichen.

Beispiele heteroaromatisch-aliphatischer Kohlenwasserstoffreste sind 2- oder 3-Pyrrolylmethyl, 2-, 3- oder 4-Pyridylmethyl, 2-(2-, 3- oder 4-Pyridyl)äthyl, 4-Imidazolylmethyl, 2-(4-Imidazolyl)äthyl, 2-Indolylmethyl, 3-Indolylmethyl, 2-(3-Indolyl)äthyl, 2-Chinolylmethyl und dergleichen.

Ein gesättigter 5- oder 6-gliedriger Heterocyclus hat mindestens ein Kohlenstoffatom, 1-3 Stickstoffatome und gegebenenfalls ein Sauerstoff- oder Schwefelatom als Ringglieder und ist mit einem seiner Ringkohlenstoffatome mit der Gruppe -CO- bzw. -O-CO-, N-CO-, N-CS-, N-CO-CO-, $-SO_2$- oder N-$SO_2$-verknüpft. Der Heterocyclus kann an einem seiner Kohlenstoffatome oder an einem Ringstickstoffatom durch Alkyl, z.B. Methyl oder Aethyl, Phenyl oder Phenylalkyl, z.B. Benzyl, oder an einem seiner Kohlenstoff atome durch Hydroxy oder Oxo substituiert und/oder an zwei benachbarten Kohlenstoffatomen benzanneliert sein. Beispiele solcher Heterocyclen sind Pyrrolidin-3-yl, 4-Hydroxypyrrolidin-2-yl, 5-Oxopyrrolidin-2-yl, Piperidin-2-yl, Piperidin-3-yl, 1-Methylpiperidin-2-yl, 1-Methylpiperidin-3-yl, 1-Methylpiperidin-4-yl, Morpholin-2-yl, Morpholin-3-yl, Thiomorpholin-2-yl, Thiomorpholin-3-yl, 1,4-Dimethylpiperazin-2-yl, 2-Indolinyl, 3-Indolinyl, 1,2,3,4-Tetrahydrochinol-2-, -3- oder -4-yl, 1,2,3,4-Tetrahydroisochinol-1-, -3-oder -4-yl, 1-Oxo-1,2,3,4-tetrahydroisochinol-3-yl und dergleichen.

Als Rest einer über die Carboxylgruppe gebundenden Aminosäure kommen natürliche α-Aminosäuren mit der L-Konfiguration, Homologe solcher Aminosäuren, z.B. worin die Aminosäurenseitenkette um eine oder zwei Methylengruppen verlängert oder verkürzt ist und/oder eine Methylgruppe durch Wasserstoff ersetzt ist, substituierte aromatische α-Aminosäuren, z.B. substituiertes Phenylalanin oder Phenylglycin, worin der Substituent Alkyl, z.B. Methyl, Halogen, z.B. Fluor, Chlor, Brom oder Jod, Hydroxy, Alkoxy, z.B. Methoxy, Alkanoyloxy, z.B. Acetoxy, Amino, Alkylamino, z.B. Methylamino, Dialkylamino, z.B. Dimethylamino, Alkanoylamino, z.B. Acetylamino oder Pivaloylamino, Alkoxycarbonylamino, z.B. t-Butoxycarbonylamino, Arylmethoxycarbonylamino, z.B. Benzyloxycarbonylamino und/oder Nitro sein kann und ein- oder mehrfach vorkommt, benzanneliertes Phenylalanin oder Phenylglycin, wie α-Naphthylalanin, oder hydriertes Phenylalanin oder Phenylglycin, wie Cyclohexylalanin oder Cyclohexylglycin, eine 5- oder 6-gliedrige cyclische benzannelierte α-Aminosäure, z.B. Indolin-2-carbonsäure oder 1,2,3,4-Tetrahydroisochinolin-3-carbonsäure, eine natürliche oder homologe α-Aminosäure, in der eine Carboxygruppe in der Seitenkette in veresterter oder amidierter Form vorliegt, z.B. als Alkylestergruppe, wie Methoxycarbonyl oder t-Butoxycarbonyl, oder als Carbamoyl-, Alkylcarbamoyl, wie Methylcarbamoyl, oder als Dialkylcarbamoylgruppe, wie Dimethylcarbamoyl, in der eine Aminogruppe der Seitenkette in acylierter Form vorliegt, z.B. als Alkanyolamino, wie Acetylamino oder Pivaloylamino, als Alkoxycarbonylamino-, wie t-Butoxycarbonylamino, oder als Arylmethoxycarbonylaminogruppe, wie Benzyloxycarbonylamino, oder in der eine Hydroxygruppe der Seitenkette in veräthertem oder veresterter Form vorliegt, z.B. als Alkoxygruppe, wie Methoxy, als Arylalkoxygruppe, wie Benzyloxy, oder als nieder Alkanoyloxygruppe, wie Acetoxy, oder Epimere solcher Aminosäuren, d.h. mit der unnatürlichen D-Konfiguration. Beispiele solcher Aminosäuren sind Glycin, Alanin, Valin, Norvalin, Leucin, Isoleucin, Norleucin, Serin, Homoserin, Threonin, Methionin, Cystein, Prolin, Trans-3- und Trans-4-hydroxyprolin, Phenylalanin, Tyrosin, 4-Nitrophenylalanin, 4-Aminophenylalanin, 4-Chlorphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutaminsäuremono-t-butylester, Glutamin, N-Dimethylglutamin, Histidin, Arginin, Lysin, N-t-Butoxycarbonyllysin, δ-Hydroxylysin, Ornithin, N-Pivaloylornithin, α,γ-Diaminobuttersäure oder α,β-Diaminopropionsäure und dergleichen. Der über die Carboxylgruppe gebundene Rest der Aminosäure kann N-terminal zur Erhöhung der Stabilität der Verbindung der Formel I gegen enzymatischen Abbau durch Alkyl, z.B. Methyl oder Aethyl, substituiert sein.

Der über die Carboxylgruppe gebundene Rest eines Di- oder Tri-peptids besteht aus zwei oder drei der oben erwähnten Aminosäuren.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfon säure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weitere hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie. Dünnschichtchromatographie, HPLC und dergleichen.

Bevorzugt sind solche Verbindungen der Formel I, worin $R^1$ Wasserstoff bedeutet. $R^2$ bedeutet vorzugsweise Imidazol-4-yl. Weiter sind solche Verbindungen der Formel I bevorzugt, worin $R^3$ Cyclohexylmethyl bedeutet. Auch bevorzugt sind solche Verbindungen der Formel I, worin $R^4$ Vinyl oder Aethyl bedeutet. Ebenfalls bevorzugt sind die Verbindungen der Formel I, worin A die Gruppe (a) oder (b) bedeutet. $R^5$ bedeutet vorzugsweise Phenyl oder Naphthyl, besonders bevorzugt Phenyl. Die bevorzugte Bedeutung von $R^6$ ist Alkylcarbonylalkyl, Cycloalkylcarbonylalkyl, Heterocycloalkylcarbonylalkyl, Aminocarbonylalkyl oder substituiertes Aminocarbonylalkyl, vorzugsweise $C_1$-$C_4$-Alkylcarbonylmethyl, $C_5$-$C_6$-Cycloalkylcarbonylmethyl, N-t-Butoxycarbonylpyrrolidinylcarbonylmethyl oder Aminocarbonylalkyl, welches durch $C_1$-$C_4$-Alkyl oder gegebenenfalls durch ein Sauerstoffatom unterbrochenes $C_4$-$C_5$-Alkylen disubstituiert oder durch Alkoxycarbonylalkyl oder Alkoxycarbonyl mono- oder di-substituiertes Aminoalkyl monosubstituiert ist. Im weiteren sind die Verbindungen der Formel I bevorzugt, worin Y den N-terminal mit Z verbundenen, bivalenten Rest von Phenylalanin, Cyclohexylalanin, 4-Chlorphenylalanin oder α-Naphthylalanin, besonders bevorzugt Phenylalanin, bedeutet. Z bedeutet vorzugsweise Acyl, besonders bevorzugt den C-terminal mit Y verbundenen Rest einer N- und/oder α-methylierten natürlichen Aminosäure mit der L-Konfiguration oder eines Epimeren einer solchen Aminosäure mit der D-Konfiguration oder eines Dipeptids aus zwei der eben

beschriebenen Aminosäuren oder die Gruppe $R^b$-CO- oder $R^a$-O-CO-, worin $R^a$ einen gegebenenfalls substituierten, gesättigten oder ungesättigten aliphatischen, cycloaliphatischen, cycloaliphatisch-aliphatischen Kohlenwasserstoffrest mit bis zu 18 Kohlenstoffatomen, einen gegebenenfalls substituierten aromatischen, heteraromatischen, aromatisch-aliphatischen oder heteroaromatisch-aliphatischen Kohlenwasserstoffrest mit bis zu 18 Kohlenstoffatomen oder einen gegebenenfalls substituierten, gesättigten 5- oder 6-gliedrigen Heterocyclus bedeutet und $R^b$ Wasserstoff bedeutet oder die Bedeutung von $R^a$ besitzt, ganz besonders bevorzugt den C-terminal mit Y verbundenen Rest von Prolin, Prolylprolin oder Histidinylprolin, wobei die Aminogruppe jeweils durch t-Butoxycarbonyl, Benzoxycarbonyl, Isovaleryl oder die Gruppe $R^b$-CO-substituiert ist, worin $R^b$ einen heteroaromatisch-aliphatischen Kohlenwasserstoffrest mit bis zu 10 Kohlenstoffatomen oder einen gesättigten 5- oder 6-gliedrigen Heterocyclus bedeutet.

Aus dem obigen folgt, dass solche Verbindungen der Formel I ganz besonders bevorzugt sind, worin $R^1$ Wasserstoff, $R^2$ Imidazol-4-yl, $R^3$ Cyclohexylmethyl, $R^4$ Vinyl oder Aethyl und $R^5$ Phenyl und $R^6$ $C_1$-$C_4$-Alkylcarbonylmethyl, $C_5$-$C_6$-Cycloalkylcarbonylmethyl, N-t-Butoxycarbonylpyrrolidinylcarbonylmethyl oder Aminocarbonylalkyl, welches durch $C_1$-$C_4$-Alkyl oder gegebenenfalls durch ein Sauerstoffatom unterbrochenes $C_4$-$C_5$-Alkylen disubstituiert oder durch Alkoxycarbonylalkyl oder Alkoxycarbonyl mono- oder disubstituiertes Aminoalkyl mono-substituiert ist, oder Y den N-terminal mit Z verbundenen, bivalenten Rest von Phenylalanin und Z den C-terminal mit Y verbundenen Rest von Prolin, Prolylprolin oder Histidinylprolin bedeuten, wobei die Aminogruppe jeweils durch t-Butoxycarbonyl, Benzoxycarbonyl, Isovaleryl oder die Gruppe $R^b$-CO- substituiert ist, worin $R^b$ einen heteroaromatisch-aliphatischen Kohlenwasserstoffrest mit bis zu 10 Kohlenstoffatomen oder einen gesättigten 5- oder 6-gliedrigen Heterocyclus bedeutet.

Ganz speziell bevorzugte Verbindungen der Formel I sind:

- t-Butyl (R)-2-[[(S)-α-[[(S)-1-[(1S,2S,4S) -1-(cyclohexylmethyl)-2-hydroxy-4-isopropylhexyl] -2-imidazol-4-yläthyl]carbamoyl] phenäthyl]carbamoyl]-1-pyrrolidincarboxylat;
- (2S,3S,5S)-2-(Boc-D-Pro-Phe-His-NH) -1-cyclohexyl-5-isopropyl-6-hepten-3-ol;
- N-(S)-[(1S,2S,4S)-1- (Cyclohexylmethyl-2-hydroxy-4-isopropyl-5-hexenyl] -α[(S)-α-3-methylbutyramido]-imidazol-4-propionamid;
- t-Butyl [(S)-α-[[(S)-1[[(1S,2S,4S) -1-cyclohexylmethyl-2-hydroxy-4- isopropyl-5-hexenyl]carbamoyl]-2-imidazol-4-yläthyl]carbamoyl]phenäthyl]carbamat;
- t-Butyl [(S)-α-[[(S)-1-[[(1S,2S,4S) -1-cyclohexylmethyl-2-hydroxy-4-isopropylhexyl] carbamoyl]-2-imidazol-4-yläthyl] carbamoyl]phenäthyl]carbamat;
- t-Butyl (S)-2-[[(R)-α-[[(S)-1-[[(1S,2S,4S)-1-(cyclohexylmethyl) -2-hydroxy-4-isopropylhexyl]carbamoyl] -2-imidazol-4-yläthyl]carbamoyl] phenäthyl]acetyl]-1-pyrrolidincarboxylat;
- (S)-N-[(1S,2S,4S)-1-(Cyclohexylmethyl) -2-hydroxy-4-isopropylhexyl] -α-[[[[(R)-α-2-hydroxy-1-(hydroxymethyl) -1-methyläthyl]carbamoyl]methyl] hydrocinnamamido]imidazol-4-propionamid;
- N-(S)-1-[[(1S,2S,4S)-1-(Cyclohexylmethyl) -2-hydroxy-4-isopropylhexyl]carbamoyl] -2-imidazol-4-yläthyl]-γ-oxo-α -(1-naphthylmethyl)-4-morpholinbutyramid;
- t-Butoxycarbonyl [2-[(R und S)-3-[[(S)-1-[[(1S,2S,4S)-1-(cyclohexylmethyl) -2-hydroxy-4-isopropyl-5-hexenyl] carbamoyl]-2-imidazol-4-yläthyl] carbamoyl]-4-phenylbutyramido]äthyl]glycin-t-butylester;
- (S)-N-[(1S,2S,4S)-1-(Cyclohexylmethyl) -2-hydroxy-4-isopropyl-5-hexenyl] -α-[(R)-α-[[[2-hydroxy-1-(hydroxymethyl) -1-methyläthyl]carbamoyl]methyl] hydrocinnamamido]imidazol-4-propionamid;
- (S)-α-[(R)-2-Benzyl-5,5-dimethyl-4-oxohexanamido]-N-[(1S,2S,4S)-1- (cyclohexylmethyl-2-hydroxy-4-isopropyl-5-hexenyl] imidazol-4-propionamid;
- (S)-N-[(1S,2S,4S)-1-(Cyclohexylmethyl) - 2-hydroxy-4-isopropyl-5-hexenyl] -α-2-[N-(morpholinocarbamoyl)-3-phenyl-L-alanyl]amino]imidazol-4-propionamid;
- (S)-α-[(R)-α-(Carbamoylmethyl) hydrocinnamamido]-N-[(1S,2S,4S) -1-(cyclohexylmethyl) -2-hydroxy-4-isopropyl-5-hexenyl]imidazol-4-propionamid;
- (S)-N-[(1S,2S,4S)-1-(Cyclohexylmethyl) -2-hydroxy-4-isopropyl-5-hexenyl] -α-[(R)-α-[(dimethylcarbamoyl)-methyl]hydrocinnamamido]imidazol-4-propionamid;
- (S)-N-[(1S,2S,4S)-1-(Cyclohexylmethyl) -2-hydroxy-4-isopropyl-5-hexenyl] -α-[(RS)-α-[-(cyclopentylcarbonyl) methyl]hydrocinnamamido]imidazol-4-propionamid;
- (S)-N-[(1S,2S,4S)-1-(Cyclohexylmethyl) -2-hydroxy-4-isopropylhexyl]-α-[(RS) -α-[(cyclopentylcarbonyl)-methyl] hydrocinnamamido]imidazol-4-propionamid und
- (2S,5S)-2-(Boc-D-Pro-Pro-Phe-His-NH) -1-cyclohexyl-5-isopropyl-6-hepten-3-ol.

Die Verbindungen der Formel I in Form von optisch reinen Diastereomeren, Diastereomerengemischen, diastereomeren Racematen oder Gemischen von diastereomeren Racematen sowie pharmazeutisch verwendbare Salz davon können hergestellt werden, indem man

a) eine Verbindung der allgemeinen Formel

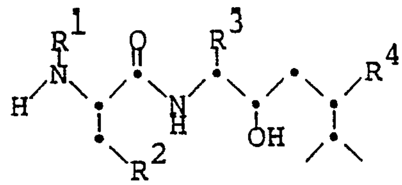

II

worin $R^1$, $R^2$, $R^3$ und $R^4$ die oben angegebene Bedeutung besitzen, mit einem die Gruppe

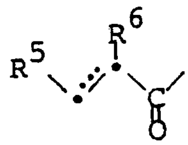

oder

-Y-Z

(a) (b)

worin $R^5$, $R^6$, Y, Z und die gestrichelte Linie die oben angegebene Bedeutung besitzen, abgebenden Acylierungsmittel umsetzt, oder

b) eine Verbindung der allgemeinen Formel

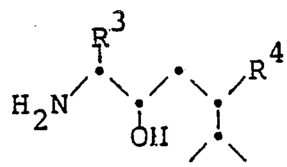

III

worin $R^3$ und $R^4$ die oben angegebene Bedeutung besitzen, mit einer Verbindung der allgemeinen Formel

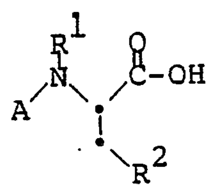

IV

worin $R^1$, $R^2$ und A die oben angegebene Bedeutung besitzen, oder einem aktivierten Derivat davon umsetzt, oder

c) zur Herstellung einer Verbindung der Formel I, worin $R^4$ Aethyl bedeutet und die übrigen Symbole die oben angegebene Bedeutung besitzen, eine Verbindung der Formel I, worin $R^4$ Vinyl bedeutet und die übrigen Symbole die oben angegebene Bedeutung besitzen, hydriert, oder

d) zur Herstellung einer Verbindung der Formel I, worin $R^4$ 1,2-Dihydroxyäthyl bedeutet und die übrigen Symbole die oben angegebene Bedeutung besitzen, eine Verbindung der Formel I, worin $R^4$ Vinyl bedeutet und die übrigen Symbole die oben angegebene Bedeutung besitzen, oxidiert, oder

e) zur Herstellung einer Verbindung der Formel I, worin A eine freie Aminogruppe enthält, aus einer entsprechenden Verbindung der Formel I, in der A eine N-geschützte Aminogruppe enthält, die N-Schutzgruppe abspaltet, und/oder

f) erwünschtenfalls ein Gemisch diastereomerer Racemate in die diastereomeren Racemate oder optisch reinen Diastereomeren auftrennt, und/oder

g) erwünschtenfalls ein Diastereomerengemisch in die optisch reinen Diastereomeren auftrennt, und/oder

h) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide

und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0° C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Falls es sich beim Acylierungsmittel um ein Peptid handelt, erfolgt die Reaktion unter in der Peptidchemie üblichen Reaktionsbedingungen, d.h. vorzugsweise in Gegenwart eines Kondensationsmittels wie HBTU (O-Benzotriazolyl-N,N,N′,N′-tetramethyluronium-hexafluorophosphat), BOP (Benzotriazol-1-yloxy-bis-(dimethylamino)phosphonium-hexafluorophosphat), HOBT (N-Hydroxybenzotriazol), DBU (1,8-Diazabicyclo-[5.4.0]undec-7-en), DCC (Dicyclohexylcarbodiimid), EDC (N-Aethyl-N′(3-dimethylaminopropyl)carbodiimid-hydrochlorid), Hünigbase (Aethyldiisopropylamin), und dergleichen. Die Reaktion wird zweckmässigerweise in einem unter den Reaktionsbedingungen inerten organischen Lösungsmittel oder Lösungsmittelgemisch bei einer Temperatur zwischen etwa 0° und 50° C, vorzugsweise bei etwa Raumtemperatur durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel III mit einer Verbindung der Formel IV erfolgt ebenfalls nach an sich in der Peptid-Chemie bekannten Methoden, d.h. unter den gleichen Bedingungen wie oben für die Umsetzung einer Verbindung der Formel II mit einem Peptid angegeben wurde. Beispiele geeigneter aktivierter Derivate einer Verbindung der Formel IV sind Säurehalogenide, Säureanhydride, gemischte Anhydride, Ester, gemischte Ester, und dergleichen.

Auch die Hydrierung einer Verbindung der Formel I, worin $R^4$ Vinyl bedeutet, erfolgt nach an sich bekannten Methoden in einem unter den Reaktionsbedingungen inerten organischen Lösungsmittel oder Lösungsmittelgemisch bei einer Temperatur zwischen etwa Raumtemperatur und 50° C, vorzugsweise bei Raumtemperatur, in Gegenwart eines Edelmetallkatalysators, wie Platin oder Palladium, vorzugsweise Palladium. Als Lösungsmittel kommen insbesondere Alkohole, wie Methanol oder Aethanol, und dergleichen in Frage.

Auch die Oxidation einer Verbindung der Formel I, worin $R^4$ Vinyl bedeutet, erfolgt nach an sich bekannten Methoden in einem unter den Reaktionsbedingungen inerten organischen Lösungsmittel oder Lösungsmittelgemisch bei einer Temperatur zwischen etwa Raumtemperatur und dem Siedepunkt des Lösungsmittels bzw. Lösungsmittelgemisches, vorzugsweise bei etwa Raumtemperatur. Als Oxidationsmittel eignet sich insbesondere Osmiumtetroxid. Als Lösungsmittel kommt insbesondere Pyridin und dergleichen in Frage.

Die Abspaltung der N-Schutzgruppe gemäss Verfahrensvariante e) erfolgt ebenfalls nach an sich bekannten Methoden in einem unter den Reaktionsbedingungen inerten organischen Lösungsmittel oder Lösungsmittelgemisch bei einer Temperatur zwischen etwa 0° C und der Raumtemperatur mit einer Säure, wie Chlorwasserstoffsäure, Trifluoressigsäure, und dergleichen. Geeignete Lösungsmittel sind Aether, wie Tetrahydrofuran oder Dioxan, Alkohole, wie Methanol, oder chlorierte Kohlenwasserstoffe, wie Methylenchlorid, und dergleichen.

Die Ausgangsstoffe der Formel II sind neu und ebenfalls Gegenstand der vorliegenden Erfindung. Diese Verbindungen können hergestellt werden, indem man eine Verbidnung der Formel III mit gegebenenfalls N-methyliertem Histidin, Norleucin oder Norvalin umsetzt. Diese Umsetzung erfolgt ebenfalls nach in der Peptid-Chemie bekannten Methoden, d.h. unter den Reaktionsbedingungen, wie sie weiter oben für die Umsetzung einer Verbindung der Formel II mit einem Peptid beschrieben sind.

Die Ausgangsstoffe der Formel III sind ebenfalls neu und Gegenstand der vorliegenden Erfindung. Sie können beispielsweise hergestellt werden, indem man einen Aldehyd der allgemeinen Formel

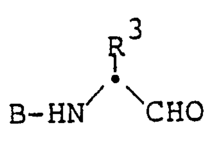

V

worin B eine Aminoschutzgruppe bedeutet, vorzugsweise t-Butoxycarbonyl oder Benzyloxycarbonyl, und $R^3$ die oben angegebene Bedeutung besitzt,
mit einer Verbindung der allgemeinen Formel

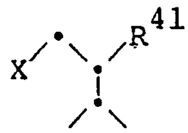

VI

worin X Chlor, Brom oder Jod, vorzugsweise Brom, und $R^{41}$ Phenyl, Furyl oder Vinyl bedeuten,
in einer Grignard-Reaktion umsetzt. Diese Umsetzung erfolgt ebenfalls nach an sich bekannten Methoden, beispielsweise in einem unter den Reaktionsbedingungen inerten Lösungsmittel, wie einem Aether, bei einer Temperatur zwischen etwa 0° und 50° C, vorzugsweise bei Raumtemperatur. Anschliessend wird aus der enthalten Verbindung die Aminoschutzgruppe B nach bekannten Methoden abgespaltet, z.B. mit Salzsäure in Dioxan bei Raumtemperatur. Falls eine Verbindung der Formel III erwünscht ist, worin $R^4$ Aethyl oder 1,2-Dihydroxyäthyl bedeutet, wird das Produkt der Grignard-Reaktion vorgängig der Abspaltung der Aminoschutzgruppe B unter den für die Verfahrensvariante c) angegebenen Reaktionsbedingungen hydriert bzw. nach bekannten Methoden oxidiert. Geeignete Oxidationsmittel sind Osmiumtetroxid, Kaliumpermanganat, Rutheniumtetroxid, und dergleichen. Die Reaktion wird in einem unter den Reaktionsbedingungen inerten organischen Lösungsmittel bzw. Lösungsmittelgemisch bei einer Temperatur zwischen etwa Raumtemperatur und 50° C durchgeführt. Geeignete Lösungsmittel sind Pyridin, aromatische Kohlenwasserstoffe, wie Benzol, und dergleichen.

Die Verbindungen der Formel V sind bekannt oder können in Analogie zur Herstellung der bekannten Verbindungen erhalten werden.

Die Verbindung der Formel VI, worin $R^{41}$ Vinyl bedeutet, ist bekannt. Die Verbindungen der Formel VI, worin $R^{41}$ Phenyl oder Furyl bedeutet, sind neu und ebenfalls Gegenstand vorliegender Erfindung.

Je ein Verfahren zur Herstellung einer Verbindung der Formel III, worin $R^4$ Phenyl, Furyl bzw. Vinyl bedeutet, ist in den nachfolgenden Schemata I-III skizziert. Den Schemata II und III kann auch noch die Herstellung der neuen Verbindungen der Formel VI, worin $R^{41}$ Phenyl oder Furyl bedeutet, entnommen werden. Bezüglich der genauen Reaktionsbedingungen wird auf den Beispielteil verwiesen.

Schema I

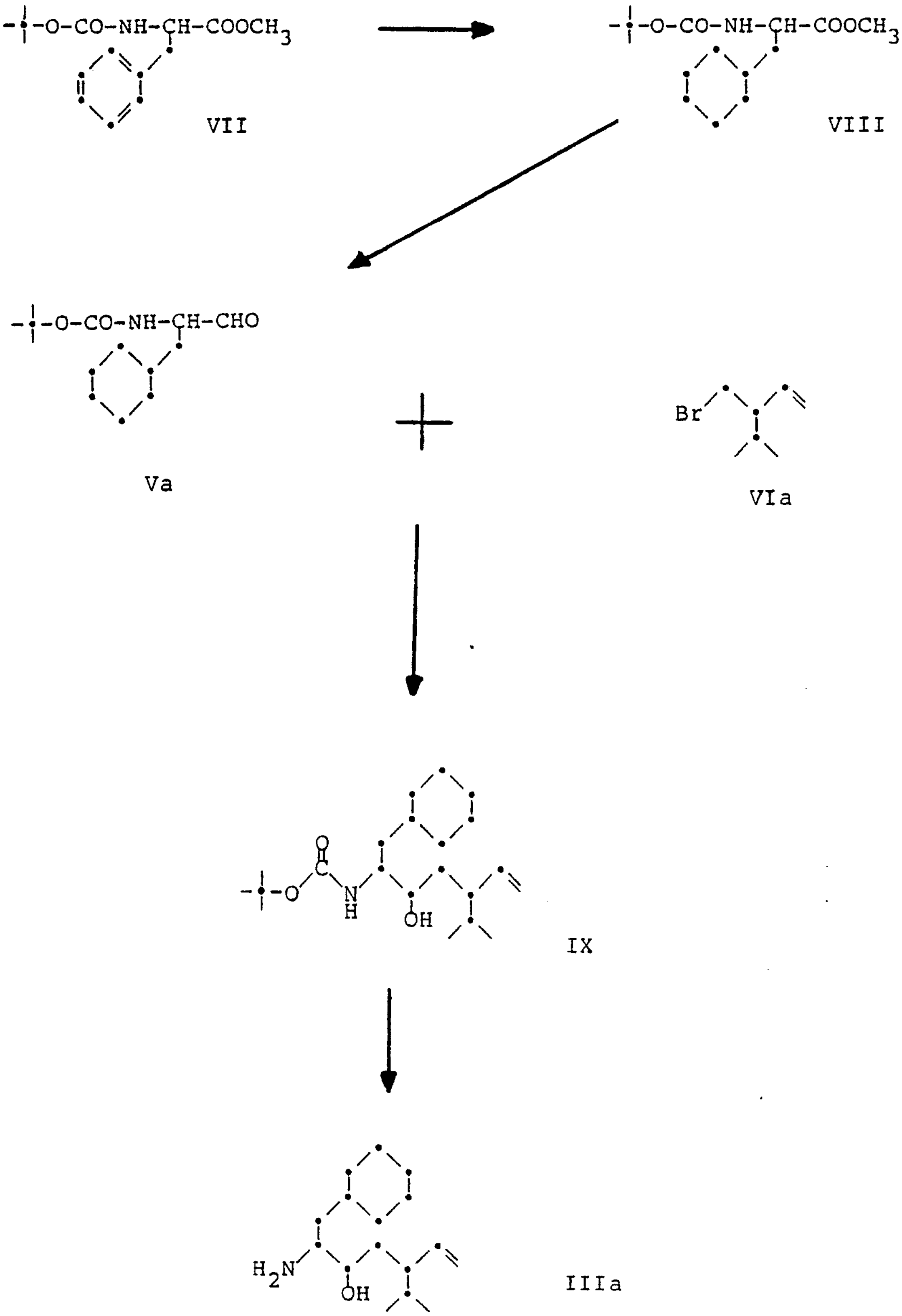

VII

VIII

Va

VIa

IX

IIIa

Schema II

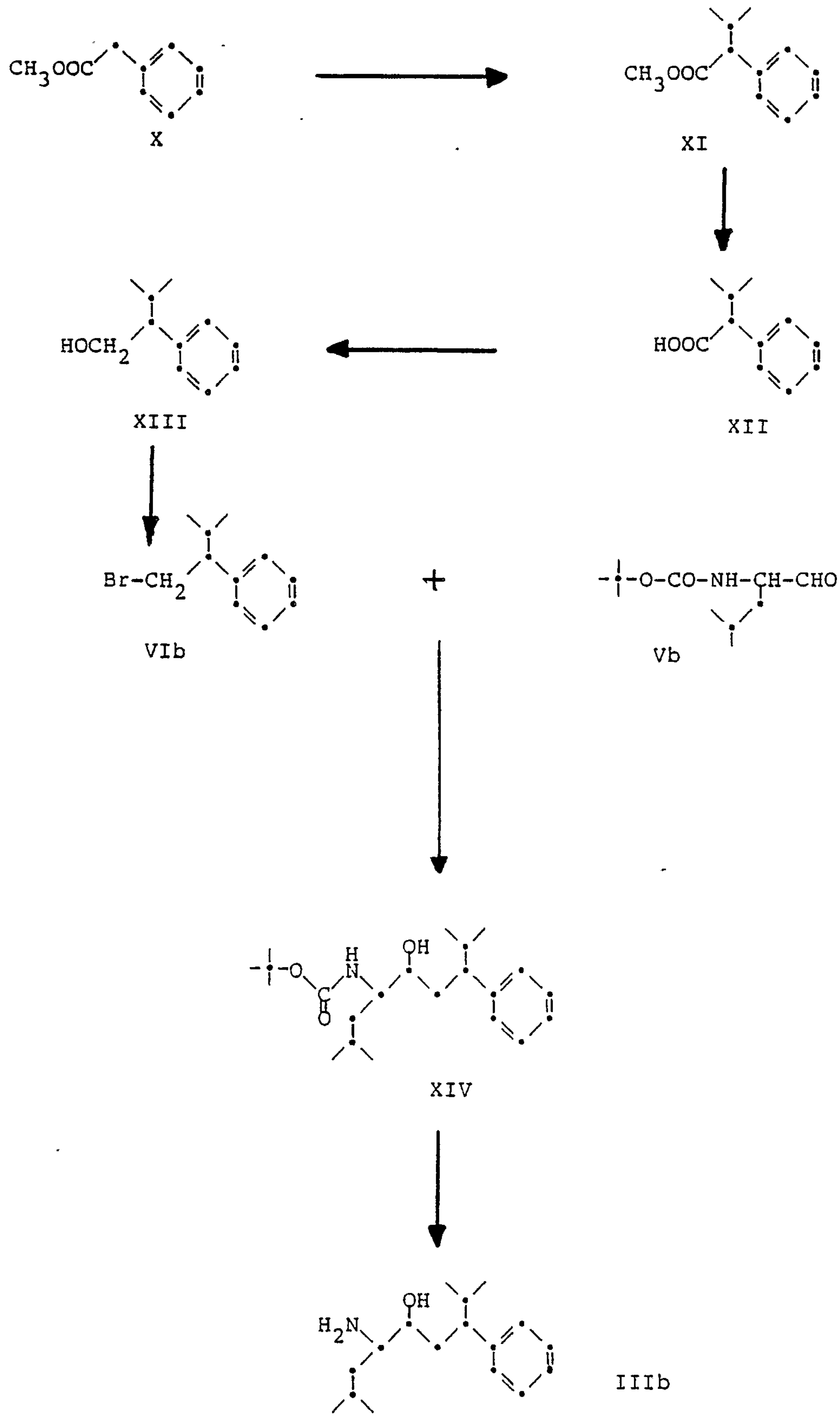

X

XI

XII

XIII

VIb

Vb

XIV

IIIb

Schema III

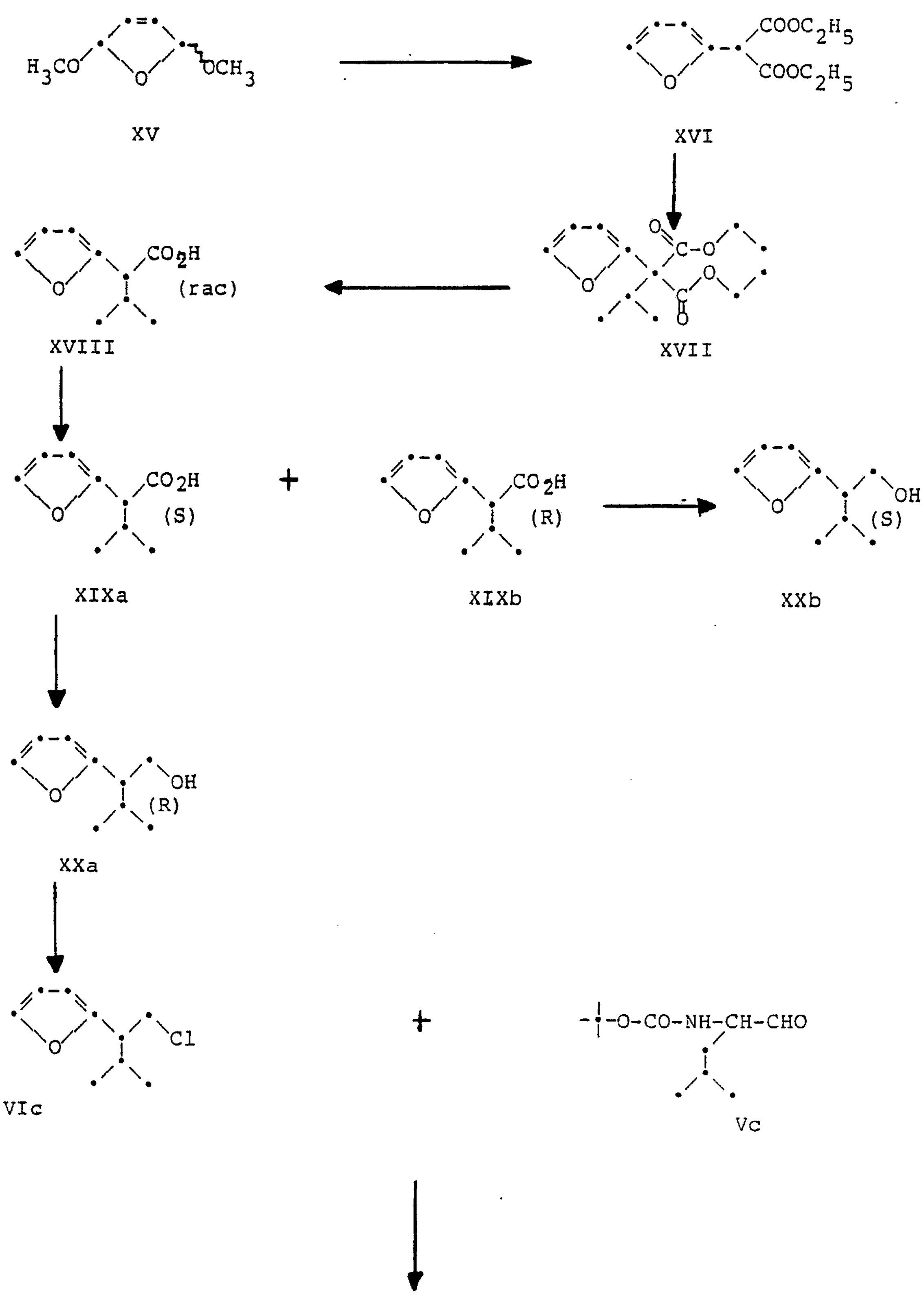

XV, XVI, XVII, XVIII, XIXa, XIXb, XXb, XXa, VIc, Vc

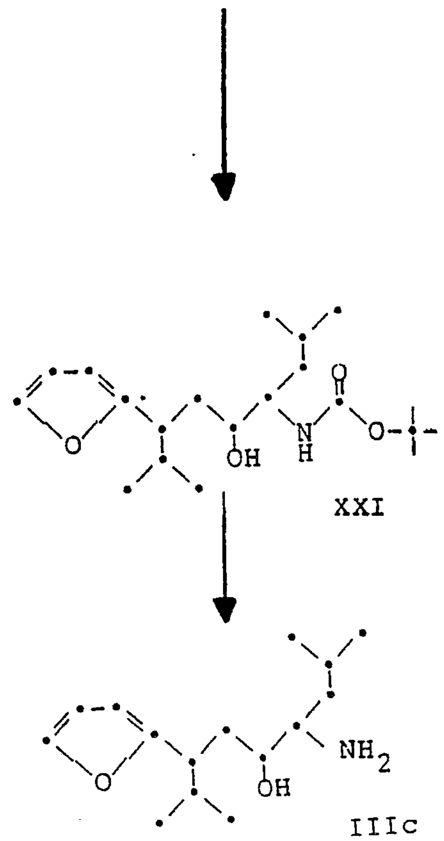

XXI

IIIc

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

In vitro-Test mit reinem Human-Renin

Der Test wird in Eppendorf Röhrchen durchgeführt. Die Inkubationsmischung besteht aus (1) 100 µl Human-Renin in Puffer A (0,1M Natriumphosphatlösung, pH 7,4, enthaltend 0,1 % Rinderserumalbumin, 0,1 % Natriumazid und 1mM Aethylendiamintetraessigsäure), genügend für eine Renin-Aktivität von 2-3 ng Angiotensin I/ ml/Std.; (2) 145 µl Puffer A; (3) 30 µl von 10 µM humanem Tetradekapeptid-Reninsubstrat (hTD) in 10 mM Salzsäure; (4) 15 µl Dimethylsulfoxid mit bzw. ohne Hemmer und (5) 10 µl einer 0,03 molaren Lösung von Hydroxychinolinsulfat in Wasser.

Die Proben werden drei Stunden bei 37° C bzw. 4° C in Triplikaten inkubiert. 2 x 100 µl Proben pro Versuchsröhrchen werden dann verwendet, um die Produktion von Angiotensin I via RIA (standard

radioimmunoassay; Clinical Assay solid phase kit) zu messen. Kreuzreaktivitäten der verwendeten Antikörper im RIA sind: Angiotensin I 100 %; Angiotensin II 0,0013 %; hTD (Angiotensin I-Val-Ile-His-Ser-OH) 0,09 %. Die Produktion von Angiotensin I wird durch die Differenz zwischen dem Versuch bei 37° C und demjenigen bei 4° C bestimmt.

Folgende Kontrollen werden mitgeführt:

(a) Inkubation von hTD-Proben ohne Renin und ohne Hemmer bei 37° C und 4° C. Die Differenz zwischen diesen beiden Werten ergibt den Grundwert der Angiotensin I-Produktion.

(b) Inkubation von hTD-Proben mit Renin, jedoch ohne Hemmer bei 37° C und 4° C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (= 100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus linearen Regressionskurve aus einem logit-log plot ermittelt.

Die in diesem Test erhaltenen Resultate sind in der folgenden Tabelle zusammengefasst:

Tabelle

| Verbindung | $IC_{50}$-Werte in µMol/lt. |
|---|---|
| A | 0,0035 |
| B | 0,0003 |
| C | 0,0090 |
| D | 0,0075 |
| E | 0,0079 |
| F | 0,0040 |
| G | 0,0150 |
| H | 0,0041 |
| I | 0,0460 |
| K | 0,0170 |
| L | 0,0070 |
| M | 0,0210 |
| N | 0,1400 |
| O | 0,0120 |
| P | 0,0460 |
| Q | 0,0680 |
| R | 0,0017 |

A= t-Butyl (R)-2-[[(S)-α-[[(S)-1-[(1S,2S,4S) -1-(cyclohexylmethyl-2-hydroxy-4-isopropylhexyl] -2-imidazol-4-yl-äthyl]carbamoyl] phenäthyl]carbamoyl]-1-pyrrolidincarboxylat;

B= (2S,3S,5S)-2-(Boc-D-Pro-Phe-His-NH) -1-cyclohexyl-5-isopropyl-6-hepten-3-ol;

C= N-(S)-[(1S,2S,4S)-1- (Cyclohexylmethyl-2-hydroxy-4-isopropyl-5-hexenyl] -α-[(S)-α-3-methylbutyramido]-imidazol-4-propionamid;

D= t-Butyl [(S)-α-[[(S)-1-[[(1S,2S,4S) -1-cyclohexylmethyl-2-hydroxy-4- isopropyl-5-hexenyl]carbamoyl] -2--imidazol-4-yläthyl]carbamoyl]phenäthyl]carbamat;

E= t-Butyl [(S)-α-[[(S)-1-[[(1S,2S,4S) -1-cyclohexylmethyl-2-hydroxy-4-isopropylhexyl] carbamoyl]-2-imidazol-4- -yläthyl] carbamoyl]phenäthyl]carbamat;

F= t-Butyl (S)-2-[[(R)-α-[[(S)-1-[[(1S,2S,4S) -1-(cyclohexylmethyl) -2-hydroxy-4-isopropylhexyl]carbamoyl] -2--imidazol-4-yläthyl]carbamoyl] phenäthyl]acetyl]-1-pyrrolidincarboxylat;

G= (S)-N-[(1S,2S,4S)-1-(Cyclohexylmethyl) -2-hydroxy-4-isopropylhexyl] -α-[[[[(R)-α-2-hydroxy-1-(hydroxymethyl) -1-methyläthyl]carbamoyl]methyl] hydrocinnamamido]imidazol-4-propionamid;

H= N-(S)-1-[[(1S,2S,4S)-1-(Cyclohexylmethyl) -2-hydroxy-4--isopropylhexyl]carbamoyl] -2-imidazol-4-yläthyl]-γ--oxo-α -(1-naphthylmethyl)-4-morpholinbutyramid;

I= t-Butoxycarbonyl [2-[(R und S)-3-[[(S)-1- [[(1S,2S,4S)--1-(cyclohexylmethyl) -2-hydroxy-4-isopropyl-5-hexenyl]-carbamoyl]-2-imidazol-4-yläthyl] carbamoyl]-4-phenylbutyramido]äthyl]glycin-t-butylester;

K= (S)-N-[(1S,2S,4S)-1-(Cyclohexylmethyl) -2-hydroxy-4-isopropyl-5-hexenyl] -α-[(R)-α-[[[2-hydroxy-1-(hydroxymethyl) -1-methyläthyl]carbamoyl]methyl] hydrocinnamamido]imidazol-4-propionamid;

L= (S)-α-[(R)-2-Benzyl-5,5-dimethyl-4-oxohexanamido] -N--[(1S,2S,4S)-1- (cyclohexylmethyl-2-hydroxy-4-isopropyl--5-hexenyl] imidazol-4-propionamid;

M= (S)-N-[(1S,2S,4S)-1-(Cyclohexylmethyl) -2-hydroxy-4-isopropyl-5-hexenyl] -α-2-[N-(morpholinocarbamoyl) -3--phenyl-L-alanyl]amino]imidazol-4-propionamid;

N= (S)-α-[(R)-α-(Carbamoylmethyl) hydrocinnamamido]-N--[(1S,2S,4S) -1-(cyclohexylmethyl) -2-hydroxy-4-isopropyl-5-hexenyl]imidazol-4-propionamid;

O= (S)-N-[(1S,2S,4S)-1-(Cyclohexylmethyl) -2-hydroxy-4-isopropyl-5-hexenyl] -α-[(R)-α-[(dimethylcarbamoyl)-methyl]hydrocinnamamido]imidazol-4-propionamid;

P= (S)-N-[(1S,2S,4S)-1-(Cyclohexylmethyl) -2-hydroxy-4-isopropyl-5-hexenyl] -α-[(RS)-α-[(cylcopentylcarbonyl)-methyl]hydrocinnamamido]imidazol-4-propionamid;

Q= (S)-N-[(1S,2S,4S)-1-(Cyclohexylmethyl) -2-hydroxy-4-isopropylhexyl]-α-[(RS) -α-[(cyclopentylcarbonyl)-methyl]hydrocinnamamido]imidazol-4-propionamid und

R= (2S,5S)-2-(Boc-D-Pro-Pro-Phe-His-NH) -1-cyclohexyl-5--isopropyl-6-hepten-3-ol.

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injectionslösungen eignen sich als Excipientien z.B. Wasser, Alkohole, Polyole, Glycerin, vegetable Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzel nen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen für Aminosäuren und Aminosäurederivate verwendet:

| | | |
|---|---|---|
| H-His-OH | = | L-Histidin |
| H-Phe-OH | = | L-Phenylalanin |
| H-Phe $\overset{R}{-}$ His-O-H | = | N-[(S)-2-Amino-3-phenylpropyl]-L-histidin |
| H-Pro-OH | = | L-Prolin |
| H-Ser-OH | = | L-Serin |
| H-Tyr-OH | = | L-Tyrosin |

| Weitere Abkürzungen: | | |
|---|---|---|
| Boc | = | t-Butoxycarbonyl |
| Bom | = | Benzyloxymethyl |
| IVA | = | Isovaleryl |
| OBz | = | Benzyloxy |
| OMe | = | Methoxy |
| Pip | = | Piperidinyl |
| Fmoc | = | 9-Fluorenylmethoxycarbonyl |
| t-Bu | = | t-Butyl |

## Beispiel 1

Ein Gemisch von 313 mg (1,18 mMol) t-Butoxycarbonyl-L-phenylalanin, 460 mg (1,18 mMol) (S)-α-Amino-N-[(1S,2S,4S)-1-(cyclohexylmethyl) -2-hydroxy-4-isopropyl-5- hexenyl]imidazol-4-propionamid, 0,15 ml (1,18 mMol) 4-Aethylmorpholin, 320 mg (2,36 mMol) HOBT und 292 mg (1,42 mMol) DCC in 20 ml Dimethylformamid wird über Nacht bei Raumtemperatur gerührt. Danach wird der ausgefallene Niederschlag abfiltriert, und das Lösungsmittel im Hochvakuum abgedampft. Der Rückstand wird in Essigester gelöst und dann nacheinander mit 2N Natriumbicarbonat-Lösung, gesättig- ten Ammoniumchlorid-Lösung, nochmals 2N Natriumbicarbonat-Lösung und nochmals gesättigter Ammoniumchlorid-Lösung gewäschen. Nach dem Trocknen der organischen Lösung über Natriumsulfat wird das Lösungsmittel unter vermindertem Druck abgedampft, und der Rückstand (870 mg) zur Reinigung an 30 g Kieselgel unter Verwendung eines 20:1:0,1-Gemisches von Methylenchlorid, Methanol und Ammoniak als Eluierungsmittel chromatographiert. Kristallisation des so erhaltenen Rohprodukts aus Methylenchlorid/Aether liefert 410 mg t-Butyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamat, Schmelzpunkt 161°.

Das als Ausgangsmaterial eingesetzte (S)-α-Amino-N-[(1S,2S,4S)-1-(cyclohexylmethyl)-2-hydroxy-4-isopropyl-5-hexenyl]imidazol-4-propionamid wurde wie folgt hergestellt:

315,3 g (1,28 mMol) t-Butoxycarbonyl-L-phenylalanin-methylester werden nach der von J. Boger et al. in J.Med.Chem., 28, 1779 (1985) beschriebenen Methode in Gegenwart von 5%-igem Rhodium auf

Aluminiumoxyd bei Raumtempera tur und 440 kPa hydriert, wobei man 315,3 g 2-t-Butoxycarbonylamino-3-cyclohexylpropionsäuremethylester als Oel erhält, das direkt in die nächste Stufe eingesetzt wird.

Zu 85,61 g (300 mMol) 2-t-Butoxycarbonylamino-3-cyclohexylpropionsäuremethylester in 1,2 l Toluol werden bei -75° 750 ml (750 mMol) einer 20%igen Lösung von Diisobutylaluminiumhydrid in Hexan im Verlauf von 90 Minuten getropft. Nach beendeter Zugabe wird das Reaktionsgemisch noch 10 Minuten bei -75° gerührt. Anschliessend werden je innerhalb von 25 Minuten bei einer Temperatur von -75° bis -70° zuerst 70 ml Methanol und dann 840 ml gesättigte Kalium-Natrium-Tartrat-Lösung zugetropft. Die sich langsam auf Raumtemperatur erwärmte milchige Reaktionslösung wird danach mit Aether extrahiert, und die Extrakte getrocknet und eingedampft, wobei man 82 g 2-t-Butoxycarbonylamino-3-cyclohexylpropylaldehyd als Oel erhält, welches ohne weitere Reinigung in die nächste Stufe eingesetzt wird.

Zu 24,65 g Magnesiumspänen in 300 ml Aether wird in einer Argonatmosphäre und bei einer Temperatur zwischen 30° und der Rückflusstemperatur des Lösungsmittels innerhalb von 2,5 Stunden eine Lösung von 179,7 g (1,01 Mol) 4-Brom-3-isopropyl-1-buten (welches nach der von R.G. Helmquist in Tetrahedron Letters, 2533 (1982) beschriebenen Methode hergestellt wurde) in 900 ml Aether getropft. Nach beendeter Zugabe wird das Reaktionsgemisch 3,5 Stunden zum Rückfluss erhitzt. Zum auf -60° abgekühlten Reaktionsgemisch wird innerhalb 75 Minuten eine Lösung von 82 g 2-t-Butoxycarbonylamino-3-cyclohexylpropylaldehyd in 900 ml Aether getropft, wobei die Temperatur -60° bis -70° beträgt. Nach beendeter Zugabe wird das Kühlbad entfernt, und das Reaktionsgemisch 21 Stunden bei Raumtemperatur unter Argon gerührt. Dann kühlt man auf 5° ab und gibt unter Rühren vorsichtig 225 ml einer gesättigten Ammoniumchloridlösung zu, wobei die Temperatur auf 20° ansteigt. Die beiden Phasen werden getrennt, und die organische Phase über Natriumsulfat getrocknet und eingedampft, wobei man 127,5 g eines gelben Oels erhält. Chromatographische Auftrennung dieses Oels an 2,5 kg Kieselgel mit Methylenchlorid, welches 2,5% Aether enthält, als Eluierungsmittel liefert 33,9 g (αS,βS)-β-t-Butoxycarbonylamino-α- [(S)-2-isopropyl-3-butenyl]-cyclohexanpropanol, 13,62 g (αS, βS)-β-t-Butoxycarbonylamino-α-[(R)-2- isopropyl-3-butenyl]-cyclohexanpropanol sowie 35,5 g eines Gemisches der beiden oben genannten Diastereomeren in Form von Oelen, MS: 354 $(M+H)^+$.

1,0 g (2,83 mMol) (αS, βS)-β-t-Butoxycarbonylamino-α-[(S)-2-ispropyl-3-butenyl]cyclohexanpropanol wird in 20 ml 2,2N Chlorwasserstoff in Dioxan gelöst und 2 Stunden bei Raumtemperatur gerührt. Danach wird das Reaktionsgemisch eingedampft und zweimal nacheinander zuerst mit Toluol versetzt und dann wiederum zur Trockene eingedampft. Auf diese Weise erhält man dünnschichtchromatographisch reines (αS, βS)-β-Amino-α-[(S)-2-isopropyl-3-butenyl]cyclohexanpropanol in Form des Hydrochlorids, welches direkt in die nächste Stufe eingesetzt wird.

1,7 g (2,83 Mmol) N-α-N-im-Bis-Fmoc-L-histidin und 0,82 g (2,83 Mmol) (αS, βS)-β-Amino-α-[(S)-2-isopropyl-3-butenyl]cyclohexanpropanol-hydrochlorid in 20 ml Dimethylformamid werden mit 0,36 ml 4-Aethylmorpholin und 0,77 g HOBT versetzt und im Eisbad gekühlt. Zur gekühlten Lösung werden 0,7 g DCC zugegeben, und das Gemisch über Nacht unter einer Argonatmosphäre gerührt, ohne dass das Eisbad entfernt wird, wodurch sich das Reaktionsgemisch langsam auf Raumtemperatur erwärmen kann. Danach wird der ausgefallene Niederschlag abfiltriert, und das Filtrat im Hochvakuum eingedampft. Der Rückstand wird auf Eis und 70 ml einer 2N Natriumbicarbonatlösung gegossen und dreimal mit je 150 ml Essigester extrahiert. Die organischen Extrakte werden einmal mit Eis und 70 ml gesättigter Ammoniumchloridlösung, einmal mit 70 ml Eis und 2N Natriumbicarbonatlösung und einmal mit 70 ml gesättigter Kochsalzlösung gewaschen, über Magnesiumsulfat getrocknet und eingedampft. Das auf diese Weise erhaltene Rohprodukt (2,31 g) wird in 3 ml Piperidin und 75 ml Methylenchlorid 3,5 Stunden bei Raumtemperatur gerührt. Dann wird bei etwa 30° eingedampft, und der Rückstand mit 60 ml Hexan trituriert, wobei man 1,44 g eines Festkörpers erhält, den man über 120 g Silicagel mit einem 12:1:0,1-Gemisch von Methylenchlorid, Methanol und Ammoniak chromatographiert. Zweimaliges Umkristallisieren des erhaltenen Rohprodukts aus Hexan liefert 580 mg von dünnschichtchromatographisch reinem (S)-α-Amino-N-[(1S,2S,4S)-1-(cyclohexylmethyl)-2-hydroxy-4-isopropyl-5-hexenyl]imidazol-4-propionamid als Kristalle, Schmelzpunkt 73°.

## Beispiel 2

130 mg (0.24 mMol) t-Butyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamat werden in 30 ml Methanol gelöst und in Gegenwart von 65 mg Palladium auf Kohle (5%ig) 1,5 Stunden bei Raumtemperatur hydriert. Nach beendeter Wasserstoffaufnahme wird der Katalysator abfiltriert, und das Filtrat eingedampft. Kristallisation

des Rückstands aus Aether/Hexan liefert 110 mg t-Butyl [(S)-α[[(S)-1-[[(1S,2S,4S)-1-(cyclohexylmethyl)-2-hydroxy-4-isopropylhexyl]carbamoyl]-2-imidazol-4-yläthyl]carbamoyl]phenäthyl]carbamat als Kristalle, Schmelzpunkt 101°.

Beispiel 3

Ein Gemisch von 83 mg (0,345 mMol) Dibenzylessigsäure, 90 mg (0,23 mMol) (S)-α-Amino-N-[(1S,2S,4S)-1-(cyclohexylmethyl)-2-hydroxy-4-isopropyl-5-hexenyl]imidazol-4-propionamid, 0,045 ml (0,345 mMol) 4-Aethylmorpholin, 93 mg (0,69 mMol) HOBT und 85 mg (0,41 mMol) DCC in 15 ml Dimethylformamid wird über Nacht bei Raumtemperatur gerührt. Danach wird der ausgefallene Niederschlag abfiltriert, und das Filtrat zur Trockene eingedampft. Dann wird der Rückstand in Essigester gelöst, und die Lösung nacheinander mit 2N Natriumbicarbonat-Lösung, gesättigter Ammoniumchlorid-Lösung, nochmals 2N Natriumbicarbonat-Lösung und nochmals gesättigter Ammoniumchlorid-Lösung gewaschen, über Natriumsulfat getrocknet und eingedampft. Der so erhaltene Rückstand wird mit einem 14:1:0,1-Gemisch von Methylenchlorid, Methanol und Ammoniak an 30 g Kieselgel chromatographiert. Kristallisation des Rückstands aus Methylenchlorid/Methanol/Hexan liefert 60 mg (S)-N-[(1S,2S,4S)-1-(Cyclohexylmethyl)-2-hydroxy-4-isopropyl-5-hexenyl] -α-(2,2-dibenzylacetamido)imidazol-4-propionamid, Schmelzpunkt 98°.

Beispiel 4

Ein Gemisch von 880 mg (2,5 mMol) (S)-α-Amino-N-[(1S,2S,4S)-2-hydroxy-1-isobutyl-4-isopropyl-5-hexenyl]-imidazol-4-propionamid, 1,99 g (7,5 mMol) t-Butoxycarbonyl-L-phenylalanin, 1,05 ml (7,5 mMol) Triäthylamin und 3,32 g (7,5 mMol) BOP in 100 ml Acetonitril wird 6 Tage bei Raumtemperatur gerührt. Danach wird das Lösungsmittel abgedampft, und der Rückstand in Essigester gelöst und nacheinander mit 2N Natriumbicarbonat-Lösung, gesättigter Ammoniumchlorid-Lösung, nochmals 2N Natriumbicarbonat-Lösung und nochmals gesättigter Ammoniumchlorid-Lösung gewaschen. Die organische Lösung wird über Natriumsulfat getrocknet und eingedampft, und der Rückstand zweimal mit Hexan trituriert. Der Rückstand wird mit einem 98:2-Gemisch von Chloroform und Methanol an 100 g Kieselgel chromatographiert. Umkristallisation des so erhaltenen Rohprodukts aus Methylenchlorid und Hexan liefert 400 mg t-Butyl [(S)-α-[[(S)-1-[[(1S,2S,4S)-2-hydroxy-1-isobutyl-4-isopropyl-5-hexenyl]carbamoyl]-2-imidazol-4-yläthyl]-carbamoyl]phenäthyl]carbamat als weisses Pulver, Schmelzpunkt 82°.

Das als Ausgangsmaterial eingesetzte (S)-α-Amino-N-[(1S,2S,4S)-2-hydroxy-1-isobutyl-4-isopropyl-5-hexenyl]imidazol-4-propionamid wurde wie folgt hergestellt:

t-Butoxycarbonyl-L-leucinmethylester wird in analoger Weise wie in Beispiel 1 beschrieben mit Diisobutylaluminiumhydrid zu 2-t-Butoxycarbonylamino-4-methyl-valeraldehyd reduziert, der ohne weitere Reinigung direkt in dei nächste Stufe eingesetzt wird.

Zu 16,8 g Magnesiumspänen in 980 ml Aether wird unter Argon und bei Raumtemperatur innerhalb von 75 Minuten eine Lösung von 142,3 g (691 mMol) 4-Brom-3-isopropyl-1-buten in 600 ml Aether getropft. Nach beendeter Zugabe wird das Reaktionsgemisch 3,25 Stunden zum Rückfluss erhitzt und danach auf -60° abgekühlt. Dazu wird innerhalb von 75 Minuten eine Lösung von 50,1 g (203,8 mMol) 2-t-Butoxycarbonylamino-4-methyl-valeraldehyd in 600 ml Aether getropft. Nach beendeter Zugabe wird das Kühlbad entfernt, und das Reaktionsgemisch 17 Stunden bei Raumtemperatur gerührt. Dann kühlt man auf 5° ab und gibt unter Rühren 150 ml einer gesättigten Ammoniumchloridlösung tropfenweise zu, wobei die Temperatur auf 20° ansteigt. Nach analoger Aufarbeitung wie in Beispiel 1 beschrieben erhält man 88,4 g eines gelben Oels, welches an 2,5 kg Kieselgel mit einem 98:2-Gemisch von Methylenchlo rid und Aether chromatographiert wird. Nach nochmaliger Chromatographie der Mischfraktion unter den gleichen Bedingungen erhält man insgesamt 30,2 g reines, dünnschichtchromatographisch einheitliches (4S)-t-Butoxycarbonylamino-(5S)-hydroxy-(7S)-isopropyl-2-methyl-8-nonen, MS: 240 $(M\text{-t-Butoxy})^+$.

In analoger Weise wie in Beispiel 1 beschrieben wird mit Chlorwasserstoff in Dioxan anschliessend die t-Butoxycarbonyl-Gruppe abgespalten, und das so erhaltene (4S)-Amino(5S)-hydroxy-(7S)-isopropyl-2-methyl-8-nonen ohne weitere Reinigung direkt in dei nächste Stufe eingesetzt.

Ein Gemisch von 550 mg (1,75 mMol) (4S)-Amino-(5S)-hydroxy-(7S)-isopropyl-2-methyl-8-nonen, 1,547 mg (2,65 mMol) N-α-N-im-Bis-Fmoc-L-histidin, 2,323 g BOP und 0,73 ml Triäthylamin in 30 ml Acetonitril wird 4 Tage bei Raumtemperatur gerührt. Danach wird das Lösungsmittel unter vermindertem Druck

abgedampft, und der Rückstand in analoger Weise wie in Beispiel 1 beschrieben aufgearbeitet. Chromatographie des Rohprodukts an 500 g Kieselgel mit einem 140:5:0,5-Gemisch vcn Methylenchlorid, Methanol und Ammoniak liefert 950 mg Fluoren-9-ylmethyl [(S)-1-[[(1S,2S,4S)-2-hydroxy-1-isobutyl-4-isopropyl-5-hexenyl]carbamoyl]-2-imidazol-4-yläthyl]-carbamat als Oel, welches direkt in die nächste Stufe eingesetzt wird, MS: 369 $(M+H)^+$.

1,3 g Fluoren-9-ylmethyl [(S)-1-[[(1S,2S,4S)-2-hydroxy-1-isobutyl-4-isopropyl-5-hexenyl]carbamoyl]-2-imidazol-4-yläthyl]carbamat in 30 ml Methylenchlorid und 2 ml Piperidin werden 1,5 Stunden bei Raumtemperatur gerührt. Danach wird das Lösungsmittel abgedampft, und der Rückstand mit Hexan trituriert. Das auf diese Weise erhaltene (S)-α-Amino-N-[(1S,2S,4S)-2-hydroxy-1-isobutyl-4-isopropyl-5-hexenyl]imidazol-4-propionamid wird ohne weitere Reinigung in die nächste Stufe eingesetzt.

## Beispiel 5

Ein Gemisch von 460 mg (1,18 mMol) (S)-α-Amino-N-[(1S,2S,4S)-1-(cyclohexylmethyl)-2-hydroxy-4-isopropyl-5-hexenyl]imidazol-4-propionamid, 313 mg (1, 18 mMol) t-Butoxycarbonyl-L-phenylalanin, 0,15 ml (1,18 mMol) 4-Aethylmorpholin, 320 mg (2,36 mMol) HOBT und 292 mg (1,42 mMol) DCC in 20 ml Dimethyleformamid wird 18 Stunden bei Raumtemperatur gerührt, und anschliessend in üblicher Weise aufgearbeitet. Chromatographie des Rückstandes an 30 g Keiselgel unter Verwendung eines 20:1:0,1-Gemisches von Methylenchlorid, Methanol und Ammoniak als Eluierungsmittel leifert 410 mg Kristalle vom Schmelzpunkt 161°.

2,5 g der obigen Verbindung werden in 50 ml 2,2N Chlorwasserstoff in Dioxan gelöst und 6 Stunden bei Raumtemperatur gerührt. Danach wird das Reaktionsgemisch unter vermindertem Druck eingedampft, und der Rückstand durch Chromatographie an Kieselgel mit einem 190:10:1-Gemisch von Methylenchlorid, Methanol und Ammoniak gereinigt, wobei man N-[(1S,2S,4S)-1-(Cyclohexylmethyl)-2-hydroxy-4-isopropyl-5-hexenyl]-α-(3-phenyl-L-alanyl)imidazol-4-propionamid als einheitliches Material erhält, MS: 538 $(M+H)^+$.

## Beispiel 6

In analoger Weise wie in Beispiel 1 beschrieben wurden die folgenden Verbindungen hergestellt:

- Aus (S)-α-Amino-N-[(1S,2S,4S)-1-(cyclohexylmethyl)-2-hydroxy-4-isopropyl-5-hexenyl]imidazol-4-propionamid und α-Benzyltetrahydro-β-oxo-4H-1,4- oxazinpropionsäure das (S)-N-[(1S,2S,4S)-1-(Cyclohexylmethyl)-2-hydroxy-4-isopropyl-5-hexenyl]-α-[(RS)-α-(morpholinocarbonyl)hydrocinnamamido]imidazol-4-propionamid als Kristalle vom Schmelzpunkt 94° (aus Methylenchlorid/Aether/Hexan);
- Aus (S)-α-Amino-N-[(1S,2S,4S)-1-(cyclohexylmethyl)-2-hydroxy-4-isopropyl-5-hexenyl]imidazol-4-propionamid und t-Butoxycarbonyl-D-prolin das (2S,3S,5S)-2-(Boc-D-Pro-Phe-His-NH)-1-cyclohexyl-5-isopropyl-6-hepten-3-ol als Kristalle vom Schmelzpunkt 132° (aus Methylenchlorid/Aether/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propianamid und t-Butylessigsäure das (S)-N-[(1S,2S,4S)-1-(Cyclohexylmethyl)-2-hydroxy-4-isopropyl-5-hexenyl]-α-[(S)-α-(3,3-dimethylbutyramido)hydrocinnamoyl]imidazol-4-propionamid als Kristalle vom Schmelzpunkt 105° (Zers.; aus Methylenchlorid/Aether/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und Isovaleriansäure das N-(S)-[(1S,2S,4S)-1-(Cyclohexylmethyl)-2-hydroxy-4-isopropyl-5-hexenyl]-α-[(S)-α-3-methylbutyramido]imidazol-4-propionamid als Schaum, MS: 622 $(M+H)^+$;
- Aus (S)-α-Amino-N-[(1S,2S,4S)-1-(cyclohexylmethyl)-2-hydroxy-4-isopropyl-5-hexenyl]imidazol-4-propionamid und (S)-α-[[(S)-2-Benzylamino-3-phenylpropyl]amino]hydrozimtsäure das Benzyl [α-[[[α-[[(S)-1-[[(1S,2S,4S)-1-(cyclohexylmethyl)-2-hydroxy-4-isopropyl-5-hexenyl]carbamoyl]-2-imidazol-4-yläthyl]-carbamoyl]phenäthyl]amino]methyl]phenäthyl]carbamat als Kristalle vom Schmelzpunkt 89° (aus Methylenchlorid/Aether/Hexan);
- Aus (S)-α-Amino-N-[(S)-1-[[(1S,2S,4S)-2-hydroxy-1-isobutyl-4-isopropyl-5-hexenyl]carbamoyl]-2-imidazol-4-yläthyl]hydrocinnamamid, das aus t-Butyl [(S)-α-[[(S)-1-[[(1S,2S,4S)-2-hydroxy-1-isobutyl-4-isopropyl-5-hexenyl]carbamoyl]-2-imidazol-4-yläthyl]carbamoyl]phenäthyl]carbamat durch 18-stündiges Stehenlassen bei Raumtemperatur in 0,3N methanolischer Salzsäure erhalten wurde, und Cyclopentancarbonsäure das (S)-α-[(RS)-Cyclopentancarboxamido]-N-[(S)-1-[[(1S,2S,4S)-2-hydroxy-1-isobutyl-4-isopropyl-5-hexenyl]-carbamoyl]-2-imidazol-4-yläthyl]hydrocinnamamid als Kristalle vom Schmelzpunkt 185° (Zers.; aus

Methanol/Methylenchlorid/Hexan):

- Aus N-[(1S,2S,4S)-1-(Cyclohexylmethyl)-2-hydroxy-4-isopropyl-5-hexenyl]-α-(3-phenyl-L-alanyl)imidazol-4-propianamid und Benzyl [(methylthio)formimidoyl]carbamat (GB Patentschrift 2.085.444) das Benzyl [N-[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]amidino]carbamat als Schaum, MS: 714 $(M+H)^+$;

- Aus N-[(1S,2S,4S)-1-(Cyclohexylmethyl)-2-hydroxy-4-isopropyl-5-hexenyl]-α-(3-phenyl-L-alanyl)imidazol-4-propianamid und t-Butoxycarbonylaminovaleriansäure das t-Butyl [4[[(S)-α-[[(S)-1- [[(1S,2S,4S)-1-(cyclohexylmethyl) -2-hydroxy-4-isopropyl-5-hexenyl]carbamoyl]-2-imidazol-4-yläthyl]carbamoyl]phenäthyl]-carbamoyl]butyl]carbamat als Kristalle vom Schmelzpunkt 110° (aus Methylenchlorid/Aether/Hexan);

- Aus (S)-α-Amino-N-[(1S,2S,4S)-1-(cyclohexylmethyl)-2-hydroxy-4-isopropyl-5-hexenyl]imidazol-4-propionamid und N-t-Butoxycarbonyl-N-methyl-L-phenylalanin das t-Butyl [(S)-α-[[(S)-1-(S)-[[(1S,2S,4S)-1-(cyclohexylmethyl)-2-hydroxy-4-isopropyl-5-hexenyl]carbamoyl]-2-imidazol-4-yl-äthyl]carbamoyl]phenäthyl]-methylcarbamat als Kristalle vom Schmelzpunkt 93° (aus Aether/Hexan);

- Aus (S)-α-Amino-N-[(1S,2S,4R)-1-(cyclohexylmethyl)-2-hydroxy-4-isopropyl-5-hexenyl]imidazol-4-propionamid und t-Butoxycarbonyl-L-phenylalanin das (2S,3S,5R)-2-(Boc-Phe-His-NH)-1-cyclohexyl-5-isopropyl-6-hepten-3-ol als Schaum, MS: 638 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und Dibenzsuberanessigsäure (EPA 0.056.616) das N-[(1S,2S,4S)-1-(Cyclohexylmethyl)-2-hydroxy-4-isopropyl-5-hexenyl]-α-[2-(10,11-dihydro-5H-dibenzo[a,d]cyclohepten-5-yl)acetamido]imidazol-4-propionamid als Kristalle vom Schmelzpunkt 135° (aus Methylenchlorid/Aether/Hexan);

-Aus (S)-α-Amino-N-[(1S,2S,4S)-1-(cyclohexylmethyl)-2-hydroxy-4-isopropyl-5-hexenyl]imidazol-4-propionamid und 3-(3-Indolyl)propionsäure das (S)-N-[(1S,2S,4S)-1-(Cyclohexylmethyl)-2-hydroxy-4-isopropyl-5-hexenyl]-α-(3-indol-3-ylpropionamido)imidazol-4-propionamid als Festkörper vom Schmelzpunkt 122° (aus Aether/Hexan);

- Aus (S)-α-Amino-N-[(1S,2S,4S)-1-(cyclohexylmethyl)-2-hydroxy-4-isopropyl-5-hexenyl]imidazol-4-propionamid und Benzylmalonsäuremonoamid das (S)-α-[(RS)-α-Carbamoylhydrocinnamamido]-N-[(1S,2S,4S)-1-(cyclohexylmethyl)-2-hydroxy-4-isopropyl-5-hexenyl]imidazol-4-propionamid als Schaum, MS: 566 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und (S)-α-Benzyl-2,5-dimethylpyrrol-1-essigsäure das (S)-N-[(1S,2S,4S)-1-(Cyclohexylmethyl)-2-hydroxy-4-isopropyl-5-hexenyl]-α-[(S)-α-(2,5-dimethylpyrrol-1-yl)hydrocinnamamido]imidazol-4-propionamid als Kristalle vom Schmelzpunkt 93° (aus Aether/Hexan);

- Aus (S)-α-Amino-N-[(1S,2S,4S)-1-(cyclohexylmethyl)-2-hydroxy-4-isopropyl-5-hexenyl]imidazol-4-propionamid und α-[(2-Hydroxyäthyl)carbamoyl]hydrozimtsäure das (S)-N-[(1S,2S,4S)-1-(Cyclohexylmethyl)-2-hydroxy-4-isopropyl-5-hexenyl]-α-[(RS)-α-[(2-hydroxyäthyl)carbamoyl]hydrocinnamamido]imidazol-4-propionamid als Schaum, MS: 610 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und α-[[2-(Dimethylamino)äthyl]carbamoyl]hydrozimtsäure das (S)-N-[(1S,2S,4S)-1-(Cyclohexylmethyl)-2-hydroxy-4-isopropyl-5-hexenyl-α-[(RS)-α-[[2-(dimethylamino)äthyl]carbamoyl]hydrocinnamamido]imidazol-4-propionamid als Schaum, MS: 637 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und Dibenzyl-cyanessigsäure das (S)-α-(α-Benzyl-α-cyanohydrocinnamamido)-N-[(1S,2S,4S)-1-(cyclohexylmethyl)-2-hydroxy-4-isopropyl-5-hexenyl]imidazol-4-propionamid als Kristalle vom Schmelzpunkt 105° (aus Aether/Hexan);

- Aus (S)-α-Amino-N-[(1S,2S,4S)-1-(cyclohexylmethyl)-2-hydroxy-4-isopropyl-5-hexenyl]imidazol-4-propionamid und Benzyl-cyanessigsäure das (RS)-α-Cyan-N-[(S)-1-[[(1S,2S,4S)-1-(cyclohexylmethyl)-2-hydroxy-4-isopropyl-5-hexenyl]carbamoyl]-2-imidazol-4-yläthyl]hydrocinnamamid als Kristalle vom Schmelzpunkt 97° (aus Methylenchlorid/Aether/Hexan);

- Aus (S)-α-Amino-N-[(1S,2S,4S)-1-(cyclohexylmethyl)-2-hydroxy-4-isopropyl-5-hexenyl]imidazol-4-propionamid und Indol-2-carbonsäure das N-[(S)-1-[[(1S,2S,4S)-1-(Cyclohexylmethyl)-2-hydroxy-4-isopropyl-5-hexenyl]carbamoyl]-2-imidazol-4-yläthyl]-2-benzimidazolcarboxamid als Kristalle vom Schmelzpunkt 123° (aus Methylenchlorid/Aether/Hexan);

- Aus (S)-α-Amino-N-[(1S,2S,4S)-1-(cyclohexylmethyl)-2-hydroxy-4-isopropyl-5-hexenyl]imidazol-4-propionamid und Isovaleriansäure das (S)-N-[(1S,2S,4S)-1-(Cyclohexylmethyl)-2-hydroxy-4-isopropyl-5-hexenyl]-α-(3-methylbutyramido)imidazol-4-propionamid als Schaum, MS: 622 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und α-Benzyl-β-oxo-1-pyrrolidinpropionsäure das N-[(1S,2S,4S)-1-(Cyclohexylmethyl)-2-hydroxy-4-isopropyl-5-hexenyl]-α-[(RS)-α-(1-pyrrolidinylcarbonyl)hydrocinnamamido]imidazol-4-propionamid als Kristalle vom Schmelzpunkt 89° (aus Methylenchlorid/Aether/Hexan);

- Aus (S)-α-Amino-N-[(1S,2S,4S)-1-(cyclohexylmethyl)-2-hydroxy-4-isopropyl-5-hexenyl]imidazol-4-propionamid und α-[(Benzyloxy)carbamoyl]hydrozimtsäure das (S)-α-[(RS)-α-[(Benzyloxy)carbamoyl]-hydrocinnamamido]-N-[(1S,2S,4S)-1-cyclohexyl-2-hydroxy-4-isopropyl-5-hexenyl]imidazol-4-propionamid als Kristalle vom Schmelzpunkt 104° (aus Methylenchlorid/Aether/Hexan);
- Aus (S)-α-Amino-N-[(1S,2S,4S)-1-(cyclohexylmethyl)-2-hydroxy-4-isopropyl-5-hexenyl]imidazol-4-propionamid und 1-[α-(Methoxycarbonyl)hydrocinnamoyl]-4-piperidincarbonsäure das Methyl (RS)-α-[[4-[[(S)-1-[[(1S,2S,4S)-1-(cyclohexylmethyl)-2-hydroxy-4-isopropyl-5-hexenyl]carbamoyl]-2-imidazol-4-yläthyl]-carbamoyl]piperidino]carbonyl]hydrocinnamat als Kristalle vom Schmelzpunkt 89° (aus Methylenchlorid/Aether/Hexan).

Die als Ausgangsstoffe eingesetzten Säuren sind entweder bekannt oder wurden wie folgt hergestellt: α-Benzyltetrahydro-β-oxo-4H-1,4-oxazinpropionsäure Benzylmalonsäuremonomethylester, welcher seinerseits nach bekannten Methoden (M. Goodman et al., J. Am. chem. Soc., 77, 8675 (1969) und Int. J. Pept. Prof. Res., 21, 84 (1983)) hergestellt worden war, wird nach üblichen Methoden mit Morpholin amidiert und anschliessend mit 1N Natronlauge zu α-Benzyltetrahydro-β-oxo-4H-1,4-oxazinpropionsäure hydrolysiert, MS: 264 $(M+H)^+$.

(S)-α-[[(S)-2-Benzylamino-3-phenylpropyl]amino]hydrozimtsäure

In analoger Weise zur in Beispiel 26 beschriebenen Herstellung von Boc-Phe$\overset{R}{-}$His(Bom)-OH wurde N-Benzyl-Phe-Phe-OMe durch Schwefelung der Amidcarbonylgruppe, Entschwefelung derselben mit Raney-Nickel und anschliessende Hydrolyse des erhaltenen Esters die (S)-α-[[(S)-2-Benzylamino-3-phenylpropyl]-amino]hydrozimtsäure hergestellt, welche direkt in die nächste Stufe eingesetzt wird.

t-Butoxycarbonylaminovaleriansäure

5-Aminovaleriansäure wird unter Schotten-Baumann-Bedingungen mit Di-t-butylcarbonat in üblicher Weise in die entsprechende N-geschützte Säure übergeführt, Schmelzpunkt 48-50° (aus Aether/Hexan).

Benzylmalonsäuremonoamid

Benzylmalonsäuremonomethylester wird mit Ammoniak in üblicher Weise amidiert und der erhaltene 2-Benzyl-2-formamidoessigsäuremethylester mit 1N Natronlauge in einem 9:1-Gemisch von Methanol und Wasser innerhalb von 30 Minuten zum Benzylmalonsäuremonoamid hydrolysiert, Rf: 0,15 in einem 90:10:1:0,5-Gemisch von Chloroform, Methanol, Wasser und Essigsäure.

(S)-α-Benzyl-2,5-dimethylpyrrol-1-essigsäure

L-Phenylalanylmethylester-hydrochlorid wird durch Ausschütteln mit einer Natriumbicarbonatlösung in Aether in die entsprechende freie Base übergeführt und in Eisessig mit Acetonylaceton 2,5 Stunden auf 100° erhitzt. Danach wird das Lösungsmittel abgedampft, und der Rückstand mit Toluol über Kieselgel filtriert, wobei man Methyl (S)-α-benzyl-2,5-dimethylpyrrol-1-acetat erhält, MS: 258 $(M+H)^+$. Der erhaltene Ester wird während 1,5 Stunden bei Raumtemperatur mit 1N Natronlauge in einem 9:1-Gemisch von Methanol und Wasser stehengelassen. Nach Extraktion der wässrigen Lösung mit Aether und Waschen der organischen Phasen erhält man (S)-α-Benzyl-2,5-dimethylpyrrol-1-essigsäure, MS: 244 $(M+H)^+$, welche direkt in die nächste Stufe eingesetzt wird.

α-[(2-Hydroxyäthyl)carbamoyl]hydrozimtsäure

In analoger Weise wie weiter oben für α-Benzyltetrahydro-β-oxo-4H-1,4-oxazinpropionsäure beschrieben wird Benzylmalonsäuremonoäthylester mit Aethanolamin amidiert und die erhaltene Verbindung zu α-[-(2-Hydroxyäthyl)carbamoyl]hydrozimtsäure verseift, MS: 251 $(M)^+$, welche direkt in die nächste Stufe eingesetzt wird.

α-[[2-(Dimethylamino)äthyl]carbamoyl]hydrozimtsäure

In analoger Weise wie weiter oben für α-Benzyltetrahydro-β-oxo-4H-1,4-oxazinpropionsäure beschrieben wird Benzylmalonsäuremonomethylester mit N,N-Dimethylaminoäthylamin amidiert und anschliessend zu α-[[2-(Dimethylamino)äthyl]carbamoyl]hydrozimtsäure verseift, [MS: 265 $(M+H)^+$], die direkt in die nächste Stufe eingesetzt wird.

Dibenzyl-cyanessigsäure

Cyanessigsäureäthylester wird in Gegenwart von Natriumäthylat mit überschüssigem Benzylbromid in Aethanol zum Dibenzyl-cyanoessigsäureäthylester dibenzyliert, welcher mit 1N Natronlauge in einem 9:1-Gemisch von Methanol und Wasser zur Dibenzyl-cyanessigsäure hydrolysiert wird. Schmelzpunkt 194° (aus Methylenchlorid/Methanol/Hexan).

Benzyl-cyanessigsäure

Cyanessigsäureäthylester wird mit Benzylbromid in Gegenwart von Natriumäthylat in Aethanol zum Benzyl-cyanessigsäureäthylester [MS: $203(M)^+$] alkyliert, welcher dann anschliessend mit 1N Natronlauge in einem 9:1-Gemisch von Methanol und Wasser zur entsprechenden Säure hydrolysiert wird. Umkristallisation des Rohprodukts aus Methylenchlorid/Hexan liefert Benzyl-cyanessigsäure als Kristalle vom Schmelzpunkt 97°.

α-Benzyl-β-oxo-1-pyrrolidinpropionsäure

In analoger Weise wie für α-Benzyltetrahydro-β-oxo-4H-1,4-oxazinpropionsäure beschrieben, wird Benzylmalonsäuremonomethylester mit Pyrrolidin amidiert und das erhaltene Produkt zur α-Benzyl-β-oxo-1-pyrrolidinpropionsäure hydrolysiert, welche direkt in die nächste Stufe eingesetzt wird, MS: 248 $(M+H)^+$.

α-[(Benzyloxy)carbamoyl]hydrozimtsäure

In analoger Weise wie für α-Benzyltetrahydro-β-oxo-4H-1,4-oxazinpropionsäure beschrieben, wird Benzylmalonsäuremonomethylester mit O-Benzylhydroxylamin amidiert und das erhaltene Produkt [MS: 314-$(M+H)^+$] mit 1N Natronlauge in einem 9:1-Gemisch von Methanol und Wasser zur entsprechenden Säure verseift wird. Umkristallisation des Rohprodukts aus Methanol/Methylenchlorid/Aether liefert α-[(Benzyloxy)-carbamoyl]hydrozimtsäure vom Schmelzpunkt 147°.

1-[α-(Methoxycarbonyl)hydrocinnamoyl]-4-piperidincarbonsäure

In analoger Weise wie für die Herstellung von α-Benzyltetrahydro-β-oxo-4H-1,4-oxazinpropionsäure beschrieben, wird Benzylmalonsäuremonomethylester mit Piperidin-4-carbonsäureäthylester amidiert und die erhaltene Verbindung [MS: $347(M)^+$] mit 1N Natronlauge in einem Gemisch von Methanol und Wasser 2 Stunden bei 0° verseift. Das erhaltene Rohprodukt wird durch Chromatographie an Silicagel mit einem 95:5:2-Gemisch von Chloroform, Aethanol und Essigester gereinigt und aus einem Gemisch von Methanol, Methylenchlorid und Aether umkristallisiert, wobei man 1-[α-(Methoxycarbonyl)hydrocinnamoyl]-4-piperidincarbonsäure erhält, Schmelzpunkt 138°.

## Beispiel 7

In analoger Weise wie in Beispiel 1 beschrieben wird N-[(1S,2S,4S)-1-(Cyclohexylmethyl)-2-hydroxy-4-isopropyl-5-hexenyl]-α-(3-phenyl-L-alanyl)imidazol-4-propianamid mit Di-t-butoxycarbonyl-aminoäthylglycin umgesetzt. Chromatographische Reinigung und Umkristallisation des Rohprodukts liefert Di-t-butyl N-[[[(S)-

α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamoyl]methyl]äthylendicarbamat, Schmelzpunkt 109°.

150 mg N-[[[(S)-α[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamoyl]methyl]äthylendicarbamat werden in 0,3N Salzsäure in Methanol gelöst und über Nacht bei Raumtemperatur stehengelassen. Danach wird das Lösungsmittel abgedampft, und der Rückstand aus Methanol/Essigester gefällt, wobei man (S)-α-[(S)-α-[2-[(2-Aminoäthyl)-amino]acetamido]hydrocinnamamido]-N-[(1S,2S,4S)-1-(cyclohexylmethyl)-2-hydroxy-4-isopropyl-5-hexenyl]-imidazol-4-propionamid-trihydrochlorid erhält, MS: 638(M+H)$^+$.

Das als Ausgangsmaterial eingesetzte Di-t-butoxycarbonylaminoäthylglycin wurde wie folgt hergestellt: N-(2-Aminoäthyl)glycin wird nach der von E.P. Heimer in Int. J. Pept. Prot. Res., 23 203 (1984) beschriebenen Methode hergestellt und in bekannter Weise unter Schotten-Baumann-Bedingungen mit Di-t-butylcarbonat in die entsprechende N-geschützte Säure übergeführt, MS: 319 (M+H)$^+$.

## Beispiel 8

60 mg (2S,3S,5S)-2-(Boc-D-Pro-Phe-His-NH)-1-cyclohexyl-5-isopropyl-6-hepten-3-ol werden in 10 ml 2,1N Chlorwasserstoff in Dioxan gelöst und 1,5 Stunden bei Raumtemperatur gerührt. Dann wird das Lösungsmittel abgedampft, und der Rückstand zweimal nacheinander zunächst mit Toluol versetzt und danach wieder zur Trockene eingedampft. Kristallisation des so erhaltenen Rückstands aus Methylenchlorid/Aether/Hexan liefert 40 mg (S)-N-[(1S,2S,4S)-1-(Cyclohexylmethyl)-2-hydroxy-4-isopropyl-5-hexenyl]-α-[(S)-α-(D-prolylamino)hydrocinnamamido]imidazol-4-propionamid-dihydrochlorid als weisse Kristalle vom Schmelzpunkt 159°.

## Beispiel 9

N-Methyl-L-histidin wird mit Benzylalkohol in Gegenwart von Salzsäure bei Raumtemperatur in den entsprechenden Benzylester übergeführt und dieser nach dem von D.H. Rich et al. in Proc. 9th American Pept. Symp., Toronto 1985, Seite 217, beschriebenen Verfahren mit t-Butoxycarbonyl-L-phenylalanin in üblicher Weise umgesetzt. Danach wird das erhaltene N-[N-t-Butoxycarbonyl)-3-phenyl-L-alanyl]-N-methyl-L-histidinbenzylester eine Stunde in Gegenwart von 5%igem Palladium auf Kohle hydriert, wobei man N-[N-(t-Butoxycarbonyl)-3-phenyl-L-alanyl]-N-methyl-L-histidin erhält, Rf: 0,2 in einem 80:20:3:3-Gemisch von Chloroform, Methanol, Wasser und Essigsäure. Diese Verbindung wird dann in üblicher Weise mit (αS, S)--Amino-α-[(S)-2-isopropyl-3-butenyl]cyclohexanpropanol umgesetzt. Das erhaltene Rohprodukt wird dann mit einem 20:1:0,1-Gemisch von Methylenchlorid, Methanol und Ammoniak an Kieselgel chromatographiert, wodurch die beiden epimeren Verbindungen aufgetrennt werden. Umkristallisation des aus der ersten Fraktion erhaltenen Rohprodukts aus Aether/Methy lenchlorid/Hexan liefert t-Butyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]methylcarbamoyl]-phenäthyl]carbamat als weisse Kristalle vom Schmelzpunkt 95°.

Aus der zweiten Fraktion erhält man nach Umkristallisieren aus Aether/Methylenchlorid/Hexan die epimere Verbindung t-Butyl [(S)-α-[[(R)-1-[[(1S,2S,4S)-1-(cyclohexylmethyl)-2-hydroxy-4-isopropyl-5-hexenyl]carbamoyl]-2-imidazol-4-yläthyl]methylcarbamoyl]phenäthyl]carbamat als weisse Kristalle vom Schmelzpunkt 85°.

In analoger Weise wie oben beschrieben wird N-t-Butoxycarbonyl-N-methyl-L-phenylalanin nach der von Rich et al. beschriebenen Methode mit N-Methyl-L-histidinbenzylester umgesetzt, der erhaltene Ester anschliessend zur entsprechenden Säure hydriert, und diese in üblicher Weise mit (αS, βS)-β-Amino-α-[(S)-2-isopropyl-3-butenyl]cyclohexanpropanol umgesetzt. Chromatographische Reinigung des Rohprodukts an Kieselgel mit einem 140:1:0,1-Gemisch von Methylenchlorid, Methanol und Ammoniak liefert dünnschichtchromatographisch reines t-Butyl [(S)-α-[[(S)-α-[[(1S,2S,4S)-1-(cyclohexylmethyl) -2-hydroxy-4-isopropyl-5-hexenyl]carbamoyl]-2-imidazol-4-yläthyl]methylcarbamoyl]phenäthyl]methylcarbamat in Form eines Schaums, MS: 666(M+H)$^+$.

## Beispiel 10

150 mg (0,20 mMol) t-Butyl 2-benzyl-2-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-3-phenylpropyl]carbamat werden in 10 ml 0,3N methanolischer Salzsäure gelöst und über Nacht bei Raumtemperatur stehenge lassen. Danach wird das Lösungsmittel abgedampft und der Rückstand zur Reinigung mit einem 20:1:0,1-Gemisch von Methylenchlorid, Methanol und Ammoniak als Eluierungsmittel an 30 g Kieselgel chromatographiert. Umkristallisation des so erhaltenen Rohprodukts aus Methylenchlorid/Aether/Hexan liefert (S)-α-(3-Amino-2,2-dibenzylpropionamido)-N-[(1S,2S,4S)-1-(cyclohexylmethyl)-2-hydroxy-4-isopropyl-5-hexenyl]imidazol-4-propionamid als weisse Kristalle vom Schmelzpunkt 89°.

Das als Ausgangsmaterial eingesetzte t-Butyl 2-benzyl-2-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-3-phenylpropyl]carbamat wurde wie folgt hergestellt:

Dibenzylcyanessigsäuremethylester wird mit Raney-Nickel in methanolischem Ammoniak zu Dibenzylmethylaminoessigsäureäthylester reduziert [MS: 284(M)$^+$], der dann mit Di-t-butylcarbonat in Triäthylamin in üblicher Weise an der Aminogruppe geschützt wird. Der auf diese Weise erhaltene Ester [MS: 398-(M+H)$^+$] wird anschliessend mit 1N Natronlauge in einem Gemisch von Methanol und Wasser zur entsprechenden Säure verseift, welche nach Umkristallisation aus Methanol/Methylenchlorid/Hexan als Kristalle vom Schmelzpunkt 157° isoliert wird. Die Säure wird danach auf übliche Weise mit (S)-α-Amino-N-[(1S,2S,4S)-1-(cyclohexylmethyl)-2-hydroxy-4-isopropyl-5-hexenyl]imidazol-4-propionamid zu t-Butyl 2-benzyl-2-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-3-phenylpropyl]carbamat umgesetzt. Umkristallisation aus Methylenchlorid/Hexan/Aether liefert weisse Kristalle vom Schmelzpunkt 97°.

## Beispiel 11

150 mg (S)-α-[(RS)-α-[(Benzyloxy)carbamoyl]hydrocinnamamido]-N-[(1S,2S,4S)-1-cyclohexyl-2-hydroxy-4-isopropyl-5-hexenyl]imidazol-4-propionamid werden in Gegenwart von 5%igem Palladium auf Kohle in Methanol 3 Stunden hy- driert. Nach beendeter Wasserstoffaufnahme wird der Kataly- sator abfiltriert, und das Filtrat eingedampft. Umkristallisation des Rückstands aus Methanol/Methylenchlorid/Aether liefert (S)-N-[(1S,2S,4S)-1-(Cyclohexylmethyl)-4-äthyl-2-hydroxy-5-methylhexyl]-α-[(RS)-α-(hydroxycarbamoyl)-hydrocinnamamido]imidazol-4-propionamid als weisse Kristalle vom Schmelzpunkt 123°.

## Beispiel 12

120 mg t-Butyl [(S)-α-[[(S)-1-(S)-[[(1S,2S,4S)-1-(cyclohexylmethyl)-2-hydroxy-4-isopropyl-5-hexenyl]-carbamoyl]-2-imidazol-4-yläthyl]carbamoyl]phenäthyl]methylcarbamat werden in 0,3N methanolischer Salzsäure gelöst und über Nacht bei Raumtemperatur gerührt. Danach wird das Lösungsmittel unter vermindertem Druck abgedampft, und der Rückstand mit t-Butylacetylchlorid und Triäthylamin in Tetrahydrofuran versetzt und über Nacht bei Raumtemperatur stehengelassen. Nach üblicher Aufarbeitung, chromatographischer Reinigung an Kieselgel unter Verwendung eines 20:1:0,1-Gemisches von Methylenchlorid, Methanol und Ammoniak und Umkristallisation aus Aether/Hexan erhält man (S)-N-[(1S,2S,4S)-1-(Cyclohexylmethyl)-2-hydroxy-4-isopropyl-5-hexenyl]-α-[(S)-α-(N,3,3-trimethylbutyramido)hydrocinnamamido]imidazol-4-propionamid als weisse Kristalle vom Schmelzpunkt 126°.

## Beispiel 13

In analoger Weise wie in Beispiel 1 beschrieben wurden die folgenden Verbindungen hergestellt:

- Aus N-[(1S,2S,4S)-1-(Cyclohexylmethyl)-2-hydroxy-4-isopropyl-5-hexenyl]-α-(3-phenyl-L-alanyl)imidazol-4-propionamid und 3-(4-Hydroxyphenyl)propionsäure das (S)-N-[(1S,2S,4S)-1-(Cyclohexylmethyl)-2-hydroxy-4-isopropyl-5-hexenyl]-α-[(S)-α-(p-hydroxyhydrocinnamamido)hydrocinnamamido]imidazol-4-propionamid als weisse Kristalle, Schmelzpunkt 120° (aus Methylenchlorid/Aether/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und Nicotinsäure das (S)-N-[(1S,2S,4S)-1-(Cyclohexylmethyl)-2-hydroxy-4-isopropyl-5-

hexenyl]-α-[(S)-α-nicotinamidohydrocinnamamido]imidazol-4-propionamid als Festkörper, MS: 643(M + H)$^+$;

- Aus N-[(1S,2S,4S)-1-(Cyclohexylmethyl)-2-hydroxy-4-isopropyl-5-hexenyl]-α-(3-phenyl-L-alanyl)imidazol-4-propionamid und 3-(4-Pyridyl)acrylsäure das (S)-N-[(1S,2S,4S)-1-(Cyclohexylmethyl)-2-hydroxy-4-isopropyl-5-hexenyl]-α-[[(S)-α-[(E)-4-pyridinacrylamido]hydrocinnamido]imidazol-4-propionamid als gelbliche Kristalle, Schmelzpunkt 135° (Zers.; aus Methylenchlorid/Aether/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und 3-(3-Pyridyl)acrylsäure das (S)-[(1S,2S,4S)-1-(Cyclohexylmethyl)-2-hydroxy-4-isopropyl-5-hexenyl]-α-[(S)-α-(3-pyridinacrylamido)hydrocinnamamido]imidazol-4-propionamid als gelbliche Kristalle, Schmelzpunkt 141° (aus Methylenchlorid/Aether);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und 3-(4-Imidazolyl)acrylsäure (Urocaninsäure) das (S)-N-[(1S,2S,4S)-1-(Cyclohexylmethyl)-2-hydroxy-4-isopropyl-5-hexenyl]-α-[(S)-α-(imidazol-4-acrylamido)hydrocinnamamido]imidazol-4-propionamid als weisse Kristalle, Schmelzpunkt 150-152° (aus Methylenchlorid/Methanol/Aether);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und D-(-)-Chinasäure das (S)-N-[(1S,2S,4S)-1-(Cyclohexylmethyl)-2-hydroxy-4-isopropyl-5-hexenyl]-α-[(S)-α-(1,3,4,5-tetrahydroxycyclohexancarboxamido)hydrocinnamamido]imidazol-4-propionamid als weisser Festkörper, MS: 712 (M + H)$^+$;
- Aus N-[(1S,2S,4S)-1-(Cyclohexylmethyl)-2-hydroxy-4-isopropyl-5-hexenyl]-α-(3-phenyl-L-alanyl)imidazol-4-propionamid und Chinaldinsäure das N-[(S)-α-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]phenäthyl]-2-chinolincarboxamid als weisser Festkörper, MS: 693 (M + H)$^+$;
- Aus N-[(1S,2S,4S)-1-(Cyclohexylmethyl)-2-hydroxy-4-isopropyl-5-hexenyl]-α-(3-phenyl-L-alanyl)imidazol-4-propionamid und Lävulinsäure das (S)-N-[(1S,2S,4S)-1-(Cyclohexylmethyl)-2-hydroxy-4-isopropyl-5-hexenyl]-α-[(S)-α-(4-oxavaleramido)hydrocinnamamido]imidazol-4-propionamid als weisse Kristalle, Schmelzpunkt 150° (Zers.; aus Methylenchlorid/Aether/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und 3-Pyridylessigsäure das (S)-N-[(1S,2S,4S)-1-(Cyclohexylmethyl)-2-hydroxy-4-isopropyl-5-hexenyl]-α-[(S)-α-(3-pyridinacetamido)hydrocinnamamido]imidazol-4-propionamid als weisse Kristalle, Schmelzpunkt 195° (Zers.; aus Methanol/Methylenchlorid/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und 4-Chlorzimtsäure das (S)-N-[(1S,2S,4S)-1-(Cyclohexylmethyl)-2-hydroxy-4-isopropyl-5-hexenyl]-α[(S)-α-(4-chlorocinnamamido)hydrocinnamamido]imidazol-4-propionamid als weisse Kristalle, Schmelzpunkt 139° (aus Methylenchlorid/Methanol/Aether);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und trans-4-Nitrozimtsäure das (S)-N-[(1S,2S,4S)-1-(Cyclohexylmethyl)-2-hydroxy-4-isopropyl-5-hexenyl]-α-[(S)-α-(4-nitrocinnamamido)hydrocinnamamido]imidazol-4-propionamid als gelbliche Kristalle, Schmelzpunkt 120° (Zers.; aus Methylenchlorid/Methanol/Aether);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und Fumarsäuremonoäthylester das Aethyl 3-[[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamoyl]acrylat als weisse Kristalle, Schmelzpunkt 175° (Zers.; aus Methylenchlorid/Methanol/Hexan);
- Aus (S)-α-Amino-N-[(1S,2S,4S)-1-(cyclohexylmethyl)-2-hydroxy-4-isopropyl-5-hexenyl]imidazol-4-propionamid und Diphenylcarbamoyl-L-phenylalanin ein Epimerengemisch aus (S) -N-[(1S,2S,4S)-1-(Cyclohexylmethyl)-2-hydroxy-4-isopropyl-5-hexenyl]-α-[[N-(diphenylcarbamoyl)-3-phenyl-L-alanyl]amino]-imidazol-4-propionamid und (S)-N-[(1S,2S,4S)-1-(Cyclohexylmethyl)-2-hydroxy-4-isopropyl-5-hexenyl]-α-[[N-(diphenylcarbamoyl)-3-phenyl-D-alanyl]amino]imidazol-4-propionamid, wobei das erstgenannte Epimere überwiegt, Schmelzpunkt für beide Epimeren 106° (aus Methylenchlorid/Aether/Hexan);
- Aus (S)-α-Amino-N-[(1S,2S,4S)-1-(cyclohexylmethyl)-2-hydroxy-4-isopropyl-5-hexenyl]imidazol-4-propionamid und Isobutoxycarbonyl-L-phenylalanin das Isobutyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamat als weisse Kristalle, Schmelzpunkte 147° (aus Aether/Methylenchlorid/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und D-Pyroglutaminsäure das (S)-N-[(1S,2S,4S)-1-[Cyclohexylmethyl]-2-hydroxy-4-isopropyl-5-hexenyl]-α-[(S)-α-[[5-oxo-D-prolyl]amino]hydrocinnanamido]imidazol-4-propionamid als weisser Festkörper, Schmelzpunkt 200° (Zers.; aus Methanol/Essigester/Hexan);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und L-Pyroglutaminsäure das (S)-N-[(1S,2S,4S)-1-[Cyclohexylmethyl]-2-hydroxy-4-isopropyl-5-hexenyl]-α-[(S)-α-[[5-oxo-L- prolyl]amino]hydrocinnamamido]imidazol-4-propionamid als weisse Kristalle,

Schmelzpunkt 148° (aus Methylenchlorid/Methanol/Aether);

- Aus N-[(1S,2S,4S)-1-(Cyclohexylmethyl)-2-hydroxy-4-isopropyl-5-hexenyl]-α-(3-phenyl-L-alanyl)imidazol-4-propionamid und N-t-Butoxycarbonyl-α-methyl-L-alanin das t-Butyl [1-[[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]-carbamoyl]-1-methyläthyl]carbamat als weisse Kristalle, Schmelzpunkt 112° (aus Methylenchlorid/Tetrahydrofuran/Hexan);

- Aus N-[(1S,2S,4S)-1-(Cyclohexylmethyl)-2-hydroxy-4-isopropyl-5-hexenyl]-α-(3-phenyl-L-alanyl)imidazol-4-propionamid und Fmoc-Ser(t-Bu)OH das 9H-Fluoren-9-yl[(S)-2-t-butoxy-1-[[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]-carbamoyl]äthyl]carbamat als weisser Festkörper, Schmelzpunkt 180° (Zers.; aus Methylenchlorid/Aether/Hexan);

- Aus N-[(1S,2S,4S)-1-(Cyclohexylmethyl)-2-hydroxy-4-isopropyl-5-hexenyl]-α-(3-phenyl-L-alanyl)imidazol-4-propionamid und Dibenzylsuberanessigsäure das (S)-N-[(1S,2S,4S)]-1-(Cyclohexylmethyl)-2-hydroxy-4-isopropyl-5-hexenyl]-α-[[N-(9H-fluoren-9-ylacetyl)-3-phenyl-L-alanyl]amino]imidazol-4-propionamid als weisse Kristalle, Schmelzpunkt 119° (aus Methylenchlorid/Aether/Hexan);

- Aus N-[(1S,2S,4S)-1-(Cyclohexylmethyl)-2-hydroxy-4-isopropyl-5-hexenyl]-α-(3-phenyl-L-alanyl)imidazol-4-propionamid und Adamantylessigsäure das (S)-α-[[N-(1-Adamantylacetyl)-3-phenyl-L-alanyl]amino]-N-[-(1S,2S,4S)-1-[cyclohexylmethyl)-2-hydroxy-4-isopropyl-5-hexenyl]imidazol-4-propion amid als weisse Kristalle, Schmelzpunkt 120° (aus Methylenchlorid/Aether/Hexan);

- Aus (S)-α-Amino-N-[(1S,2S,4S)-1-(cyclohexylmethyl)-2-hydroxy-4-isopropyl-5-hexenyl]imidazol-4-propionamid und Phenyloxycarbonyl-L-phenylalanin das Benzyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamat als weisse Kristalle, Schmelzpunkt 144° (aus Methylenchlorid/Aether);

- Aus (S)-α-Amino-N-[(1S,2S,4S)-1-(cyclohexylmethyl)-2-hydroxy-4-isopropyl-5-hexenyl]imidazol-4-propionamid und 2,2,2-Trichloräthoxycarbonyl-L-phenylalanin das 2,2,2-Trichloräthyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]-carbamat als weisse Kristalle, Schmelzpunkt 109° (aus Methylenchlorid/Hexan);

- Aus N-[(1S,2S,4S)-1-(Cyclohexylmethyl)-2-hydroxy-4-isopropylhexyl]-α-[(3-phenyl-N-D-prolyl-L-alanyl)-amino]imidazol-4-propionamid und 4-Imidazolylpropionsäure, welche ihrerseits durch Hydrierung von Urocaninsäure hergestellt wurde, das N-[(1S,2S,4S)-1-(Cyclohexylmethyl)-2-hydroxy-4-isopropylhexyl]-α-[(S)-α-[-(R)-1-(3-imidazol-4-ylpropionyl]-2-pyrrolidincarboxamido]hydrocinnamamido]imidazol-4-propionamid in Form eines Schaums, MS: 760 $(M+H)^+$;

- Aus N-[(1S,2S,4S)-1-(Cyclohexylmethyl)-2-hydroxy-4-isopropyl-5-hexenyl]-α-(3-phenyl-L-alanyl)imidazol-4-propionamid und (R)-1-[(3-Hydroxy-2-pyridyl)carbonyl]-2-pyrrolidincarbonsäure das (S)-N-[(1S,2S,4S)-1-(Cyclohexylmethyl)-2-hydroxy-4-isopropyl-5-hexenyl]-α-[(S)-α-[(R)-1-[(3-hy droxy-2-pyridyl)carbonyl]-2-pyrrolidincarboxamido]hydrocinnamamido]imidazol-4-propionamid als weisser Festkörper, Schmelzpunkt 198° (Zers.; aus Methylenchlorid/Methanol/Aether/Hexan);

- Aus N-[(1S,2S,4S)-1-(Cyclohexylmethyl)-2-hydroxy-4-isopropyl-5-hexenyl]-α-(3-phenyl-L-alanyl)imidazol-4-propionamid und (R)-1-[(Dibenzylacetyl)-2-pyrrolidincarbonsäure das (S)-N-[(1S,2S,4S)-1-(Cyclohexylmethyl)-2-hydroxy-4-isopropyl-5-hexenyl]-α-[(S)-α-[(R)-1-(dibenzylacetyl)-2-pyrrolidincarboxamido]hydrocinnamamido]imidazol-4-propionamid als weisse Kristalle, Schmelzpunkt 104° (aus Methylenchlorid/Aether/Hexan);

- Aus N-[(1S,2S,4S)-1-(Cyclohexylmethyl)-2-hydroxy-4-isopropyl-5-hexenyl]-α-(3-phenyl-L-alanyl)imidazol-4-propionamid und 1-t-Butoxycarbonylaminocyclohexancarbonsäure das t-Butyl 1-[[(S)-α-[[(S)-1-[(1S,2S,4S)-1-(cyclohexylmethyl-2-hydroxy-4-isopropyl-5-hexenyl]-2-imidazol-4-yläthyl]carbamoyl]phenäthyl]carbamoyl]-cyclohexancarbamat als weisser Festkörper, MS: 764 $(M+H)^+$;

- Aus N-[(1S,2S,4S)-1-(Cyclohexylmethyl)-2-hydroxy-4-isopropylhexyl]-α-[(3-phenyl-N-D-prolyl-L-alanyl)-amino]imidazol-4-propionamid und Isovaleriansäure das (S)-N-[(1S,2S,4S)-1-(Cyclohexylmethyl)-2-hydroxy-4-isopropylhexyl]-α-[(S)-α-[(R)-1-isovaleryl-2-pyrrolidincarboxamido]hydrocinnamamido]imidazol-4-propionamid als Schaum, MS: 721 $(M+H)^+$;

- Aus (S)-N-[(1S,2S,4S)-1-(Cyclohexylmethyl)-2-hydroxy-4-isopropyl-5-hexenyl]-α-[(S)-α-(D-prolylamino)-hydrocinnamamido]imidazol-4-propionamid dihydrochlorid und Isovaleriansäure das (S)-N-[(1S,2S,4S)-1-(Cyclohexylmethyl)-2-hydroxy-4-isopropyl-5-hexenyl]-α-[[(S)-α-[(1-isovaleryl-L-propyl)carbonyl]-3-phenyl-L-alanyl]amino]imidazol-4-propionamid als Schaum, MS: 719 $(M+H)^+$.

Das als Ausgangsmaterial eingesetzte N-[(1S,2S,4S)-1-(Cyclohexylmethyl)-2-hydroxy-4-isopropylhexyl]-α-[(3-phenyl-N-D-prolyl-L-alanyl)amino]imidazol-4-propionamid wurde aus dem in Beispiel 16 beschriebenen t-Butyl (R)-2-[[(S)-α-[[(S)-1-[(1S,2S,4S)-1-(cyclohexylmethyl)-2-hydroxy-4-isopropylhexyl]-2-imidazol-4-yläthyl]carbamoyl]phenäthyl]carbamoyl]-1-pyrrolidincarboxylat hergestellt, indem die t-Butoxycarbonylgruppe in analoger Weise wie in Beispiel 8 beschrieben mit Chlorwasserstoff in Dioxan abgespalten wurde.

Die als Ausgangsstoffe eingesetzten Säuren sind entweder bekannt oder wurden wie folgt hergestellt:

Diphenylcarbamoyl-L-phenylalanin

Behandeln von L-Phenylalanin mit Diphenylcarbamoylchlorid in Dimethylformamid in Gegenwart von Triäthylamin liefert nach Umkristallisation aus Methanol/Methylenchlorid/Hexan die erwünschte Säure in Form von weissen Kristallen, Schmelzpunkt 167°.

Isobutoxycarbonyl-L-phenylalanin

Behandlung von L-Phenylalanin mit Isobutylchloroformiat in analoger Weise wie oben beschrieben liefert die erwünschte Säure als ein beim Stehenlassen auskristallisierendes Oel [MS: 266$(M+H)^+$], welches direkt in die nächste Stufe eingesetzt wird.

Phenyloxycarbonyl-L-phenylalanin

In analoger Weise wie oben beschrieben erhält man durch Umsetzen von L-Phenylalanin und Phenylchloroformiat die erwünschte Säure in Form eines Schaums [MS: 286$(M+H)^+$], welche direkt in die nächste Stufe eingesetzt wird.

2,2,2-Trichloräthoxycarbonyl-L-phenylalanin

L-Phenylalanin wird in einer 2N Nattriumbicarbonatlösung mit 2,2,2-Trichloräthylchlorformiat umgesetzt, wobei man nach üblicher Aufarbeitung die erwünschte Säure als Kristalle erhält, Schmelzpunkt 126° (aus Methylenchlorid/Hexan).

(R)-1-[(3-Hydroxy-2-pyridyl)carbonyl]-2-pyrrolidincarbonsäure

3-Hydroxypicolinsäure wird in üblicher Weise mit D-Prolinbenzylester kondensiert, und der erhaltene Ester danach hydrogenolytisch in die erwünschte Säure übergeführt, welche direkt in die nächste Stufe eingesetzt wird.

(R)-1-(Dibenzylacetyl)-2-pyrrolidincarbonsäure

Dibenzylessigsäure wird in üblicher Weise mit D-Prolinbenzylester kondensiert, und der erhaltene Ester hydrogenolytisch in die erwünschte Säure übergeführt, welche direkt in die nächste Stufe eingesetzt wird.

1-t-Butoxycarbonylaminocyclohexancarbonsäure

1-Aminocyclohexancarbonsäure wird in üblicher Weise mit Di-t-butyldicarbonat in die entsprechende N-geschützte Säure übergeführt, welche direkt in die nächste Stufe eingesetzt wird.

Beispiel 14

Man löst 40 mg 9H-Fluoren-9-yl [(S)-2-t-butoxy-1-[[(S)-α-[[(S) -1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamoyl]äthyl]-carbamat in 5 ml Methylenchlorid und 0,4 ml Piperidin und lässt 2 Stunden bei Raumtemperatur stehen. Danach dampft man das Reaktionsgemisch zur Trockene ein und chromatographiert den Rückstand an

Kieselgel mit einem 14:1:0,1-Gemisch von Methylenchlorid, Methanol und Ammoniak. Umkristallisation des so erhaltenen Rohprodukts liefert (S)-α-[[N-(3-t-Butoxy-L-alanyl)-3-phenyl-L-alanyl]amino]-N-[(1S,4S)-1-(cyclohexylmethyl)-2-hydroxy-4-isopropyl-5-hexenyl]imidazol-propionamid als weissen Festkörper, Schmelzpunkt 103° (Zers.; aus Methylenchlorid/Aether/Hexan).

## Beispiel 15

Man löst t-Butyl [4[[(S)-α-[[(S)-1-[[(1S,2S,4S)-1-(cyclohexylmethyl)-2-hydroxy-4-isopropyl-5-hexenyl]-carbamoyl]-2-imidazol-4-yläthyl]carbamoyl]phenäthyl]carbamoyl]butyl]carbamat in 0,3N methanolischer Salzsäure und lässt 18 Stunden bei Raumtemperatur stehen. Danach dampft man das Reaktionsgemisch zur Trockene ein und chromatographiert den Rückstand an Kieselgel, wobei man (S)-α-(5-Aminovaleramido)-N-[(S)-1-[[(1S,2S,4S)-1-(cyclohexylmethyl)-2-hydroxy-4-isopropyl-5-hexenyl]carbamoyl]-2-imidazol-4-yläthyl]hydrocinnamamid-dihydrochlorid als Schaum erhält, MS: 637 $(M+H)^+$.

In analoger Weise wie oben beschrieben, jedoch unter Verwendung von 3,5N Chlorwasserstoff in Essigsäure, erhält man aus t-Butyl (R)-2-[[(S)-α-[[(S)-1-[(1S,2S,4S)-1-(cyclohexylmethyl)-2-hydroxy-4-isopropylhexyl]-2-imidazol-4-yläthyl]carbamoyl]phenäthyl]carbamoyl]-1-pyrrolidincarboxylat, dessen Herstellung in Beispiel 16 beschrieben ist, das N-[(1S,2S,4S)-1-(Cyclohexylmethyl)-2-hydroxy-4-isopropylhexyl]-α-[(3-phenyl-N-D-prolyl-L-alanyl)amino]imidazol-4-propionamid als dünnschichtchromatographisch einheitlichen, hygroskopischen Festkörper, MS: 635 $(M+H)^+$.

## Beispiel 16

In analoger Weise wie in Beispiel 2 beschrieben wurden durch Hydrierung der entsprechenden Olefine die folgenden Verbindungen hergestellt:

- Aus Aethyl 3-[[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamoyl]acrylat das Aethyl [[(S)-α-[[(S)-1-[[(1S,2S,4S)-1-(cyclohexylmethyl)-2-hydroxy-4-isopropylhexyl]carbamoyl]-2-imidazol-4-yläthyl]carbamoyl]phenäthyl]-carbamoyl]propionat als dünnschichtchromatographisch einheitlicher Festkörper, MS: 668 $(M+H)^+$;
- Aus (S)-α-[[N-(1-Adamantylacetyl)-3-phenyl-L-alanyl]amino]-N-[(1S,2S,4S)-1-[cyclohexylmethyl)-2-hydroxy-4-isopropyl-5-hexenyl]imidazol-4-propionamid das (S)-α-[(S)-α-[2-(1-Adamantyl)acetamido]-hydrocinnamamido]-N-[(1S,2S,4S)-1-cyclohexylmethyl)-2-hydroxy-4-isopropylhexyl]imidazol-4-propionamid als weisser Festkörper, MS: 717 $(M+H)^+$;
- Aus (S)-N-[(1S,2S,4S)-1-(Cyclohexylmethyl)-2-hydroxy-4-isopropyl-5-hexenyl]-α-[(S)-α-(3-pyridinacetamido)hydrocinnamamido]imidazol-4-propionamid das (S)-N-[(1S,2S,4S)-1-(Cyclohexylmethyl)-2-hydroxy-4-isopropylhexyl]-α-[(S)-α-[2-(3-pyridyl)acetamido]hydrocinnamamido]imidazol-4-propionamid als weisse Kristalle, Schmelzpunkt 211° (aus Methylenchlorid/Methanol/Aether);
- Aus (S)-N-[(1S,2S,4S)-1-[Cyclohexylmethyl]-2-hydroxy-4-isopropyl-5-hexenyl]-α[(S)-α-[[5-oxo-L-prolyl]-amino]hydrocinnamamido]imidazol-4-propionamid das (S)-N-[(1S,2S,4S)-1-(Cyclohexylmethyl)-2-hydro xy-4-isopropylhexyl]-α-[(S)-α-[(S)-5-oxo-2-pyrrolidincarboxamido]hydrocinnamamido]imidazol-4-propionamid als dünnschichtchromatographisch einheitlicher Festkörper, MS: 651 $(M+H)^+$;
- Aus dem Epimerengemisch von (S)-N-[(1S,2S,4S)-1-[Cyclohexylmethyl]-2-hydroxy-4-isopropyl-5-hexenyl]-α-[(S)-α-[[5-oxo-D-prolyl]amino]hydrocinnamamido]imidazol-4-propionamid und (S)-N-[(1S,2S,4S)]-1-[Cyclohexylmethyl]-2-hydroxy-4-isopropyl-5-hexenyl]-α-[(S)-α-[[5-oxo-L-prolyl]amino]hydrocinnamamido]-imidazol-4-propionamid das (S)-N-[(1S,2S,4S)-1-(Cyclohexylmethyl)-2-hydroxy-4-isopropylhexyl]-α-[(S)-α-[-(RS)-5-oxo-2-pyrrolidincarboxamido]hydrocinnamamido]imidazol-4-propionamid als weisser Festkörper, MS: 651 $(M+H)^+$;
- Aus (S)-N-[(1S,2S,4S)-1-(Cyclohexylmethyl)-2-hydroxy-4-isopropyl-5-hexenyl]-α-[[N-(9H-fluoren-9-ylacetyl)-3-phenyl-L-alanyl]amino]imidazol-4-propionamid das (S)-N-[(1S,2S,4S)-1-(Cyclohexylmethyl)-2-hydroxy-4-isopropylhexyl]-α-[(S)-α-[2-(10,11-dihydro-5H-dibenzo[a,d]cyclohepten-5-yl)acetamido]hydrocinnamamido]-imidazol-4-propionamid als dünnschichtchromatographisch einheitlicher weisser Festkörper, MS: 775 $(M+H)^+$;
- Aus (S)-N-[(1S,2S,4S)-1-(Cyclohexylmethyl)-2-hydroxy-4-isopropyl-5-hexenyl]-α-[[N-(diphenylcarbamoyl)-3-phenyl-D-alanyl]amino]imidazol-4-propionamid das Aethyl [(S)-α-[[(S)-1-[[(1S,2S,4S)-1-(cyclohexylmethyl)-2-hydroxy-4-isopropylhexyl]carbamoyl]-2-imidazol-4-yläthyl]carbamoyl]phenäthyl]carbamat als weisse Kristalle, Schmelzpunkt 196° (aus Methanol/Methylenchlorid/Aether);

- Aus Benzyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamat das Phenyl [(S)-α-[[(S)-1-[[(1S,2S,4S)-1-(cyclohexylmethyl)-2-hydroxy-4-isopropylhexyl]carbamoyl]-2-imidazol-4-yläthyl]carbamoyl]phenäthyl]-carbamat als weisse Kristalle, Schmelzpunkt 157° (aus Methylenchlorid/Methanol/Aether);

- Aus (S)-N-[(1S,2S,4S)-1-(Cyclohexylmethyl)-2-hydroxy-4-isopropyl-5-hexenyl]-α-[(S)-α-(4-nitrocinnamamido)hydrocinnamamido]imidazol-4-propionamid das (S)-α-[(S)-α-(4-Aminohydrocinnamamido)-hydrocinnamamido]-N-[(1S,2S,4S)-1-(cyclohexylmethyl)-2-hydroxy-4-isopropylhexyl]imidazol-4-propionamid als weisse Kristalle, Schmelzpunkt 174° (aus Methylenchlorid/Methanol/Aether);

- Aus (2S,3S,5S)-2-(Boc-D-Pro-Phe-His-NH)-1-cyclohexyl-5-isopropyl-6-hepten-3-ol das t-Butyl (R)-2-[[(S)-α-[[(S)-1-[(1S,2S,4S)-1-(cyclohexylmethyl)-2-hydroxy-4-isopropylhexyl]-2-imidazol-4-yläthyl]carbamoyl] phenäthyl]carbamoyl]-1-pyrrolidincarboxylat als weisse Kristalle, Schmelzpunkt 173° (aus Methylenchlorid/Methanol/Hexan);

- Aus (S)-N-[(1S,2S,4S)-1-(Cyclohexylmethyl)-2-hydroxy-4-isopropyl-5-hexenyl]-α-[(S)-α-(4-oxavaleramido)-hydrocinnamamido]imidazol-4-propionamid das (S)-N-[(1S,2S,4S)-1-(Cyclohexylmethyl)-2-hydroxy-4-isopropylhexyl]-α-[(S)-α-(4-oxovaleramido)hydrocinnamamido]imidazol-4-propionamid als Schaum, MS: 638 $(M+H)^+$;

- Aus (S)-N-[(1S,2S,4S)-1-(Cyclohexylmethyl)-2-hydroxy-4-isopropyl-5-hexenyl]-α-[(S)-α-(4-hydroxyhydrocinnamamido)hydrocinnamamido]imidazol-4-propionamid das (S)-N-[(1S,2S,4S)-1-(Cyclohexyl methyl)-2-hydroxy-4-isopropylhexyl]-α-[(S)-α-(4-hydroxyhydrocinnamamido)hydrocinnamamido]imidazol-4-propionamid als weisser Festkörper, Schmelzpunkt 120° (Zers.; aus Methylenchlorid/Aether/Hexan);

- Aus (S)-[(1S,2S,4S)-1-(Cyclohexylmethyl)-2-hydroxy-4-isopropyl-5-hexenyl]-α-[(S)-α-(3-pyridinacrylamido)-hydrocinnamamido]imidazol-4-propionamid das (S)-N-[(1S,2S,4S)-1-(Cyclohexylmethyl)-2-hydroxy-4-isopropylhexyl]-α-[(S)-α-[3-(3-pyridyl)propionamido]hydrocinnamamido]imidazol-4-propionamid als weisse Kristalle, Schmelzpunkt 195° (aus Methanol/Aether/Hexan);

- Aus (S)-N-[(1S,2S,4S)-1-(Cyclohexylmethyl)-2-hydroxy-4-isopropyl-5-hexenyl[-α-[(S)-α-nicotinamidohydrocinnamamido]imidazol-4-propionamid das N-[(S)-α-[[(S)-1-[[(1S,2S,4S)-1-(Cyclohexylmethyl)-2-hydroxy-4-isopropylhexyl]carbamoyl]-2-imidazol-4-yläthyl]carbamoyl]phenäthyl]-nicotinamid als weisser Festkörper, Schmelzpunkt 112° (Zers.; aus Methylenchlorid/Aether/Hexan);

- Aus (S)-N-[(1S,2S,4S)-1-(Cyclohexylmethyl)-2-hydroxy-4-isopropyl-5-hexenyl]-α-[(S)-α-(imidazol-4-acrylamido)hydrocinnamamido]imidazol-4-propionamid das N-[(1S,2S,4S)-1-(Cyclohexylmethyl)-2-hydroxy-4-isopropyl]-α-[(S)-α-imidazol-4-propionamidohydrocinnamamido]imidazol-4-propionamid als weisse Kristalle, Schmelzpunkt 118° (aus Methanol/Aether/Hexan);

- Aus t-Butyl [1-[[(S)-α-[[(S)-1-[[(1S,2S,4S)-1-(cyclohexylmethyl)-2-hydroxy-4-isopropyl-5-hexenyl]-carbamoyl]-2-imidazol-4-yläthyl]carbamoyl]phenäthyl]carbamoyl]-1-methyläthyl]carbamat das t-Butyl [1-[[-(S)-α-[[(S)-1-[[(1S,2S,4S)-1-(cyclohexylmethyl)-2-hydroxy-4-isopropylhexyl] carbamoyl]-2-imidazol-4-yläthyl]-carbamoyl]hydrocinnamoyl]carbamoyl]-1-methyläthyl]carbamat als Schaum, MS: 725 $(M+H)^+$.

## Beispiel 17

Ein Gemisch von 95 mg (0,133 mMol) N-[(1S,2S,4S)-1-(Cyclohexylmethyl)-2-hydroxy-4-isopropylhexyl]-α-[(3-phenyl-N-D-prolyl-L-alanyl)amino]imidazol-4-propionamid, 0,025 ml (0,199 mMol) Pivaloylchlorid und 0,185 ml (1,33 mMol) Triäthylamin in 10 ml Tetrahydrofuran wird über Nacht bei Raumtemperatur gerührt. Nach üblicher Aufarbeitung und chromatographischer Reinigung an Kieselgel mit einem 20:1:0,1-Gemisch von Methylenchlorid, Methanol und Ammoniak erhält man (S)-N-[(1S,2S,4S)-1-(Cyclohexylmethyl)-2-hydroxy-4-isopropylhexyl]-α-[(S)-α-[(R)-1-pivaloyl-2-piperidincarboxamido]hydrocinnamamido]imidazol-4-propionamid als dünnschichtchromatographisch einheitlichen Festkörper, MS: 722 $(M+H)^+$.

## Beispiel 18

20 mg (S)-α-[[N-(3-t-Butoxy-L-alanyl)-3-phenyl-L-alanyl]amino]-N-[(1S,4S)-1-(cyclohexylmethyl)-2-hydroxy-4-isopropyl-5-hexenyl]imidazol-propionamid werden in 1 ml wässriger Trifluoressigsäure (95 %ig) gelöst und 2,5 Stunden bei Raumtemperatur gerührt. Eindampfen des Reaktionsgemisches und chromatographische Reinigung an Kieselgel mit einem 8:1:0,1-Gemisch von Methylenchlorid, Methanol und Ammoniak liefert dünnschichtchromatographisch einheitliches (S)-N-[(1S,2S,4S)-1-(Cyclohexylmethyl)-2-hydroxy-4-isopropyl-5-hexenyl]-α-[[N-(L-serylamino)-3-phenyl-L-alanyl]amino]imidazol-4-propionamid, MS: 625 $(M+H)^+$.

## Beispiel 19

195 mg N-[(R)-2-Benzyl-5,5-dimethyl-4-oxohexanoyl]-N-methyl-L-histidinbenzylester werden in Gegenwart von 25 mg Palladium auf Kohle (5%ig) 2 Stunden hydriert, und das erhaltene N-[(R)-2-Benzyl-5,5-dimethyl-4-oxohexanoyl]-N-methyl-L-histidin in üblicher Weise mit (αS,βS)-β-Amino-α-[(S)-2-isopropyl-3-butenyl]cyclohexanpropanol umgesetzt. Chromatographische Reinigung des erhaltenen Rohprodukts an Kieselgel mit einem 140:10:0,1-Gemisch von Methylenchlorid, Methanol und Ammoniak liefert N-[-(1S,2S,4S)-1-(Cyclohexylmethyl)-2-hydroxy-4-isopropyl-5-hexenyl]-α-[(R)-2-benzyl-N,5,5-trimethyl-4-oxohexanamido]imidazol-4-propionamid als dünnschichtchromatographisch einheitliche Substanz, MS: 635 $(M+H)^+$.

Als Nebenprodukt erhält man das weniger polare epimere N-[(1S,2S,4S)-1-(Cyclohexylmethyl)-2-hydroxy-4-isopropyl-5-hexenyl]-α-[(R)-2-benzyl-N,5,5-trimethyl-4-oxohexanamido]imidazol-4-propionamid, MS: 635 $(M+H)^+$.

Der als Ausgangsmaterial eingesetzte N-[(R)-2-Benzyl-5,5-dimethyl-4-oxohexanoyl]-N-methyl-L-histidinbenzylester wurde durch Kondensation von N-(α-methyl)-L-histidinbenzylester mit 2-(R)-Benzyl-4-oxo-5,5,5-trimethylvaleriansäure hergestellt und direkt in die nächste Stufe eingesetzt.

## Beispiel 20

100 mg (0,164 mMol) Aethyl (R)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyrat werden in einer Lösung von 30% Hydrazinhydrat in Methanol 3 Stunden bei Raumtemperatur gerührt. Danach wird das Reaktionsgemisch eingedampft, und der Rückstand mit einem 14:1:0,1-Gemisch von Methylenchlorid, Methanol und Ammoniak an Kieselgel chromatographiert. Kristallisation des so erhaltenen Rohprodukts aus Methanol/Methylenchlorid/Aether liefert (R)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]4-phenylbutyrohydrazid als weisse Kristalle, Schmelzpunkt 109°.

Das als Ausgangsmaterial eingesetzte Aethyl (R)-3-[[(S)-1-[[(1S,2S,4S)-1-(cyclohexylmethyl) -2-hydroxy-4-isopropyl-5-hexenyl]carbamoyl]-2-imidazol-4-yläthyl]carbamoyl]-4-phenylbutyrat wurde wie in Beispiel 40 beschrieben, hergestellt.

## Beispiel 21

0,083 ml (0,54 mMol) 4-Piperidincarbonsäureäthylester werden mit 210 mg (0,36 mMol) Aethyl 1-[(R)-3-[[(S)-1-[[(1S,2S,4S)-1-(cyclohexylmethyl)-2-hydroxy-4-isopropyl-5-hexenyl]carbamoyl]-2-imidazol-4-yläthyl]-carbamoyl]-4-phenylbutyryl]carboxylat in 20 ml Acetonitril in Gegenwart von Bis-(2-oxo-3-oxazolidinyl)-phosphinchlorid bei Raumtemperatur umgesetzt. Nach Aufarbeiten in üblicher Weise und Chromatographie an Kieselgel erhält man Aethyl 1-[(R)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyryl]-4-piperidincarboxylat als Schaum, MS: 720 $(M+H)^+$.

In analoger Weise wie oben beschrieben wurden die folgenden Verbindungen hergestellt:

- Aus (R)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbuttersäure und L-Prolinol das (S)-N-[(1S,2S,4S)-1-(Cyclohexylmethyl)-2-hydroxy-4-isopropyl-5-hexenyl]-α-[(R)-α-[[(2-hydroxymethyl)-1-pyrrolidinyl]carbonyl]-methyl]hydrocinnamamido]imidazol-4-propionamid als weisser Festkörper, Schmelzpunkt 103° (Zers.; aus Methylenchlorid/Aether/Hexan);
- Aus (R)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbuttersäure und N-Methyläthanolamin das (S)-N-[(1S,2S,4S)-1-

(Cyclohexylmethyl)-2-hydroxy-4-isopropyl-5-hexenyl]-α-[(R)-α-[[(2-hydroxyäthyl)methylcarbamoyl]methyl]-hydrocinnamamido]imidazol-4-propionamid als weisser Festkörper, Schmelzpunkt 82° (Zers.; aus Methylenchlorid/Aether/Hexan);

- Aus (R)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbuttersäure und N,N-Dimethyläthylendiamin das (S)-N-[(1S,2S,4S)-1-(Cyclohexylmethyl)-2-hydroxy-4-isopropyl-5-hexenyl]-α-[(R)-α-[[[2-(dimethylamino)äthyl]carbamoyl]methyl]-hydrocinnamamido]imidazol-4-propionamid als weisser Festkörper, Schmelzpunkt 98° (aus Methylenchlorid/Hexan);
- Aus (R)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbuttersäure und N,N-Dimethylpropylendiamin das (S)-N-[(1S,2S,4S)-1-(Cyclohexylmethyl)-2-hydroxy-4-isopropyl-5-hexenyl]-α-[[[3-(dimethylamino)propyl]carbamoyl]methyl]-hydrocinnamamido]imidazol-4-propionamid als weisse Kristalle, Schmelzpunkt 98° (aus Methylenchlorid/Aether).

Die als Ausgangsmaterial eingesetzte (R)-3-[[(S)-1-[[(1S,2S,4S)-1-(Cyclohexylmethyl)-2-hydroxy4-isopropyl-5-hexenyl]carbamoyl]-2-imidazol-4-yläthyl]carbamoyl]-4-phenylbuttersäure wurde wie folgt hergestellt:

100 mg (0,16 mMol) Aethyl (R)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyrat (vgl. Beispiel 40) in 3 ml Aethanol werden mit 0,66 ml 0,5N (0,33 mMol) Natronlauge versetzt und 2,5 Stunden bei Raumtemperatur gerührt. Dann neutralisiert man mit 0,33 ml (0,33 mMol) 1N Salzsäure, gibt Essigester zu und wäscht mit gesättigter Kochsalzlösung. Dann wird die organische Phase über Natriumsulfat getrocknet, und das Lösungsmittel unter vermindertem Druck entfernt, wobei man 91 mg (95 %) (R)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbuttersäure als amorphes Pulver erhält, MS: 581 $(M+H)^+$.

## Beispiel 22

70 mg (0,1 mMol) Aethyl 1-[(R)-3-[[(S)-1-[[(1S,2S,4S)-1-cyclohexylmethyl)-2-hydroxy-4-isopropyl-5-hexenyl]carbamoyl]-2-imidazol-4-yläthyl]carbamoy]-4-phenylbutyryl]-4-piperidincarboxylat werden in 1N Natronlauge in einem 9:1-Gemisch von Methanol und Wasser suspendiert und 3 Stunden bei Raumtemperatur gerührt. Danach wird das Lösungsmittel abgedampf, und der Rückstand aus Methanol/Methylenchlorid/Hexan umkristallisiert, wobei man 1-[(R)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbutyryl]-4-piperidincarbonsäure als weisse Kristalle erhält, Schmelzpunkt 169°.

## Beispiel 23

Umsetzung von (S)-N-[(1S,2S,4S)-1-(Cyclohexylmethyl)-2-hydroxy-4-isopropyl-5-hexenyl]-α-[(S)-d-(D-prolylamino)hydrocinnamamido]imidazol-4-propionamid-dihydrochlorid (vgl. Beispiel 8) mit Fmoc-Ser(t-Bu)-OH in analoger Weise wie in Beispiel 1 beschrieben, Behandlung der erhaltenen Fmoc-geschützten Verbindung mit Piperidin und anschliessende Chromatographie des Rohprodukts an Kieselgel mit einem 200:10:1-Gemisch von Methylenchlorid, Methanol und Ammoniak liefert dünnschichtchromatographisch reines (S)-α-[[N-[1-(3-Butoxy-L-alanyl)-D-propyl]-3-phenyl-D-alanyl]amino]-N-[(2S,3S,4S)-3-(cyclohexylmethyl)-2-hydroxy-4-isopropyl-5-hexenyl]imidazol-4-propionamid als Schaum, MS: 779 $(M+H)^+$.

## Beispiel 24

Ein Gemisch von 100 mg (0,17 mMol) 2-t-Butyl. [(S)-α-[[(S)-1-[[(1S,2S,4S)-2-Hydroxy-1-isobutyl-4-isopropyl-5-hexenyl]carbamoyl]-2-imidazol-4-yläthyl]carbamoyl]phenäthyl]carbamat und 65 mg (0,25 mMol) Osmiumtetroxyd in 20 ml Pyridin wird über Nacht bei Raumtemperatur gerührt. Danach werden 4 ml 33%ige Natriumbisulfitlösung zugegeben, und das Gemisch eine weitere Stunde gerührt. Chromatographie des nach üblicher Aufarbeitung erhaltenen Rohprodukts an 20 g Kieselgel mit einem 9:1:0,1-Gemisch von

Chloroform, Methanol und Ammoniak liefert 65 mg dünnschichtchromatographisch einheitliches (2RS,3S,5S,6S)-6-(Boc-Phe-His-NH)-3-isopropyl-8-methyl-1,2,5-nonantriol, MS: 632 $(M+H)^+$.

## Beispiel 25

Ein Gemisch von 411 mg (0,82 mMol) (RS)-α-[[(S)-α-(Boc-Pro-NH)phenäthyl]carbamoyl]imidazol-4-propionsäure, 200 mg (0,82 mMol) (αS,βS)-β-Amino-α-[(S)-2-isopropyl-3-butenyl]cyclohexanpropanol, 0,1 ml (0,82 mMol) 4-Aethylmorpholin, 222 mg (1,64 mMol) HOBT und 203 mg (0,99 mMol) DCC wird in 20 ml Dimethylformamid gelöst und 2 Tage bei Raumtemperatur gerührt. Der ausgefallene Niederschlag wird abfiltriert, und das Lösungsmittel unter vermindertem Druck abgedampft. Nach üblicher Aufarbeitung erhält man 590 mg eines Rohprodukts, welches an 30 g Kieselgel mit einem 20:1:0,1-Gemisch von Methylenchlorid, Methanol und Ammoniak Chromatographiert wird. Umkristallisation des dünnschichtchromatographisch einheitlichen Produkts aus Methylenchlorid/Aether/Hexan liefert 260 mg t-Butyl (S)-2-[[(S)-α-[(RS)-α-[[(1S,2S,4S)-1-(cyclohexylmethyl-2-hydroxy-4-isopropyl-5-hexenyl]carbamoyl]imidazol-3-propionamido]-phenäthyl]carbamoyl]-1-pyrrolidincarboxylat als Kristalle, Schmelzpunkt 112°.

Die als Ausgangsmaterial eingesetzte (RS)-α-[[(S)-α-(Boc-Pro-NH)phenäthyl]carbamoyl]imidazol-4-propionsäure wurde wie folgt hergestellt:

10,8 g (30 mMol) Boc-Pro-Phe-OH und 3,0 g (30 mMol) N-Methylmorpholin werden in 120 ml Tetrahydrofuran gelöst und auf -20° abgekühlt. Zu dieser Lösung werden tropfenweise 4,1 g Chlorameisensäureisobutylester in 15 ml Tetrahydrofuran gegeben, und nach 5 Minuten bei -20° eine Lösung von 3,9 g (60 mMol) Natriumazid in 30 ml Wasser zugetropft. Nach beendeter Zugabe rührt man 30 Minuten bei 0°, verdünnt dann mit eisgekühltem Essigester auf das doppelte Volumen und behandelt das Reaktionsgemisch in der Folge zunächst mit kalter gesättigter Natriumbicarbonatlösung und dann mit kalter gesättigter Natriumchloridlösung. Die organische Phase wird abgetrennt, über Magnesiumsulfat getrocknet und unter vermindertem Druck bei einer Temperatur von 20-25° abgedampft. Der Rückstand wird unter einer Stickstoffatmosphäre in 300 ml Toluol gelöst und 30 Minuten auf 80° erwärmt. Dann werden 4,9 g (45 mMol, 1,5 Moläquivalente) Benzylalkohol zugegeben, und das Reaktionsgemisch 6 Stunden zum Rückfluss erhitzt. Nach dem Abkühlen und Eindampfen der Reaktionslösung wird der Rückstand mit einem 1:1-Gemisch von Aether und Hexan verrührt, und der ausgefallene Niederschlag abfiltriert. Nach dem Trocknen des Filtrates unter vermindertem Druck erhält man 11 g (79 %) [(R)-1-(Boc-Pro-NH)-1-(benzylamino)äthyl]-benzol, Schmelzpunkt 155-157°.

4,7 g (10 mMol) der obigen Verbindung in 300 ml Tetrahydrofuran werden in Gegenwart von 1,0 g Palladium auf Kohle (10 %ig) 3 Stunden bei 343,25 kPa hydriert. Nach beendeter Wasserstoffaufnahme wird der Katalysator abfiltriert, und das Filtrat unter vermindertem Druck eingedampft. Der Rückstand wird in 60 ml Dimethylformamid gelöst, und 1,98 g (10 mMol) (RS)-(Imidazol-4-ylmethyl)-malonsäuremonomethylester (M. Goodman et al., Int. J. Pept. Prot. Res. 17, 72-88 (1981)) zugegeben. Die erhaltene Lösung wird dann auf 0° abgekühlt und mit 3,0 g (20 mMol) HOBT und 2,2 g (12 mMol) EDC versetzt. Dann lässt man das Reaktionsgemisch auf Raumtemperatur erwärmen und rührt 48 Stunden bei dieser Temperatur. Nach dem Eindampfen des Reaktionsgemisches im Hochvakuum wird der Rückstand in Essigester gelöst, mit Wasser gewaschen und anschliessend mit 1N Zitronensäure extrahiert. Der Zitronensäureextrakt wird mit Natriumbicarbonat neutralisiert und mit Essigester extrahiert. Der organische Extrakt wird dann über Natriumsulfat getrocknet und unter vermindertem Druck eingedampft. Chromatographie des Rückstandes an Kieselgel und Umkristallisation des erhaltenen Rohprodukts aus Essigester/Aether liefert 2,5 g (49 %) (RS)-α-[[(S)-α-(Boc-Pro-NH)-phenäthyl]carbamoyl]imidazol-4-propionsäuremethylester, Schmelzpunkt 142-143°.

1,54 g (3 mMol) des obigen Esters werden in 6 ml Aethanol gelöst und mit 3,15 ml (3,15 mMol, 1,05 Moläquivalente) 1N Natronlauge versetzt. Nach 3-stündigem Rühren werden unter Eiskühlung 3,15 ml (3,15 mMol) 1N $H_2SO_4$ zugegeben, und das Reaktionsgemisch unter vermindertem Druck eingedampft. Der Rückstand wird mit 300 ml Aethanol verrührt, und das unlösliche Natirumsulfat abfiltriert. Einengen der äthanolischen Lösung liefert 1,47 g (98 %) (RS)-α-[[(S)-α-(Boc-Pro-NH)phenäthyl]carbamoyl]imidazol-4-propionsäure, Schmelzpunkt 120° (Zers.).

## Beispiel 26

Ein Gemisch von 1,05 g (2,06 mMol) Boc-Phe $\frac{R}{}$His(Bom)OH, 0,5 g (2,06 mMol) (αS,βS)-β-Amino-α-[(S)-2-isopropyl-3-butenyl]cyclohexanpropanol, 0,26 ml (2,06 mMol) 4-Aethylmorpholin, 0,56 g (4,12 mMol) HOBT und 0,51 g (2,47 mMol) DCC in 40 ml Dimethylformamid wird bei Raumtemperatur über Nacht gerührt. Dann wird vom ausgefallenen Harnstoff abfiltriert, und das Lösungsmittel unter vermindertem Druck eingedampft. Aufarbeiten des Rückstands in üblicher Weise liefert 1,69 g t-Butyl [(S)-1-benzyl-2-[[(S)-1-[[(1S,2S,4S)-1-(cyclohexylmethyl)-2-hydroxy-4-isopropyl-5-hexenyl]carbamoyl]-2-imidazol-4-yläthyl]amino]-äthyl]carbamat, welches direkt in die nächste Stufe eingesetzt wird.

1,69 g (ca. 2,06 mMol) t-Butyl [(S)-1-benzyl-2-[[(S)-1-[[(1S,2S,4S)-1-(cyclohexylmethyl)-2-hydroxy-4-isopropyl-5-hexenyl]carbamoyl]-2-imidazol-4-yläthyl]amino]äthyl]carbamat werden in Gegenwart von 0,5 g Palladium auf Kohle (5%ig) in 50 ml eines 4:1-Gemisches von Essigsäure und Wasser und 0,8 g N,N'-Dimethyläthylendiamin über Nacht bei Raumtemperatur hydriert. Nach beendeter Wasserstoffaufnahme wird der Katalysator abfiltriert, und das Filtrat einge dampft und zweimal nacheinander zuerst mit Toluol versetzt und dann wiederum zur Trockene eingedampft. Chromatographie des Rückstands an 180 g Kieselgel mit einem 20:1:0,1-Gemisch von Methylenchlorid, Methanol und Ammoniak als Eluierungsmittel liefert 1,13 g eines Rohprodukts, welches aus Methylenchlorid/Aether/Hexan umkristallisiert wird, wobei man 730 mg t-Butyl [(S)-1-benzyl-2-[[(S)-1-[[(1S,2S,4S)-1-(cyclohexylmethyl)-2-hydroxy-4-isopropylhexyl]-carbamoyl]-2-imidazol-4-yläthyl]amino]äthyl]carbamat als Kristalle erhält, Schmelzpunkt 144°.

Das als Ausgangsmaterial eingesetzte Boc-Phe $\frac{R}{}$His(Bom)-OH wurde wie folgt hergestellt:

58 g Boc-Phe-His(Bom)OMe, welches nach üblichen Methoden durch Kupplung von Boc-Phe-OH mit His(Bom)OMe hergestellt wurde, 30,35 g Lawesson-Reagenz und 700 ml Benzol werden über Nacht zum Rückfluss erhitzt. Danach wird das Reaktionsgemisch abgekühlt und zur Trockene eingedampft. Der Rückstand wird dann durch 1,5 g Kieselgel zunächst mit Methylenchlorid und dann mit Methylenchlorid unter progressiver Zugabe von Aethanol (bis zu 7 %) filtriert. Dabei erhält man 35,56 g [(S)-2-(Boc-NH)-3-phenylthiopropionyl]His(Bom)OMe in Form eines Schaumes, MS: 553 $(M+H)^+$.

0,5 g der obigen Thioverbindung und 5 g Raney-Nickel in 30 ml Aethanol werden während 2 Stunden in einer Wasserstoffatmosphäre gerührt. Dann wird der Katalysator abfiltriert, und das Filtrat eingedampft. Chromatographie des Rohprodukts an 30 g Kieselgel mit Chloroform, einem 98:2-Gemisch von Chloroform und Aethanol und einem 95:5-Gemisch von Chloroform und Aethanol als Eluierungsmittel liefert 0,24 g [(S)-2-(Boc-NH)-3-phenylpropyl]His(Bom)OMe in Form eines Schaumes, MS: 523 $(M+H)^+$.

170 mg des obigen Esters in 2 ml 1N Natronlauge in einem 1:1-Gemisch von Methanol und Wasser werden über Nacht bei Raumtemperatur gerührt. Dann wird 1 ml konz. Essigsäure zugegeben, und das Reaktionsgemisch unter vermindertem Druck eingedampft. Der Rückstand wird 3 mal mit je 150 ml Methanol erwärmt und abdekantiert. Die methanolische Phase wird eingedampft, und der Rückstand an 30 g Kieselgel mit einem 4:1:1-Gemisch von Butanol, Essigsäure und Wasser chromatographiert, wobei man 100 mg [(S)-2-(Boc-NH)-3-phenylpropyl]His(Bom)OH in Form eines Schaums erhält, MS: 509 $(M+H)^+$.

## Beispiel 27

0,17 g (0,56 mMol) 2-(RS)-Benzyl-4-(4-chlorphenyl)-4-oxo-buttersäure werden in 10 ml Dimethylformamid gelöst und mit 0,20 g (0,51 mMol) (S)-α-Amino-N-[(1S,2S,4S)-1-(cyclohexylmethyl)-2-hydroxy-4-iso propyl-5-hexenyl]imidazol-4-propionamid, 0,057 g (0,56 mMol) Triäthylamin und 0,21 g (0,56 mMol) HBTU versetzt und über Nacht bei Raumtemperatur gerührt. Anschliessend wird im Hochvakuum eingedampft. Der Rückstand wird in Essigester gelöst und dann mit gesättigter Natriumbicarbonatlösung und gesättigter Kochsalzlösung gewaschen. Nach dem Trocknen der organischen Phase über Natriumsulfat wird das Lösungsmittel unter vermindertem Druck abgedampft, und der Rückstand zur Reinigung an Kieselgel unter Verwendung eines 95:5-Gemisches von Methylenchlorid und Methanol, welches 0,5 % Ammoniumhydroxyd enthält, chromatographiert. Auf diese Weise werden 270 mg (78 %) (S)-α-[(RS)-α-(4-Chlorphenylacetyl)-hydrocinnamamido]-N-[(1S,2S,4S)-1-(cyclohexylmethyl)-2-hydroxy-4-isopropyl-5-hexenyl]imidazol-4-propionamid in Form eines gelblichen Schaums erhalten.
MS: 675 $(M^+)$.

Die als Ausgangsmaterial eingesetzte 2-(RS)-Benzyl-4-(4-chlorphenyl)-4-oxo-buttersäure wurde wie folgt hergestellt:

Zu einer Suspension von 0,87 g Natriumhydrid-Dispersion (55 %ig in Oel) in 20 ml Dimethylformamid werden bei Raumtemperatur 5,0 g (20 mMol) Benzylmalonsäurediäthylester getropft. Man rührt anschliessend das Reaktionsgemisch 20 Minuten bei Raumtemperatur und tropft danach 4,67 g (20 mMol) omega-Brom-4-chloracetophenon in 25 ml Dimethylformamid zu. Nach beendeter Zugabe wird das tiefbraun

gefärbte Reaktionsgemisch über Nacht bei Raumtemperatur gerührt und danach im Hochvakuum eingedampft. Der Rückstand wird in Methylenchlorid gelöst, mit Wasser gewaschen und über Natriumsulfat getrocknet. Danach wird das Lösungsmittel unter vermindertem Druck abgedampft, und der Rückstand zur Reinigung mit einem 6:1-Gemisch von Hexan und Aether an Kieselgel chromatographiert, wobei man 5,17 g (64 %) 2-(RS)-Benzyl-2-carbäthoxy-4-(4-chlorphenyl)-4-oxobuttersäure als gelblichen Festkörper erhält.
MS: 357 $(M-OC_2H_5)^+$, 311 $(M-Benzyl)^+$.

Zu einer Lösung 1,64 g (4,1 mMol) des oben genannten Aethylesters in 10 ml Aethanol und 9 ml Wasser werden 6,15 ml (3 Moläquivalente) 2N Natronlauge gegeben, und das Reaktionsgemisch 16 Stunden bei Raumtemperatur gerührt. Anschliessend wird mit 1N Salzsäure auf pH 3 gestellt und mit Methylenchlorid extrahiert. Die organische Phase wird über Natriumsulfat getrocknet, unter vermindertem Druck eingedampft, und der kristalline Rückstand bis zur Beendigung der $CO_2$-Entwicklung auf 150-160° erhitzt. Nach dem Abkühlen wird der Rückstand in Aether gelöst und durch Zugabe von Hexan zur Kristallisation gebracht. Es werden dabei 970 mg (79 %) 2-(RS)-Benzyl-4-(4-chlorphenyl)-4-oxobuttersäure als gelbliche Kristalle erhalten, Schmelzpunkt 137-138°.

## Beispiel 28

Zu einer Lösung von 1,45 g (4,43 mMol) 2-(RS)-(1-Naphthylmethyl)-3-morpholinocarbonylpropionsäure-(EPA 0.200.406) in 60 ml Dimethylformamid werden 1,57 g (4,03 mMol) (S)-α-Amino-N-[(1S,2S,4S)-1-(cyclohexylmethyl)-2-hydroxy-4-isopropyl-5-hexenyl]imidazol-4-propionamid, 0,45 g (4,43 mMol) Triäthylamin und 1,68 g (4,43 mMol) HBTU gegeben. Die erhaltene gelbe Reaktionslösung wird dann 15 Stunden bei Raumtemperatur gerührt und anschliessend im Hochvakuum eingedampft. Der Rückstand wird in 150 ml Methylenchlorid gelöst, die organische Phase mit 2mal 30 ml gesättigter Natriumbicarbonatlösung und 30 ml Wasser gewaschen, über Natriumsulfat getrocknet und unter vermindertem Druck eingedampft. Der Rückstand wird zur Reinigung und Trennung der beiden Epimeren mit einem 95:5-Gemisch von Methylenchlorid und Methanol, welches 0,5 % Ammoniumhydroxyd enthält, an Kieselgel chromatographiert. Dabei erhält man 855 mg (30 %) eines Isomeren mit dem Rf-Wert 0,45 und 726 mg (26 %) eines Isomeren mit dem Rf-Wert 0,33. Beide Isomeren zeigen das gleiche Massenspektrum, MS: 700 $(M+H)^+$

Die als Ausgangsmaterial eingesetzte 2-(RS)-(1-Naphthylmethyl)-3-morpholinocarbonylpropionsäure wurde wie folgt hergestellt:

Zu einer Lösung von 3,63 g (12,6 mMol) 3-Aethoxycarbonyl-4-(1-naphthyl)buttersäure(EPA 0.181.110) in 50 ml Dimethylformamid werden 1,10 g (12,6 mMol) Morpholin, 2,42 g (12,6 mMol) EDC und 3,43 g (25,2 mMol) HOBT gegeben, und die Reaktionslösung 15 Stunden bei Raumtemperatur gerührt und anschliessend im Hochvakuum eingedampft. Der Rückstand wird in Essigester gelöst, und die organische Phase mit Wasser, gesättigter Natriumbicarbonatlösung und gesättigter Kochsalzlösung gewaschen, über Natriumsulfat getrocknet und unter vermindertem Druck eingedampft. Der Rückstand wird mit einem 98:2-Gemisch von Methylenchlorid und Aethanol an Kieselgel chromatographiert, wobei man 3,78 g (84 %) 2-(RS)-(1-Naphthylmethyl)-3-morpholinocarbonylpropionsäureäthylester als gelbliches Oel erhält, welches nach längerem Stehenlassen auszukristallisieren beginnt.
MS: 355 $(M^+)$

1,7 g (4,78 mMol) des oben genannten Aethylesters werden in 6 ml Aethanol gelöst und nach Zugabe von 7,2 ml (7,2 mMol) 1N Natronlauge 3 Stunden bei 50° gerührt. Nach dem Abkühlen gibt man 7,5 ml (7,5 mMol) 1N Salzsäure zur tiefgelben Reaktionslösung und dampft anschliessend unter vermindertem Druck ein. Der Rückstand wird in 100 ml Methylenchlorid gelöst, und die organische Phase mit 2mal 20 ml Wasser gewaschen, über Natriumsulfat getrocknet und unter vermindertem Druck eingedampft, wobei man 1,45 g (92 %) 2-(RS)-(1-Naphthylmethyl)-3-morpholinocarbonylpropionsäure als gelben Schaum erhält, der direkt in die nächste Stufe eingesetzt wird.
MS: 327 $(M^+)$

## Beispiel 29

109 mg (0,38 mMol) 3-(RS)-(1-Naphthylmethyl)bernsteinsäure-1-äthylester werden in 6 ml Acetonitril und 1 ml Dimethylformamid gelöst und nach Zugabe von 135 mg (0,35 mMol) (S)-α-Amino-N-[(1S,2S,4S)-1-(cyclohexylmethyl)-2-hydroxy-4-isopropyl-5-hexenyl]imidazol-4-propionamid, 39 mg (0,38 mMol) Triäthylamin und 144 mg (0,38 mMol) HBTU 6 Stunden bei Raumtemperatur gerührt. Anschliessend wird die Reaktionslösung im Hochvakuum eingedampft, und der Rückstand in 50 ml Essigester gelöst und mit 3mal 10 ml gesättiger Natriumbicarbonatlösung gewaschen. Die organische phase wird über Natriumsulfat getrocknet und unter vermindertem Druck eingedampft, und der Rückstand mit einem 95:5-Gemisch von Methylenchlorid und Methanol, welches 0,1 % Ammoniumhydroxyd enthält, an Kieselgel chromatographiert, wobei man 103 mg (45 %) (RS)-β-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-1-naphthylbuttersäureäthylester als gelblichen Schaum erhält.

MS: 594 $(M\text{-Aethanol-Wasser})^+$

Der als Ausgangsmaterial eingesetzte 3-(RS)-(1-Naphthylmethyl)bernsteinsäure-1-äthylester wurde wie folgt hergestellt:

820 mg (3,44 mMol) 2-(1-Naphthylmethylen)bernsteinsäureanhydrid(EPA 0.181.110) werden in 5 ml Aethanol 4 Stunden zum Rückfluss erhitzt. Anschliessend wird das Reaktionsgemisch unter vermindertem Druck eingedampft, der Rückstand in 10 ml gesättigter Natriumbicarbonatlösung aufgenommen und mit Aether extrahiert. Die wässrige Phase wird mit 1N Salzsäure angesäuert und mit Aether extrahiert. Nach dem Trocknen der Aetherextrakte über Natriumsulfat und Eindampfen unter vermindertem Druck verbleiben 710 mg (73 %) 3-(RS)-(1-Naphthylmethylen)bernsteinsäure-1-äthylester.

Diese 710 mg (2,5 mMol) des Aethylesters werden in 5 ml Aethanol gelöst und in Gegenwart von 100 mg Palladium auf Kohle (10 %ig) 15 Stunden hydriert. Danach wird der Katalysator abfiltriert, das Fitlrat unter vermindertem Druck eingedampft und der Rückstand mit einem 98:2-Gemisch von Methylenchlorid und Methanol an Kieselgel chromatographiert, wobei 190 mg (27 %) 3-(RS)-(1-Naphthylmethyl)-bernsteinsäure-1-äthylester erhalten werden.

MS: 286 $(M)^+$

## Beispiel 30

In analoger Weise wie in Beispiel 27 beschrieben, wurden durch Kondensation von (αRS,S)-α-Benzyl-1-t-butoxycarbonyl-γ-oxo-2-pyrrolidinbuttersäure mit (S)-α-Amino-N-[(1S,2S,4S)-1-(cyclohexylmethyl)-2-hydroxy-4-isopropyl-5-hexenyl]imidazol-4-propionamid und anschliessender Epimerentrennung durch Chromatographie t-Butyl (S)-2-[[(S)-α-[[(S)-1-[[(1S,2S,4S)-1-(cyclohexylmethyl)-2-hydroxy-4-isopropyl-5-hexenyl]-carbamoyl]-2-imidazol-4-yläthyl]carbamoyl]phenäthyl]acetyl]-1-pyrrolidincarboxylat (weniger polares Isomeres) und t-Butyl (S)-2-[[(R)-α-[[(S)-1-[[(1S,2S,4S)-1-(cyclohexylmethyl)-2-hydroxy-4-isopropyl-5-hexenyl]-carbamoyl]-2-imidazol-4-yläthyl]carbamoyl]phenäthyl]acetyl]-1-pyrrolidincarboxylat hergestellt.

MS: 734 $(M+H)^+$

Die als Ausgangsmaterial eingesetzte (αRS,S)-α-Benzyl-1-t-butoxycarbonyl-γ-oxo-2-pyrrolidinbuttersäure wurde in Analogie zur Herstellung von 2-(RS)-Benzyl-4-(4-chlorphenyl)-4-oxobuttersäure (vgl. Beispiel 27) aus N-t-Butoxycarbonyl-prolyl-brommethan(EPA 0.029.163) und Benzylmalonsäurediäthylester erhalten, MS: 362 $(M+H)^+$.

## Beispiel 31

In analoger Weise wie in Beispiel 2 beschrieben wurden durch katalytische Hydrierung von t-Butyl (S)-2-[[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]acetyl]-1-pyrrolidincarboxylat und t-Butyl (S)-2-[[(R)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]-acetyl]-1-pyrrolidincarboxylat das t-Butyl (S)-2-[[(S)-α-[[(S)-1-[[(1S,2S,4S)-1-(cyclohexylmethyl)-2-hydroxy-4-isopropylhexyl]carbamoyl]-2-imidazol-4-yl-äthyl]carbamoyl]phenäthyl]acetyl]-1-pyrrolidincarboxylat (weniger polares Isomeres) und das t-Butyl (S)-2-[[(R)-α-[[(S)-1-[[(1S,2S,4S)-1-(cyclohexylmethyl)-2-hydroxy-4-isopropylhexyl]carbamoyl]-2-imidazol-4-yläthyl]carbamoyl]phenäthyl]acetyl]-1-pyrrolidincarboxylat hergestellt.

MS: 736 $(M+H)^+$

## Beispiel 32

In analoger Weise wie in Beispiel 1 beschrieben wurde 1-[1-[(RS)-3-[[(S)-1-[[(1S,2S,4S)-1-(Cyclohexylmethyl)-2-hydroxy-4-isopropylhexyl]carbamoyl]-2-imidazol-4-yläthyl]carbamoyl]-4-phenylbutyryl]-D-prolyl]-L-prolinbenzylester als Gemisch von 2 Epimeren durch Kondensation von (S)-α-Amino-N-[(1S,2S,4S)-1-(cyclohexylmethyl)-2-hydroxy-4-isopropylhexyl]imidazol-4-propionamid mit (αRS,2R)-α-Benzyl-2-[[(S)-2-[(benzyloxy)carbonyl]-1-pyrrolidinyl]carbonyl]-γ-oxo-1-pyrrolidinbuttersäure hergestellt.
MS 867 $(M+H)^+$

Die als Ausgangsmaterial eingesetzte Säure wurde wie folgt hergestellt:

Ausgehend von 2-Benzyl-3-methoxycarbonylpropionsäure (L.D. Byers und R. Wolfenden, J. Biol. Chem., 247, 606 (1972)) wurde nach dem von U. Widmer in Synthesis 1983, Seite 135 beschriebenen Verfahren 2-Benzyl-3-methoxycarbonylpropionsäure-t-butylester hergestellt, MS: 222 $(M-C_4H_8)^+$.

415 mg des obigen t-Butylesters werden in 3 ml t-Butylalkohol gelöst und in Gegenwart von 1,5 ml 1N Natronlauge 3 Stunden bei Raumtemperatur gerührt. Dann wird die Reaktionslösung unter vermindertem Druck eingedampft, und der Rückstand in Wasser gelöst und mit Aether gewaschen. Die wässri ge Phase wird danach mit 1,5 ml 1N Salzsäure neutralisiert und mit Methylenchlorid extrahiert. Die organischen Extrakte werden über Natriumsulfat getrocknet und unter vermindertem Druck eingedampft, wobei 2-Benzyl-3-carboxypropionsäure-t-butylester als farbloses Oel erhalten wird, MS: 208 $(M-C_4H_8)^+$.

370 mg (1,4 mMol) des oben genannten Halbesters, 409 mg (1,2 mMol) H-D-Pro-Pro-OBz und 270 mg (2 Moläquivalente) Triäthylamin werden in 8 ml Dimethylformamid gelöst. Zu dieser Lösung werden 530 mg (1,4 mMol) HBTU gegeben, und das Reaktionsgemisch 15 Stunden bei Raumtemperatur gerührt und anschliessend im Hochvakuum zur Trockene eingedampft. Der Rückstand wird in Essigester gelöst und mit gesättigter Natriumbicarbonatlösung gewaschen. Die organische Phase wird über Natriumsulfat getrocknet und unter vermindertem Druck eingedampft. Chromatographie des Rückstandes an Kieselgel mit einem 98:2-Gemisch von Methylenchlorid und Methanol liefert (αRS,2R)-α-Benzyl-2-[[(S)-2-[(benzyloxy)carbonyl]-1-pyrrolidinyl]carbonyl]-γ-oxo-1-pyrrolidinbuttersäure-t-butylester als farblosen Schaum.
MS: 549 $(M+H)^+$

505 mg (0,9 mMol) des obigen t-Butylesters werden in 10 ml 2N Salzsäure/Essigsäure 3 Stunden bei Raumtemperatur gerührt. Danach wird das Reaktionsgemisch unter vermindertem Druck eingedampft, wobei man (αRS,2R)-α-Benzyl-2-[[(S)-2-[(benzyloxy)carbonyl]-1-pyrrolidinyl]carbonyl]-γ-oxo-1-pyrrolidinbuttersäure als farblosen Schaum erhält. MS: 493 $(M+H)^+$.

Das als Ausgangsmaterial eingesetzte (S)-α-Amino-N-[(1S,2S,4S)-1-(cyclohexylmethyl)-2-hydroxy-4-isopropylhexyl]imidazol-4-propionamid wurde wie folgt hergestellt:

50 mg (S)-α-Amino-N-[(1S,2S,4S)-1-(cyclohexylmethyl)-2-hydroxy-4-isopropyl-5-hexenyl]imidazol-4-propionamid werden in 3 ml Methanol gelöst und in Gegenwart von 7 mg Palladium auf Kohle bei Normaldruck 3 Stunden bei Raumtemperatur hydriert. Danach wird der Katalysator abfiltriert und mit Methanol nachgewaschen. Die alkoholische Lösung wird dann unter vermindertem Druck eingedampft, wobei man (S)-α-Amino-N-[(1S,2S,4S)-1-(cyclohexylmethyl)-2-hydroxy-4-isopropylhexyl] imidazol-4-propionamid als farblosen Schaum erhält.
MS: 393 $(M+H)^+$.

## Beispiel 33

In analoger Weise wie in Beispiel 1 beschrieben, wurden die folgenden Verbindungen hergestellt:

- Aus (S)-α-Amino-N-[(1S,2S,4S)-1-(cyclohexylmethyl)-2-hydroxy-4-isopropyl-5-hexenyl]imidazol-4-propionamid und (αRS,2R)-α-Benzyl-2-[[(S)-2-[(benzyloxy)carbonyl]-1-pyrrolidinyl]carbonyl]-γ-oxo-1-pyrrolidinbuttersäure den 1-[1-[(RS)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbutyryl]-D-prolyl]-L-prolinbenzylester als 1:1-Epimerengemisch, MS: 865 $(M+H)^+$;
- Aus (S)-α-Amino-N-[(1S,2S,4S)-1-(cyclohexylmethyl)-2-hydroxy-4-isopropyl-5-hexenyl]-imidazol-4-propionamid und N-(t-Butoxycarbonyl)-N-[2-[(RS)-3-carboxy-4-phenylbutyramido]äthyl]glycin-t-butylester die beiden epimeren Verbindungen t-Butoxycarbonyl [2-[(R und S)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyramido]äthyl]glycin-t-butylester, MS: 837 $(M+H)^+$;
- Aus (S)-α-Amino-N-[(1S,2S,4S)-1-(cyclohexylmethyl)-2-hydroxy-4-isopropyl-5-hexenyl]imidazol-4-propionamid und (R)-α-[[[2-Hydroxy-1-(hydroxymethyl)-1-methyl]carbamoyl]methyl]hydrozimtsäure das (S)-N-[-(1S,2S,4S)-1-(Cyclohexylmethyl) -2-hydroxy-4-isopropyl-5-hexenyl]-α-[(R)-α-[[[2-hydroxy-1-(hydroxymethyl)-1-methyläthyl]carbamoyl]methyl]hydrocinnamamido]imidazol-4-propionamid, MS: 668 $(M+H)^+$;

-Aus (S)-α-Amino-N-[(1S,2S,4S)-1-(cyclohexylmethyl)-2-hydroxy-4-isopropyl-5-hexenyl]imidazol-4-propionamid und 5-[(RS)-3-Carboxy-4-phenylbutyramido]valeriansäure-t-butylester das t-Butyl 5-[(RS)-3-[[(S)-1-[[-(1S,2S,4S)-1-(cyclohexylmethyl)-2-hydroxy-4-isopropyl-5-hexenyl]carbamoyl]-2-imidazol-4-yläthyl]-carbamoyl]-4-phenylbutyramido]valerat, MS: 736 $(M+H)^+$;

- Aus (S)-α-Amino-N-[(1S,2S,4S)-1(cyclohexylmethyl)-2-hydroxy-4-isopropyl-5-hexenyl]imidazol-4-propionamid und 3-Carboxy-4-phenylbutyryl-L-prolin-t-butylester den 1-[(RS)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbutyryl] -L-prolin-t-butylester, MS: 734 $(M+H)^+$;

- Aus (S)-α-Amino-N-[(1S,2S,4S)-1(cyclohexylmethyl)-2-hydroxy-4-isopropyl-5-hexenyl]imidazol-4-propionamid und (RS)-2-Benzylmalonsäuremonomethylester (F: Texier et al., Tetrahedron, 1974, 3185,) das Methyl (RS)-α-[[(S)-1-[[(1S,2S,4S)-1-(cyclohexylmethyl)-2-hydroxy-4-isopropyl-5-hexenyl]carbamoyl] -2-imidazol-4-yläthyl]carbamoyl]hydrocinnamat als 1:1-Gemisch der beiden Epimeren, MS: 580 $(M^+)$;

-Aus (S)-α-Amino-N-[(1S,2S,4S)-1-(cyclohexylmethyl)-2- -hydroxy-4-isopropyl-5-hexenyl]imidazol-4-propionamid und 3-(RS)-aethoxycarbonyl-4-(1-naphthyl)buttersäure(EPA 0.181.110) das Aethyl (RS)-α-[[[(S)-1-[[-(1S,2S,4S)-1-(cyclohexylmethyl)-2-hydroxy-4-isopropyl-5-hexenyl]carbamoyl]-2-imidazol-4-yläthyl]-carbamoyl]methyl]-1-naphthalinpropionat als 1:1 Gemisch der beiden Epimeren, MS 659 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und (RS)-[(4-Benzyl-4-piperidinyl)carbamoyl]imidazol-4-propionsäure das (RS)-α-(1-Benzyl-4-piperidinyl)-N-[(S)-1-[[(1S,2S,4S)-1-(cyclohexylmethyl)-2-hydroxy-4-isopropyl-5-hexenyl]carbamoyl]-2-imidazol-4-yläthyl]imidazol-4-propionamid als 1:1 Gemisch der beiden Epimeren, MS: 729 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und 2-(RS)-Benzyl-3-morpholinocarbonylpropionsäure und anschliessender chromatographischer Trennung der beiden Epimeren das weniger polare (S)-N-[(1S,2S,4S)-1-(Cyclohexylmethyl)-2-hydroxy-4-isopr opyl-5-hexenyl]-α-[(S)-α-[(morpholinocarbonyl)methyl]hydrocinnamamido]imidazol-4-propionamid, MS: 650 $(M+H)^+$, und das polarere (S)-N-[(1S,2S,4S)-1-(Cyclohexylmethyl)-2-hydroxy-4-isopropyl-5-hexenyl]-α-[(R)-α-[(morpholinocarbonyl)methyl]hydrocinnamamido]imidazol-4-propionamid, MS: 650 $(M+H)^+$.

Die als Ausgangsstoffe eingesetzten Säuren wurden wie folgt hergestellt:

N-(t-Butoxycarbonyl)-N-[2-(RS)-3-carboxy-4-phenylbutyramidoäthyl]glycin-t-butylester

0,84 g (2,4 mMol) N-(t-Butoxycarbonyl)-N-(2-benzyloxycarbonylaminoäthyl)glycin (U.S. Patentschrift 4.145.337) werden in 5 ml Toluol suspendiert und auf 80° erhitzt. Zu der auf diese Weise erhaltenen klaren Lösung werden innerhalb von 20 Minuten 1,96 g N,N-Dimethylformamid-di-t-butylacetal getropft. Nach beendeter Zugabe wird das Reaktionsgemisch 30 Minuten bei 80° gerührt, dann abgekühlt und nacheinander mit Wasser, gesättigter Natriumbicarbonatlösung und gesättigter Kochsalzlösung gewaschen. Die organische Phase wird dann über Natriumsulfat getrocknet und unter vermindertem Druck eingedampft, wobei man N-(t-Butoxycarbonyl)-N-(2-benzyloxycarbonylaminoäthyl)glycin-t-butylester als farbloses Oel erhält, MS: 409 $(M+H)^+$.

0,78 g (1,9 mMol) N-(t-Butoxycarbonyl)-N-(2-benzyloxycarbonylaminoäthyl)glycin-t-butylester werden in 20 ml Methanol gelöst und in Gegenwart von 0,1 g Palladium auf Kohle bei pH 4,5 hydriert. Der pH-Wert wird mittels Methanol/Salzsäure konstant gehalten. Nach beendeter Wasserstoffaufnahme wird der Katalysator abfiltriert, das Filtrat eingedampft, und der Rückstand in Methylenchlorid gelöst und mit gesättigter Natriumcarbonatlösung gewaschen. Nach dem Trocknen der organischen Phase über Natriumsulfat und Eindampfen unter vermindertem Druck erhält man N-(t-Butoxycarbonyl)-2-(2-aminoäthyl)glycin-t-butylester in Form eines Oels.

NMR (250 MHZ, TMS, $CDCl_3$): δ 1,44, 1,47 (2xs, 18H) 2,83 (s,2H) 3,36 (s,2H), 3,79, 3,87 (2xs,2H).

Kondensation von 3-Aethoxycarbonyl-4-phenylbuttersäure mit N-(t-Butoxycarbonyl)-2-(2-aminoäthyl)-glycin-t-butylester liefert N-(t-Butoxycarbonyl)-N-[2-[(RS)-3-(äthoxycarbonyl)-4-phenylbutyramido]äthyl]-glycin-t-butylester [MS: 492 $(M)^+$], der seinerseits mit 1N Natronlauge in Aethanol bei 50° zur erwünschten Säure N-(t-Butoxycarbonyl)-N-[2-[(RS)-3-carboxy-4-phenylbutyramido]äthyl]glycin-t-butylester hydrolysiert wird, MS: 408 $(M-C_4H_8)^+$.

(R)-α-[[[2-Hydroxy-1-(hydroxymethyl)-1-methyl]carbamoyl]methyl]hydrozimtsäure

3-Aethoxycarbonyl-4-phenylbuttersäure (W.G. Kofron und L.G. Wideman, J. Org. Chem. 37, 555 (1972) und 2-Amino-2-methyl-1,3-propandiol werden in Gegenwart von EDC und HOBT in Dimethylformamid bei Raumtemperatur kondensiert, wobei man (RS)-α-[[[2-Hydroxy-1-(hydroxymethyl)-1-methyl]carbamoyl]-methyl]hydrozimtsäureäthylester erhält, MS: 324 $(M+H)^+$.

Zu einer Suspension von 0,54 g (1,67 mMol) (RS)-α-[[[2-Hydroxy-1-(hydroxymethyl)-1-methyl] carbamoyl]methyl]hydrozimtsäureäthylester in 15 ml Wasser werden 50 mg α-Chymotrypsin gegeben, wobei durch Zugabe von 0,1N Natronlauge der pH-Wert bei 7,1 gehalten wird (Temperatur 25°). Nach 18 Stunden sind 9 ml 0,1N Natronlauge verbraucht, und die Reaktionsmischung wird mit Aether extrahiert. Die wässrige Phase wird mit 1N Salzsäure auf pH 4 gestellt und anschliessend unter vermindertem Druck eingedampft. Der Rückstand wird mit Essigester digeriert. Die organische Phase wird unter vermindertem Druck eingedampft, und der Rückstand an Kieselgel mit einem 9:1-Gemisch von Methylenchlorid und Methanol Flash-chromatographiert, wobei man (R)-α-[[[2-Hydroxy-1-(hydroxymethyl)-1-methyl]carbamoyl]-methyl]hydrozimtsäure als farblosen Schaum erhält, MS: 264 $(M-CH_2OH)^+$.

5-[(RS)-3-Carboxy-4-phenylbutyramido]valeriansäure-t-butylester

3-Aethoxycarbonyl-4-phenylbuttersäure und 5-Aminovaleriansäure-t-butylester (J.H.C. Nayler et al., J. Med. Chem. 20, 1445 (1977)) werden in Gegenwart von EDC und HOBT in Dimethylformamid zum 5-[(RS)-3-(Aethoxycarbonyl)-4-phenylbutyramido]valeriansäure-t-butylester kondensiert, MS: 392 $(M+H)^+$. Verseifung dieses Esters mit 1N Natronlauge in Aethanol bei 50° liefert 5-[(RS)-3-Carboxy-4-phenylbutyramido]-valeriansäure-t-butylester, MS: 307 $(M-C_4H_8)^+$

3-Carboxy-4-phenylbutyryl-L-prolin-t-butylester

Die Synthese von 3-Carboxy-4-phenylbutyryl-L-prolin-t-butylester [MS: 361 $(M)^+$] durch Kondensation von 3-Aethoxycarbonyl-4-phenylbuttersäure und L-Prolin-t-butylester zum 3-Aethoxycarbonyl-4-phenylbutyryl-L-prolin-t-butylester [MS: 389 $(M)^+$] und dessen anschliessende Verseifung erfolgt analog zur nachstehend beschriebenen Herstellung von 2-(RS)-Benzyl-3-morpholinocarbonylpropionsäure.

(RS)-[(4-Benzyl-4-piperidinyl)carbamoyl]imidazol-4-propionsäure

2 g (10 mMol) (RS)-(Imidazol-4-ylmethyl)malonsäuremonomethylester (nach allgemein bekanntem Verfahren aus Malonsäuredimethylester durch Alkylierung mit Chlormethylimidazol und anschliessende Verseifung hergestellt) und 1,91 g (10 mMol) 4-Amino-benzylpiperidin werden mit 1,91 g (10 mMol) EDC in 100 ml Dimethylformamid 72 Stunden bei Raumtemperatur gerührt. Danach wird die Reaktionslösung unter vermindertem Druck eingedampft, und der Rückstand in 100 ml Methylenchlorid gelöst und mit 30 ml Wasser gewaschen. Dann wird die organische Phase über Natriumsulfat getrocknet und zur Trockene eingedampft. Der Rückstand wird mit Aether digeriert und danach abfiltriert, wobei man (RS)-[(4-Benzyl-4-piperidinyl)carbamoyl]imidazol-4-propionsäuremethylester als farbloses Pulver erhält, MS: 370 $(M)^+$.

371 mg (1 mMol) des obigen Methylesters werden in 10 ml eines 1:1-Gemisches von Methanol und Wasser gelöst und mit 1 ml 1N Kaliumhydroxyd in Methanol 24 Stunden bei Raumtemperatur gerührt. Danach dampft man die Reaktionslösung unter vermindertem Druck ein, löst den Rückstand in Wasser und wäscht dieses mit Aether. Dann wird die wässrige Phase mit 1 Moläquivalent Salzsäure angesäuert und unter vermindertem Druck eingedampft, wobei man (RS)-[(4-Benzyl-4-piperidinyl)carbamoyl]imidazol-4-propionsäure als farbloses Oel erhält, MS: 312 $(M-CO_2)^+$.

2-(RS)-Benzyl-3-morpholinocarbonylpropionsäure

1,0 g (4,2 mMol) 3-Aethoxycarbonyl-4-phenylbuttersäure werden in 25 ml Dimethylformamid gelöst und mit 0,37 g (4,2 mMol) Morpholin, 0,81 g (4,2 mMol) EDC und 1,3 g (8,4 mMol) HOBT versetzt und 48 Stunden bei Raumtemperatur gerührt. Danach wird die Reaktionslösung im Hochvakuum eingedampft, und der Rückstand in Essigester gelöst und nacheinander mit Wasser, gesättigter Natriumbicarbonatlösung und gesättigter Kochsalzlösung gewaschen. Dann wird die organische Phase über Natriumsulfat getrocknet und unter vermindertem Druck eingedampft, und der Rückstand unter Verwendung eines 95:5-Gemisches von

Methylenchlorid und Aethanol an Kieselgel chromatographiert, wobei man 2-(RS)-1-Benzyl-3-morpholinocarbonylpropionsäureäthylester als farbloses Oel erhält, MS: 305 $(M)^+$.

0,42 g (1,4 mMol) des obigen Esters werden in 2 ml Aethanol gelöst und mit 2,1 ml (1,5 Moläquivalente) 1N Natronlauge versetzt. Die Reaktionslösung wird dann 4 Stunden bei 50° gerührt, und der Rückstand in Wasser gelöst und mit Aether gewaschen. Die wässrige Phase wird mit 2,3 ml 1N Salzsäure angesäuert und mit Methylenchlorid extrahiert. Die organischen Extrakte werden über Natriumsulfat getrocknet und unter vermindertem Druck eingedampft, wobei man 2-(RS)-Benzyl-3-morpholinocarbonylpropionsäure als farbloses Oel erhält, welches direkt in die nächste Stufe eingesetzt wird, MS: 277 $(M)^+$.

## Beispiel 34

100 mg (0,13 mMol) t-Butoxycarbonyl [2-[(RS)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyramido]äthyl]glycin-t-butylester werden in 2 ml Essigsäure gelöst und 3 Stunden mit 4 ml 1N Salzsäure/Essigsäure bei Raumtemperatur gerührt. Danach wird das Reaktionsgemisch unter vermindertem Druck eingedampft, und der Rückstand mit Aether digeriert und abfiltriert, wobei man N-[2-[(RS)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbutyramido]äthyl]glycin-dihydrochlorid als Pulver erhält, MS: 681 $(M+H)^+$.

## Beispiel 35

54 mg t-Butyl 5-[(RS)-3-[[(S)-1-[[(1S,2S,4S)-1(cyclohexylmethyl)-2-hydroxy-4-isopropyl-5-hexenyl]-carbamoyl]-2-imidazol-4-yläthyl]carbamoyl]-4-phenylbutyramido]valerat werden in 1 ml Essigsäure gelöst und bei Raumtemperatur mit 2 ml Salzsäure/Essigsäure (1,5N) versetzt und 2,5 Stunden bei dieser Temperatur gerührt. Dann wird das Reaktionsgemisch unter vermindertem Druck eingedampft, und der Rückstand mit Aether digeriert und abfiltriert, wobei man 5-[(RS)-3-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]-4-phenylbutyramido]valeriansäure-hydrochlorid als farbloses Pulver erhält, MS: 680 $(M+H)^+$.

## Beispiel 36

In analoger Weise wie in Beispiel 27 beschrieben wurden die folgenden Verbindungen hergestellt:

- Aus (S)-α-Amino-N-[(1S,2S,4S)-1-(cyclohexylmethyl)-2-hydroxy-4-isopropyl-5-hexenyl]imidazol-4-propionamid und (RS)-2-Benzyl-6,6-dimethyl-4-oxoheptansäure das (S)-N-[(1S,2S,4S)-1(Cyclohexylmethyl)-2-hydroxy-4-isopropyl-5-hexenyl]-α-[(RS)-α-(4,4-dimethyl-2-oxopentyl) hydrocinnamamido]imidazol-4-propionamid, MS: 635 $(M+H)^+$;
- Aus (S)-α-Amino-N-[(1S,2S,4S)-1-(cyclohexylmethyl)-2-hydroxy-4-isopropyl-5-hexenyl]imidazol-4-propionamid und (RS)-α-Benzyl-γ-oxo-cyclohexanbuttersäure und anschliessender Trennung der beiden Epimeren das weniger polare (S)-α-[(S)-α-[(Cyclohexylcarbonyl)methyl]hydrocinnamamido]-N-[(1S,2S,4S)-1-(cyclohexylmethyl)-2-hydroxy-4-isopropyl-5-hexenyl]imidazol-4-propionamid [MS: 647 $(M+H)^+$] und das polarere (S)-α-[(R)-α-[(Cyclohexylcarbonyl)methyl]hydrocinnamamido]-N-[(1S,2S,4S)-1-(cyclohexylmethyl)-2-hydroxy-4-isopropyl-5-hexenyl]imidazol-4-propionamid, MS: 647 $(M+H)^+$;
- Aus (S)-α-Amino-N-[(1S,2S,4S)-1-(cyclohexylmethyl)-2-hydroxy-4-isopropyl-5-hexenyl]imidazol-4-propionamid und (RS)-α-Benzyl-γ-oxo-cyclopentanbuttersäure das (S)-N-[(1S,2S,4S)-1-(Cyclohexylmethyl)-2-hydroxy-4-isopropyl-5-hexenyl]-α-[(RS-α-[(cyclopentylcarbonyl)methyl]hydrocinnamamido]imidazol-4-propionamid, MS: 633 $(M+H)^+$;
- Aus (S)-α-Amino-N-(1S,2S,4S)-1-(cyclohexylmethyl)-2-hydroxy-4-isopropyl-5-hexenyl]imidazol-4-propionamid und (RS)-2-Benzyl-5,5-dimethyl-4-oxohexansäure das (S)-α-[(RS)-2-Benzyl-5,5-dimethyl-4-oxohexananamido]-N-[(1S,2S,4S)-1-(cyclohexylmethyl)-2-hydroxy-4-isopropyl-5-hexenyl]imidazol-4-propionamid, MS: 621 $(M+H)^+$;
- Aus (S)-α-Amino-N-[(1S,2S,4S)-1-(cyclohexylmethyl)-2-hydroxy-4- isopropyl-5-hexenyl]imidazol-4-propionamid und (R)-2-Benzyl-5,5-dimethyl-4-oxohexansäure das (S)-α-[(R)-2-Benzyl-5,5-dimethyl-4-oxohexanamido]-N[(1S,2S,4S)-1-(cyclohexylmethyl)-2-hydroxy-4-isopropyl-5-hexenyl]imidazol-4-propionamid, MS: 621 $(M+H)^+$;

- Aus (S)-α-Amino-N-[(1S,2S,4S)-1-(cyclohexylmethyl)-2-hydroxy-4-isopropyl-5-hexenyl]imidazol-4-propionamid und (αRS,S)-α-Benzyl-1-(benzyloxycarbonyl)-γ-oxo-2-pyrrolidinbuttersäure das Benzyl (S)-2-[[(RS)-α-[[(S)-1-[[(1S,2S,4S)-1-(cyclohexylmethyl)-2-hydroxy-4-isopropyl-5-hexenyl]carbamoyl]-2-imidazol-4-yläthyl]-carbamoyl]phenäthyl]acetyl]-1-pyrrolidincarboxylat, MS: 768 $(M+H)^+$.

Die als Ausgangsstoffe eingesetzten Säuren wurden in Analogie zur Herstellung von 2-(RS)-Benzyl-4-(4-chlorphenyl)-4-oxobuttersäure (vgl. Beispiel 27) wie folgt hergestellt:

- Aus 1-Chlor-4,4-dimethyl-2-pentanon, welches seinerseits in an sich bekannter Weise aus 3,3-Dimethylbuttersäurechlorid durch Umsetzen mit Diazomethan und Chlorwasserstoff hergestellt wurde, und Benzylmalonsäurediäthylester die (RS)-2-Benzyl-6,6-dimethyl-4-oxoheptansäure;
- Aus Bromacetylcyclohexan (M. Gaudry und A. Marquet, Tetrahedron, 1970, 5611) und Benzylmalonsäurediäthylester die (RS)-α-Benzyl-γ-oxocyclohexanbuttersäure;
- Aus Bromacetylcyclopentan (M. Gaudry und A. Marquet, Tetrahedron, 1970, 5611) und Benzylmalonsäurediäthylester die (RS)-α-Benzyl-γ-oxocyclopentanbuttersäure;
- Aus N-Benzyloxycarbonyl-prolyl-brommethan, welches in Analogie zur Herstellung von N-t-Butoxycarbonyl-prolyl-brommethan (EPA 0.129.163) hergestellt wurde, und Benzylmalonsäurediäthylester die (αRS,S)-α-Benzyl-1-(benzyloxcarbonyl)-γ-oxo-2-pyrrolidinbuttersäure.

Die ebenfalls als Ausgangsstoffe eingesetzte (RS)-2-Benzyl-5,5-dimethyl-4-oxohexansäure und die enantiomere (R)-2-Benzyl-5,5-dimethyl-4-oxohexansäure sind aus der europäischen Patentpublikation 0 184 550 bekannt.

## Beispiel 37

In analoger Weise wie in Beispiel 2 beschrieben wurden die folgenden Verbindungen durch katalytische Hydrierung der entsprechenden Olefine hergestellt:

- (S)-N-[(1S,2S,4S)-1-(Cyclohexylmethyl) -4-äthyl-2-hydroxy-5-methylhexyl]-α-[(R)-α-[(morpholinocarbonyl)-methyl]hydrocinnamamido]imidazol-4-propionamid, MS: 652 $(M+H)^+$;
- (S)-α-[(R)-α-(3,3-Dimethyl-2-oxobutyl)hydrocinnamamido]-N-[(1S,2S,4S)-1-(cyclohexylmethyl)-2-hydroxy-4-isopropylhexyl]imidazol-4-propionamid, MS: 623 $(M+H)^+$;
- (S)-N-[(1S,2S,4S)-1-(Cyclohexylmethyl)-2-hydroxy-4-isopropylhexyl]-α-[[[[(R)-α-2-hydroxy-1-(hydroxymethyl)-1-methyläthyl]carbamoyl]methyl]hydrocinnamamido]imidazol-4-propionamid, MS: 620 $(M+H)^+$;
- N-[(S)-1-[[(1S,2S,4S)-1-(Cyclohexylmethyl)-2-hydroxy-4-isopropylhexyl]carbamoyl]-2-imidazol-4-yläthyl]-γ-oxo-α-(1-naphthylmethyl)-4-morpholinbutyramid, MS: 702 $(M+H)^+$.

## Beispiel 38

90 mg (0.23 mMol) (S)-α-Amino-N-[(1S,2S,4S)-1-(cyclohexylmethyl)-2-hydroxy-4-isopropyl-5-hexenyl]-imidazol-4- propionamid und 0.047 ml Hünigbase in 2 ml Acetonitril werden bei Raumtemperatur tropfenweise mit einer Lösung von 60,7 mg (S)-α-[(3,3-Dimethylbutyryl)oxy]hydrozimtsäure und 101 mg BOP in 5 ml Acetonitril versetzt. Man rührt 4 Stunden bei Raumtemperatur, giesst anschliessend in 2N Natriumbicarbonatlösung und extrahiert mit Methylenchlorid. Die organische Phase wird mit Ammoniumchloridlösung gewaschen und über Natriumsulfat getrocknet. Das Lösungsmittel wird unter vermindertem Druck entfernt, und der Rückstand an Kieselgel chromatographiert, wobei man nach Eluieren mit einem 20:1-Gemisch von Methylenchlorid und Methanol 50 mg (34 %) (S)-α-[[(S)-1-. [[(1S,2S,4S)-1-(Cyclohexylmethyl)-2-hydroxy-4-isopropyl-5-hexenyl]carbamoyl]-2-imidazol-4-yläthyl]carbamoyl]phenäthyl-3,3-dimethylbutyrat als gelbes Oel erhält.

MS: 637 $(M+H)^+$

Die als Ausgangsmaterial eingesetzte (S)-α-[(3,3-Dimethylbutyryl)oxy]hydrozimtsäure wurde wie folgt hergestellt:

2,1 g (8,2 mMol) (S)-2-Hydroxy-3-phenylpropionsäurebenzylester (Isv. Acad. Navk SSR, Ser. Khim. 1966(3), 519) und 1,24 ml Triäthylamin in 100 ml Methylenchlorid werden bei Raumtemperatur innerhalb von 2 Stunden tropfenweise mit 1,26 ml t-Butylacetylchlorid versetzt. Danach wird das Reaktionsgemisch 6

Stunden bei 40° gerührt. Anschliessend wird auf Wasser gegossen und mit Methylenchlorid extrahiert. Nach dem Trocknen über Natriumsulfat wird das Lösungsmittel unter vermindertem Druck entfernt, und das Rohprodukt durch Flash-chromatographie an Kieselgel mit einem 1:3-Gemisch von Aether und Petroläther gereinigt, wobei man 1,4 g (48 %) (S)-α-[(2,3-Dimethylbutyryl)oxy]hydrozimtsäurebenzylester erhält.
MS: 238 $[M-(CH_3)_3CCH_2COOH]^+$

Die oben erhaltenen 1,4 g (3,95 mMol) des Benzylesters werden in 80 ml Aethanol gelöst und 2 Stunden bei Raumtemperatur und in Gegenwart von 0,4 g Palladium auf Kohle hydriert. Abfiltrieren des Katalysators und Entfernen des Lösungsmittels unter vermindertem Druck liefert 1 g (96 %) (S)-α-[(3,3-Dimethylbutyl)oxy]hydrozimtsäure als farbloses Oel, welches direkt in die nächste Stufe eingesetzt wird.

## Beispiel 39

100 mg (0,394 mMol) (αS,βS)-β-Amino-α-[(S)-2-isopropyl-3-butenyl]cyclohexanpropanol in einem Gemisch von 3,5 ml Dimethylformamid und 7 ml Acetonitril werden bei Raumtemperatur mit 0,079 ml Hünigbase ersetzt. Danach gibt man 174 mg BOP und 150 mg (0,394 mMol) (S)-α-(Dibenzylacetoxy)-imidazol-4-propionsäure zu und rührt die Lösung 6 Stunden bei Raumtemperatur. Danach verdünnt man mit 60 ml Essigester und wäscht mit 1N Salzsäure und Natriumbicarbonatlösung. Dann trocknet man die organische Phase über Natriumsulfat und entfernt das Lösungsmittel unter vermindertem Druck. Der Rückstand wird mit einem 10:1-Gemisch von Methylenchlorid und Methanol an Kieselgel chromatographiert, wobei man 179 mg (S)-1-[[(1S,2S,4S)-1-(Cyclohexylmethyl)-2-hydroxy-4-isopropyl-5-hexenyl]carbamoyl]-2-(imidazol-4-yl)äthyldibenzylacetat als amorphes Pulver erhält.
MS: 596 $(M-OH)^+$

Die oben als Ausgangsmaterial eingesetzte (S)-α-(Dibenzylacetoxy)imidazol-4-propionsäure wurde wie folgt hergestellt:

Man leitet bei 0° während 3 Minuten trockenes Chlorwasserstoffsäuregas durch eine Suspension von 2,5 g α-Hydroxyimidazolpropionsäure (C.E. Baker und A. Merster, JACS 73, 1336) in 80 ml Benzylalkohol, und lässt die erhaltene Lösung anschliessend 12 - 20 Stunden bei Raumtemperatur stehen. Danach wird der überschüssige Benzylalkohol in Hochvakuum abdestilliert, und der Rückstand in Methylenchlorid gelöst und mit Natriumbicarbonatlösung gewaschen. Die organische Phase wird über Natriumsulfat getrocknet und unter vermindertem Druck eingedampft. Der Rückstand wird durch Flash-Chromatographie mit einem 10:1-Gemisch von Methylenchlorid und Methanol an Kieselgel gereinigt, wobei man 3,5 g (89 %) (S)-α-Hydroxyimidazolpropionsäurebenzylester als gelbes Oel erhält.
MS: 246 $(M)^+$

Zu 10,53 g (43,8 mMol) Dibenzylessigsäure in 50 ml Pyridin tropft man bei -5° 6,19 ml (48,3 mMol) Benzolsulfonylchlorid zu, und rührt die Reaktionslösung 30 Minuten bei Raumtemperatur. Anschliessend gibt man eine Lösung von 6 g (24,4 mMol) (S)-α-Hydroxyimidazolpropionsäurebenzylester in 5 ml Pyridin tropfenweise bei -5° zu und rührt die Lösung zunächst 2 Stunden bei 0° und danach weitere 2 Stunden bei Raumtemperatur. Dann wird das Reaktionsgemisch auf 3N Salzsäure/Eis gegossen und mit Essigester extrahiert. Die organischen Extrakte werden danach mit 2N Natriumbicarbonatlösung gewaschen, über Natriumsulfat getrocknet und unter vermindertem Druck eingedampft. Der Rückstand wird an Kieselgel unter Verwendung eines 15:1-Gemisches von Methylenchlorid und Essigester Flash-chromatograhiert, wobei man 6,35 g (47 %) eines 1:1-Gemisches von (S)-α-Dibenzylacetoxy-N-(phenylsulfonyl)imidazol-4-propionsäurebenzylester und (S)-α,N-Bis(dibenzylacetoxy)imidazol-4-propionsäurebenzylester als gelbes Oel erhält, welches ohne weitere Reinigung in die nächste Stufe eingesetzt wird.

Zu 5,74 g (9,4 mMol) des obigen Gemisches in 25 ml Methanol gibt man 95 ml einer 0,3N Sazlsäure/Methanol-Lösung und rührt 2 Stunden bei Raumtemperatur. Danach giesst man auf Eis/Natriumbicarbonat und extrahiert das Produkt mit Essigester, trocknen über Natriumsulfat und entfernt das Lösungsmittel unter vermindertem Druck. Der Rückstand wird durch Flash-Chromatographie an Kieselgel gereinigt, wobei man 3,2 g (70 %) (S)-α-(Dibenzylacetoxy)imidazol-4-propionsäurebenzylester erhält.
MS = 468 $(M)^+$

3,2 g (6,83 mMol) (S)-α-(Dibenzolacetoxy)imidazol-4-propionsäurebenzylester in 150 ml Methanol werden 2 Stunden bei Raumtemperatur in Gegenwart von 660 mg Palladium auf Kohle hydriert. Danach wird der Katalysator abfiltriert, und der Filterrückstand mehrmals mit einem 1:1-Gemisch von Methylenchlorid und Methanol gewaschen. Danach wird die Lösung unter vermindertem Druck soweit eingeengt, bis Kristallisation eintritt. Umkristallisation des Kristallisates aus Methylenchlorid/Methanol liefert 2,5 g (96 %) (S)-α-(Dibenzylacetoxy)imidazol-4-propionsäure, Schmelzpunkt 210°.

Beispiel 40

Zu 236,2 mg (1 mMol) R-(+)-α-Benzylbernsteinsäuremonoäthylester(S.G. Cohen, A. Milovanovic, JACS 90, 3495 (1968)) und 390,6 mg (1 mMol) (S)-α-Amino-N-[(1S,2S,4S)-1-(cyclohexylmethyl)-2-hydroxy-4-isopropyl-5-hexenyl]imidazol-4-propionamid in Acetonitril werden 442 mg (1 mMol) BOP und 0,204 ml Hünigbase gegeben, und die erhaltene Lösung 20 Stunden bei Raumtemperatur gerührt. Danach wird das Reaktionsgemisch auf 1N Salzsäure/ Eis gegossen und mit Essigester extrahiert. Die organischen Extrakte werden mit Natriumbicarbonatlösung gewaschen, über Natriumsulfat getrocknet und eingedampft. Der Rückstand wird mit einem 10:1-Gemisch von Methylenchlorid und Methanol als Eluierungsmittel an Kieselgel chromatographiert, wobei man 465 mg (76 %) Aethyl (R)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyrat als amorphes Pulver erhält.
MS: 609 $(M+H)^+$

Beispiel 41

Zu 66,5 mg (0,404 mMol) (S)-(+)-α-Methylhydrozimtsäure (A.W. Schrecker, J. Org. Chem. 22, 33 (1957)) und 157 mg (0,404 mMol) (S)-α-Amino-N-[(1S,2S,4S)-1-(cyclohexylmethyl)-2-hydroxy-4-isopropyl-5-hexenyl]imidazol-4-propionamid in 10 ml Acetonitril gibt man 181 mg (0,404 mMol) BOP und 0,083 ml Hünigbase und rührt 16 Stunden bei Raumtemperatur. Danach wird die Reaktionslösung auf 1N Salzsäure/Eis gegossen und mit Essigester extrahiert. Die organischen Extrakte werden mit Natriumbicarbonatlösung gewaschen und über Natriumsulfat getrocknet. Dann wird unter vermindertem Druck das Lösungsmittel entfernt, und der Rückstand an Kieselgel mit einem 10:1-Gemisch von Methylenchlorid und Methanol chromatographiert, wobei man 136 mg (63 %) N-[(1S,2S)-1-(Cyclohexylmethyl)-2-hydroxy-4-isopropyl-5-hexenyl]-α-[(S)-α-methylhydrocinnamamido]imidazol-4-propionamid als amorphen Festkörper erhält. MS: 537 $(M+H)^+$

Beispiel 42

Zu 73 mg α-Methylzimtsäure und 117,75 mg (S)-α-Amino-N-[(1S,2S,4S)-1-(cyclohexylmethyl)-2-hydroxy-4-isopropyl-5-hexenyl]imidazol-4-propionamid in 5 ml Acetonitril werden 135 mg BOP und 0,062 ml Hünigbase gegeben und 20 Stunden bei Raumtemperatur gerührt. Danach wird die Reaktionslösung auf 3N Salzsäure/Eis gegossen und mit Essigester extrahiert. Die organische Phase wird mit 2N Natriumcarbonatlösung gewaschen und über Natriumsulfat getrocknet. Dann wird das Lösungsmittel unter vermindertem Druck entfernt, und der Rückstand mit einem 10:1-Gemisch von Methylenchlorid und Methanol an Kieselgel chromatographiert, wobei man 100 mg N-[[(1S,2S,4S)-1-(Cyclohexylmethyl)-2-hydroxy-4-isopropyl-5-hexenyl]-α-[α-methylcinnamoyl]amino]-imidazol-4-propionamid als amorphen Festkörper erhält.
MS: 535 $(M+H)^+$

Beispiel 43

106 mg (0.404 mMol) t-Butoxycarbonyl-α,β-dehydrophenylalanin (H. Poisel, Chem. Ber. 110, 948 (1977)) und 157 mg (S)-α-Amino-N-[(1S,2S,4S)-1-(cyclohexylmethyl)-2-hydroxy-4-isopropyl-5-hexenyl]-imidazol-4-propionamid in 10 ml Acetonitril werden mit 181 mg BOP und 0,083 ml Hünigbase versetzt und anschliessend 16 Stunden bei Raumtemperatur gerührt. Danach giesst man die erhaltene Lösung auf 1N Salzsäure/Eis, extrahiert mit Essigester, wäscht mit 2N Natriumcarbonatlösung, trocknet über Natriumsulfat und entfernt das Lösungsmittel unter vermindertem Druck. Der Rückstand wird mit einem 20:1-Gemisch von Methylenchlorid und Methanol an Kieselgel chromatographiert, wobei man 144 mg (54 %) (S)-α-[α-(t-Butoxycarbonylamino)cinnamoyl]-N-[(1S,2S,4S)-1-(cyclohexylmethyl)-2-hydroxy-4-isopropyl-5-hexenyl]-imidazol-4-propionamid als amorphen Festkörper erhält.
MS: 636 $(M+H)^+$

Beispiel 44

100 mg (0,256 mMol) (S)-α-Amino-N-[(1S,2S,4S)-1-(cyclohexylmethyl)-2-hydroxy-4-isopropyl-5-hexenyl]imidazol-4-propionamid und 0.052 ml Hünigbase in 2 ml Acetonitril werden mit 92 mg (S)-α-(Diphenylacetoxy)hydrozimtsäure und 113 mg BOP in 2 ml Acetonitril versetzt und 12 Stunden bei Raumtemperatur gerührt. Dann giesst man die Reaktionslösung auf 1N Salzsäure/Eis, extrahiert mit Essigester, wäscht die organische Phase mit Natriumcarbonatlösung, trocknet über Natriumsulfat und entfernt schliesslich das Lösungsmittel unter vermindertem Druck. Der Rückstand wird an Kieselgel mit einem 10:1-Gemisch von Methylenchlorid und Methanol chromatographiert, wobei man 130 mg (S)-α-[[(S)-1-[[(1S,2S)-1-(Cyclohexylmethyl)-2-hydroxy-4-isopropyl-5-hexenyl]carbamoyl]-2-imidazol-4-yläthyl]-carbamoyl]phenäthyldibenzylacetat in Form eines amorphen Festkörpers erhält.
MS: 733 $(M+H)^+$

Die als Ausgangsstoff verwendete (S)-α-(Diphenylacetoxy)hydrozimtsäure wurde wie folgt hergestellt:

Man versetzt 1,06 g (5 mMol) Diphenylessigsäure in 5 ml Pyridin bei -5° tropfenweise mit 0,833 ml (6,5 mMol) Benzolsulfonylchlorid und rührt die Lösung anschliessend 30 Minuten bei Raumtemperatur. Dann gibt man 1,28 g (5 mMol) (S)-2-Hydroxy-3-phenylpropionsäurebenzylester (Isv. Acad. Navk SSR, Ser. Khim. 1966(3), 519) in 5 ml Pyridin zu und rührt 2 Stunden bei 0-10° und weitere 2 Stunden bei Raumtemperatur. Die Reaktionslösung wird mit Wasser versetzt und mit Essigester extrahiert. Die organischen Phasen werden mit 1N Salzsäure und Natriumbicarbonatlösung gewaschen und über Natriumsulfat getrocknet. Das Lösungsmittel wird unter vermindertem Druck abgedampft, und der Rückstand an Kieselgel mit einem 4:1-Gemisch von Petroläther/Aether Flash-chromatographiert, wobei man 1,8 g (80 %) Benzyl-(S)-α-(Diphenylacetoxy)hydrocinnamat in Form eines gelben Oels erhält.
MS: 359 $(M\text{-Benzyl})^+$

Die erhaltenen 1,8 g des oben genannten Benzylesters werden in 180 ml Essigester gelöst, mit 300 mg Palladium auf Kohle versetzt und bis zur Aufnahme der theoretischen Menge Wasserstoff bei Raumtemperatur hydriert (ungefähr 2 Stunden). Der Katalysator wird dann abfiltriert und das Lösungsmittel unter vermindertem Druck entfernt, wobei die erwünschte Säure langsam auszukristallisieren beginnt. Abfiltrieren der ausgefallenen Kristalle liefert 1,3 g (90 %) (S)-α-(Diphenylacetoxy)hydrozimtsäure.
MS: kein Molpeak
212 $(\text{Diphenylessigsäure})^+$
167 $(\text{Diphenylmethyl})^+$

Beispiel 45

100 mg (αS,βS)-β-Amino-α-[(S)-2-isopropyl-3-butenyl]cyclohexanpropanol in 20 ml Acetonitril werden mit 0.080 ml Hünigbase, 174 mg BOP und 140 mg (S)-3-Carbamoyl-2-(dibenzylacetoxy)propionsäure versetzt und 12 Stunden bei Raumtemperatur gerührt. Danach giesst man die Reaktionslösung auf 1N Salzsäure/Eis, extrahiert sie mit Essigester, wäscht die Extrakte mit Natriumcarbonatlösung und trocknet diese schliesslich über Natriumsulfat. Dann wird unter vermindertem Druck das Lösungsmittel entfernt, und der Rückstand mit einem 20:1-Gemisch von Methylenchlorid und Methanol an Kieselgel chromatographiert, wobei man 142 mg (60 %) (S)-2-Carbamoyl-1-[[(1S,2S,4S)-1-(cyclohexylmethyl)-2-hydroxy-4-isopropyl-5-hexenyl]carbamoyl]äthyldibenzylacetat in Form eines Harzes erhält.
MS: 591 $(M+H)^+$

Die als Ausgangsstoff verwendete (S)-3-Carbamoyl-2-(dibenzylacetoxy)propionsäure wurde wie folgt hergestellt:

480 mg (2 mMol) Dibenzylessigsäure in 3 ml Pyridin werden bei -5° mit 0,34 ml Benzolsulfonylchlorid versetzt und anschliessend 30 Minuten bei Raumtemperatur gerührt. Danach werden 447 mg (2 mMol) Benzyl (S)-β-malamidat zugegeben, und die Reaktionslösung wird zunächst 2 Stunden bei 0° und dann weitere 2 Stunden bei Raumtemperatur gerührt. Dann verdünnt man die Reaktionslösung mit 100 ml Essigester, gibt verdünnte Salzsäure zu und trennt die beiden erhaltenen Phasen. Die organische Phase wird mit Natriumcarbonatlösung gewaschen, über Natriumsulfat getrocknet und unter vermindertem Druck eingedampft. Chromatographie des Rückstandes an Kieselgel unter Verwendung eines 4:1-Gemisches von Methylenchlorid und Essigester als Eluierungsmittel liefert 539 mg (59 %) Benzyl (S)-3-carbamoyl-2-(dibenzylacetoxy)propionat in Form eines gelben Oels.
MA: 354 $(M\text{-Benzyl})^+$

520 mg des oben genannten Benzylesters in 5 ml Methanol werden in Gegenwart von 100 mg Palladium auf Kohle bei Raumtemperatur während zwei Stunden hydriert. Danach wird der Katalysator abfiltriert und das Lösungsmittel unter vermindertem Druck entfernt, wobei man 370 mg (90 %) (S)-3-Carbamoyl-2-(dibenzylacetoxy)propionsäure als weissen Festkörper erhält.
MS: 338 $(M-NH_3)^+$

## Beispiel 46

100 mg (0.256 mMol) (S)-α-Amino-N-[(1S,2S,4S)-1-(cyclohexylmethyl)-2-hydroxy-4-isopropyl-5-hexenyl]imidazol-4-propionamid und 0.052 ml Hünigbase in 2 ml Acetonitril werden bei Raumtemperatur mit 100 mg N-(Dibenzylcarbamoyl)-3-phenyl-L-alanin und 113 mg BOP versetzt und anschliessend 12 Stunden bei Raumtemperatur gerührt. Danach giesst man die Reaktionslösung auf 1N Salzsäure/Eis, extrahiert mit Essigester, trocknet die Extrakte üBer Natriumsulfat und entfernt das Lösungsmittel unter vermindertem Druck. Chromatographie des Rückstands an Kieselgel unter Verwendung eines 10:1-Gemisches von Methylenchlorid und Methanol als Eluierungsmittel liefert 130 mg 1,1-Dibenzyl-3-[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-phenäthyl]harnstoff in Form eines amorphen Festkörpers.
MS: 761 $(M+H)^+$

Das als Ausgangsstoff verwendete N-(Dibenzylcarbamoyl)-3-phenyl-L-alanin wurde wie folgt hergestellt:

1 g (5,06 mMol) Dibenzylamin und 1,73 ml (10,1 mMol) Hünigbase in 50 ml Methylenchlorid werden tropfenweise unter Eiskühlung mit 2,6 ml Phosgen in Toluol (20 %, 5,06 mMol) versetzt, und die erhaltene Lösung 3 Stunden bei 0° gerührt. Dann gibt man 1,1 g L-Phenylalaninmethylester zu und erhitzt 12 Stunden auf 40°. Danach giesst man das Reaktionsgemisch in Wasser, extrahiert mit Methylenchlorid und trocknet die Extrakte über Natriumsulfat. Dann wird das Lösungsmittel unter vermindertem Druck entfernt, und der Rückstand an Kieselgel unter Verwendung eines 15:1-Gemisches von Methylenchlorid und Aether als Eluierungsmittel chromatographiert, wobei man 900 mg (45 %) N-(Dibenzylcarbamoyl)-3-phenyl-L-alaninmethylester als weissen Festkörper erhält.
MS: 402 $(M)^+$

900 mg (2,23 mMol) des oben genannten Methylesters in 20 ml Aethanol werden mit 9 ml 0.5N Natronlauge (4.46 mMol) versetzt und 1 Stunde auf 40° erhitzt. Danach wird die Reaktionslösung angesäuert, und das Produkt mit Methylenchlorid extrahiert. Die organische Phase wird über Natriumsulfat getrocknet, und das Lösungsmittel unter vermindertem Druck entfernt, wobei man 100 mg (92 %) N-(Dibenzylcarbamoyl)-3-phenyl-L-alanin als amorphen Festkörper erhält.
MS: 388 $(M)^+$

## Beispiel 47

100 mg (0,52 mMol) (S)-α-Amino-N-[(1S,2S,4S)-1-(cyclohexylmethyl)-2-hydroxy-4-isopropyl-5-hexenyl]-imidazol-4-propionamid und 0,052 ml Hünigbase in 2 ml Acetonitril werden bei Raumtemperatur mit 99 mg N-(Morpholinocarbamoyl)-3-phenyl-L-alanin und 113 mg BOP in 2 ml Acetonitril versetzt und 12 Stunden bei Raumtemperatur gerührt. Danach giesst man die Reaktionslösung auf 1N Salzsäure/Eis, extrahiert diese mit Essigester, trocknet die organischen Extrakte über Natriumsulfat und entfernt das Lösungsmittel unter vermindertem Druck. Danach chromatographiert man den Rückstand mit einem 10:1-Gemisch von Methylenchlorid und Methanol an Kieselgel, wobei man 130 mg (67 %) (S)-N-[(1S,2S,4S)-1-(Cyclohexylmethyl)-2-hydroxy-4-isopropyl-5-hexenyl]-α-2-[N-(morpholinocarbamoyl)-3-phenyl-L-alanyl]amino]imidazol-4-propionamid als amorphen Festkörper erhält.
MS:651 $(M+H)^+$

Das als Ausgangsstoff eingesetzte N-(Morpholinocarbamoyl)-3-phenyl-L-alanin wurde wie folgt hergestellt:

Zu 0,6 ml (6,95 mMol) Morpholin und 2,4 ml (13,9 mMol) Hünigbase in 20 ml Methylenchlorid gibt man tropfenweise unter Eiskühlung 3,6 ml Phosgen in Toluol (20 %, 6,95 mMol) und rührt die Lösung 3 Stunden bei 0°. Danach gibt man 1,5 g (6,95 mMol) Phenylalaninmethylester-hydrochlorid zu und rührt die Reaktionslösung 12 Stunden bei 40°. Anschliessend giesst man die Reaktionslösung auf Eis und extrahiert sie mit Methylenchlorid. Die organische Phase wird über Natriumsulfat getrocknet und unter vermindertem

Druck eingedampft. Chromatographie des Rückstandes an Kieselgel unter Verwendung eines 2:1-Gemisches von Methylenchlorid und Aether als Eluierungsmittel liefert 400 mg (20 %) N-(Morpholinocarbamoyl)-3-phenyl-L-alaninmethylester in Form eines Harzes.
MS: 292 $(M)^+$

400 mg (1,37 mMol) des oben erwähnten Methylesters in 10 ml Aethanol werden mit 5,5 ml 0,5N Natronlauge (2,75 mMol) versetzt. Dann rührt man das Reaktionsgemisch 1 Stunde bei 40°, giesst es anschliessend auf 3N Salzsäure/Eis, extrahiert es mit Methylenchlorid, trocknet die organischen Extrakte über Natriumsulfat und entfernt schliesslich das Lösungsmittel unter vermindertem Druck. So erhält man 210 mg (55 %) N-(Morpholinocarbamoyl)-3-phenyl-L-alanin in Form eines amorphen Festkörpers.
MS: 278 $(M)^+$

## Beispiel 48

Man rührt eine Lösung von 100 mg (0,256 mMol) (S)-α-Amino-N-[(1S,2S,4S)-1-(cyclohexylmethyl)-2-hydroxy-4-isopropyl-5-hexenyl]imidazol-4-propionamid, 0,052 ml Hünigbase, 113 mg BOP und 81 mg N-(t-Butoxycarbonyl)-β-pyrazol-1-yl-L-alanin (L.D. Arnold, T.H. Kalantar, J.C. Vederas, JACS 107, 7105 (1985)) in Acetonitril 12 Stunden bei Raumtemperatur. Danach giesst man das Reaktionsgemisch auf 1N Salzsäure/Eis, extrahiert es mit Essigester, trocknet die Extrakte über Natriumsulfat und entfernt das Lösungsmittel schliesslich unter vermindertem Druck. Chromatographie des Rückstands an Kieselgel mit einem 7:1-Gemisch von Methylenchlorid und Methanol liefert 80 mg (50 %) weisse Kristalle von t-Butyl [(S)-1-[(1S,2S,4S)-1-(Cyclohexylmethyl)-2-hydroxy-4-isopropyl-5-hexenyl]carbamoyl]-2-imidazol-4-yläthyl]-carbamoyl-2-pyrazol-1-yläthyl]carbamat.
MS: 628 $(M+H)^+$

## Beispiel 49

Zu 100 mg (0.256 mMol) (S)-α-Amino-N-[(1S,2S,4S)-1-(cyclohexylmethyl)-2-hydroxy-4-isopropyl-5-hexenyl]imidazol-4-propionamid 0.052 ml Hünigbase in 5 ml Acetonitril gibt man eine Lösung von 103 mg N-[[2-(4-Biphenyloxy)äthyl]carbamoyl]-3-phenyl-L-alanin und 113 mg BOP in Acetonitril und rührt anschliessend 12 Stunden bei Raumtemperatur. Anschliessend giesst man das Reaktionsgemisch auf 1N Salzsäure/Eis, extrahiert es mit Essigester, trocknet die Extrakte über Natriumsulfat und dampft unter vermindertem Druck zur Trockene ein. Durch Chromatographie an Kieselgel mit einem 10:1-Gemisch von Methylenchlorid und Methanol wird das Rohprodukt gereinigt, wobei man 90 mg (45 %) 1-[2-(4-Biphenylyloxy)äthyl]-3-[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]harnstoff als amorphes Pulver erhält.
MS: 777 $(M+H)^+$

Die als Ausgangsstoff eingesetzte N-[[2-(4-Biphenyloxy)äthyl]carbamoyl]-3-phenyl-L-alanin wurde wie folgt hergestellt:

Zu 3 g (14,06 mMol) 2-(4-Biphenyloxy)äthylamin (DOS 2.500.692) in 100 ml Toluol gibt man bei 0° 4,8 ml Hünigbase und 7 ml einer 20 %-igen Phosgenlösung in Toluol und rührt das Reaktionsgemisch anschliessend eine Stunde bei 0°. Danach gibt man 6 g L-Phenylalaninbenzylester zu und rührt die Lösung 12 Stunden bei 90°. Danach wird das Reaktionsgemisch in üblicher Weise aufgearbeitet und das Rohprodukt durch Chromatographie mit einem 10:1-Gemisch von Methylenchlorid und Methanol an Kieselgel gereinigt, wobei man 4,5 g (67 %) N-[[2-(4-Biphenyloxy)äthyl]carbamoyl]-3-phenyl-L-alaninbenzylester erhält.

4,5 g (9,4 mMol) des oben genannten Benzylesters in 250 ml Aethanol wird in Gegenwart von 0,5 g Palladium auf Kohle bis zur Aufnahme der theoretischen Wasserstoffmenge bei Raumtemperatur hydriert. Danach wird der Katalysator abfiltriert, das Lösungsmittel unter vermindertem Druck abgedampft, und der Rückstand aus Aether/Methylenchlorid/Methanol umkristallisiert, wobei man 2,1 g (55 %) N-[[2-(4-Biphenylyloxy)äthyl]carbamoyl]-3-phenyl-L-alanin erhält, Schmelzpunkt 175°;
$[\alpha]^{20}_{589} = +8,4^\circ$ (c = 1 %, Methanol).

## Beispiel 50

Zu 100 mg (0,256 mg) (S)-α-Amino-N-[(1S,2S,4S)-1-(cyclohexylmethyl)-2-hydroxy-4-isopropyl-5-hexenyl]imidazol-4-propionamid und 0,052 ml Hünigbase in 5 ml Acetonitril gibt man eine Lösung von 100 mg (S)-α-[(Dibenzylcarbamoyl)oxy]hydrozimtsäure und 113 mg BOP in 2 ml Acetonitril und rührt anschliessend 12 Stunden bei Raumtemperatur. Die Aufarbeitung erfolgt in üblicher Weise. Man erhält so 150 mg (77 %) (S)-α-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]phenäthyldibenzylcarbamat.
MS: 762 $(M+H)^+$

Die als Ausgangsstoff verwendete (S)-α-(Dibenzylcarbamoyloxy)hydrozimtsäure wurde wie folgt hergestellt:

Zu 1,0 g (3,9 mMol) (S)-2-Hydroxy-3-phenylpropionsäure und 1,68 ml (3,9 mMol) Hünigbase in 20 ml Tetrahydrofuran tropft man unter Eiskühlung 2,23 ml (4,3 mMol) einer 20 %-igen Phosgenlösung in Toluol zu und rührt anschliessend 1,5 Stunden bei 0-10°. Dann gibt man tropfenweise 0,824 ml (4,3 mMol) Dibenzylamin in 20 ml Tetrahydrofuran zu und rührt die Reaktionslösung weitere 12 Stunden bei Raumtemperatur. Anschliessend giesst man diese auf Eis, neutralisiert mit Kaliumbicarbonatlösung und extrahiert mit Essigester. Das erhaltene Rohprodukt wird zur Reinigung an Kieselgel unter Verwendung eines 3:1-Gemisches von Petroläther und Aether Flash-chromatographiert, wobei man 825 mg (42 %) (S)-α-[(Benzyloxy)carbonyl]phenäthyldibenzylcarbamat als Harz erhält.
MS: 388 $(M\text{-Benzyl})^+$

0,8 g des oben beschriebenen Benzylesters wird in 30 ml eines 4:1-Gemisches von Methanol und Essigester in Gegenwart von 80 mg Palladium auf Kohle 2,5 Stunden bei Raumtemperatur hydriert. Danach wird der Katalysator abfiltriert, und das Lösungsmittel unter vermindertem Druck abgedampft. Dabei erhält man 461 mg (73 %) (S)-α-[(Dibenzylcarbamoyl)oxy]hydrozimtsäure in Form eines Harzes.
MS: 389 $(M)^+$

## Beispiel 51

Man sättigt eine Lösung von 100 mg Aethyl (R)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyrat in 30 ml Methanol während 1-2 Minuten mit Ammoniak und lässt anschliessend 12 Stunden bei Raumtemperatur rühren. Danach wird das Lösungsmittel unter vermindertem Druck entfernt, und der Rückstand mit 10 % Methanol in Methylenchlorid als Eluierungsmittel an Kieselgel chromatographiert, wobei man 63 mg (66 %) (S)-α-[(R)-α-(Carbamoylmethyl)hydrocinnamamido]-N-[(1S,2S,4S)-1-(cyclohexylmethyl)-2-hydroxy-4-isopropyl-5-hexenyl]imidazol-4-propionamid als Harz erhält.
MS: 580 $(M+H)^+$

## Beispiel 52

Eine Lösung von 84,62 mg (0,3 mMol) t-Butoxycarbonyl-α-methyl-D,L-phenylalanin, 135 mg BOP, 118 mg (S)-α-Amino-N-[(1S,2S,4S)-1-(cyclohexylmethyl)-2-hydroxy-4-isopropyl-5-hexenyl]imidazol-4-propionamid und 0,062 ml Hünigbase wird 12 Stunden bei Raumtemperatur gerührt. Das Reaktionsgemisch wird danach wie in Beispiel 48 beschrieben aufgearbeitet, wobei man 125 mg (64 %) t-Butyl [(R oder S)-α-[[(S)-1-[[(1S,2S,4S)-1-(cyclohexylmethyl)-2-hydroxy-4-isopropyl-5-hexenyl]carbamoyl]-2-imidazol-4-yläthyl]-carbamoyl]-α-methylphenäthyl]carbamat als Epimerengemisch erhält. Durch Chromatographie an Kieselgel mit 10 % Methanol in Methylenchlorid lassen sich die beiden Epimeren auftrennen. Das polarere Epimere besitzt einen Rf-Wert von 0,38, während das weniger polare einen solchen Wert von 0,46 aufweist.
MS: 652 $(M+H)^+$

Das als Ausgangsmaterial verwendete t-Butoxycarbonyl-α-methyl-D,L-phenylalanin wurde wie folgt hergestellt:

Zu 2,0 g (11,16 mMol) α-Methyl-D,L-phenylalanin in 11,16 ml 1N Natronlauge und 10 ml t-Butylalkohol werden bei Raumtemperatur tropfenweise 2,44 g (11,16 mMol) Di-t-butyldicarbonat in 5 ml t-Butylalkohol gegeben, und anschliessend wird das Reaktionsgemisch über Nacht bei Raumtemperatur gerührt. Danach gibt man Wasser zu, wäscht mit Pentan, stellt die wässrige Phase durch Zugabe von Kaliumbisulfatlösung

auf pH 2 und extrahiert mit Essigester. Die organische Phase wird dann über Natriumsulfat getrocknet und eingeengt, wobei 1,56 g (50 %) t-Butoxycarbonyl-α-methyl-D,L-phenylalanin als weisses Pulver ausfällt, das direkt in die nächste Stufe eingesetzt wird.

## Beispiel 53

Man rührt ein Gemisch von 100 mg (0,45 mMol) N-Benzyl-3-carbamoyl-D-alanin, 187 mg (0,45 mMol) (S)-α-Amino-N-[(1S,2S,4S)-1-(cyclohexylmethyl)-2-hydroxy-4-isopropyl-5-hexenyl]imidazol-4-propionamid, 0,093 ml Hünigbase und 202 mg BOP in 10 ml eines 2:1-Gemisches von Acetonitril und Dimethylformamid während 18 Stunden bei Raumtemperatur. Danach giesst man das Reaktionsgemisch in wässrige Ammoniumchloridlösung, extrahiert mit Essigester, wäscht die Extrakte mit Natriumcar bonatlösung, trocknet diese über Natriumsulfat und dampft unter vermindertem Druck zur Trockene ein. Chromatographie des Rückstandes an Kieselgel mit 10 % Methanol in Methylenchlorid als Eluierungsmittel liefert 87 mg (32 %) (S)-α-[(R)-2-(Benzylamino)-3-carbamoylpropionamido]-N-[(1S,2S,4S)-1-(cyclohexylmethyl)-2-hydroxy-4-isopropyl-5-hexenyl]imidazol-4-propionamid als Harz.
MS: 595 $(M+H)^+$

Das als Ausgangsmaterial verwendete N-Benzyl-3-carbamoyl-D-alanin wurde wie folgt hergestellt:

1,5 g (10 mMol) D-Asparagin-hydrat in 5 ml 2N Natronlauge werden mit 1,01 ml (10 mMol) Benzaldehyd versetzt, und das Reaktionsgemisch 20 Minuten bei Raumtemperatur homogen gerührt. Anschliessend gibt man portionsweise 114 mg (3 mMol) Natriumborhydrid zu, rührt 30 Minuten bei Raumtemperatur, gibt wiederum 114 mg Natriumborhydrid zu und rührt schliesslich noch einmal 30 Minuten bei Raumtemperatur. Danach wird die wässrige Phase mit Methylenchlorid gewaschen und dann mit 1N Salzsäure auf pH 6-7 eingestellt, wobei Kristallisation einsetzt. Die ausgefallenen Kristalle werden abfiltriert, mit Wasser und Aether gewaschen und schliesslich im Hochvakuum getrocknet, wobei man 0,6 g (27 %) N-Benzyl-3-carbamoyl-D-alanin als weisses Pulver erhält.
MS: 223 $(M+H)^+$

## Beispiel 54

80 mg (0,13 mMol) Aethyl (R)-3-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-4-phenylbutyrat werden in 2 ml einer 5,6 molaren alkoholischen Dimethylaminlösung aufgenommen und 3 Stunden zum Rückfluss erhitzt. Dann wird die Lösung eingedampft, und der Rückstand mit einer 20:1-Mischung von Methylenchlorid und Methanol an Kieselgel chromatographiert, wobei man 33 mg (20 %) (S)-N-[(1S,2S,4S)-1-(Cyclohexylmethyl)-2-hydroxy-4-isopropyl-5-hexenyl]-α-[(R)-α-[(dimethylcarbamoyl)methyl]hydrocinnamamido]imidazol-4-propionamid als amorphes Pulver erhält.
MS: 608 $(M+H)^+$

## Beispiel 55

In analoger Weise wie in Beispiel 40 beschrieben wurden die folgenden Verbindungen hergestellt:

-Aus 117 mg (0,3 mMol) (S)-α-Amino-N-[(1S,2S,4S)-1-(cyclohexylmethyl)-2-hydroxy-4-isopropyl-5-hexenyl]-imidazol-4-propionamid und 75 mg (0,3 mMol) N-Benzyl-3-äthoxycarbonyl-D-alanin 85 mg (45 %) Aethyl (R)-3-(benzylamino)-3-[[(S)-1-[[(1S,2S,4S)-1(cyclohexylmethyl)-2-hydroxy-4-isopropyl-5-hexyl]carbamoyl]-2-imidazol-4-yläthyl]carbamoyl]propionat als amorphes Pulver, MS: 624 $(M+H)^+$;

- Aus 117 mg (0,3 mMol) (S)-α-Amino-N-[(1S,2S,4S)-1-(cyclohexylmethyl)-2-hydroxy-4-isopropyl-5-hexenyl]-imidazol-4-propionamid und 88 mg (0,3 mMol) N-Benzyloxycarbonyl-L-asparagin 136 mg (71 %) Benzyl [-(S)-1-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-2-carbamoyläthyl]carbamat als amorphes Pulver, MS: 639 $(M+H)^+$;

- Aus 157 mg (0,4 mMol) (S)-α-Amino-N-[(1S,2S,4S)-1-(cyclohexylmethyl)-2-hydroxy-4-isopropyl-5-hexenyl]-imidazol-4-propionamid und 136 mg (0,4 mMol) N-Benzoyl-γ-benzyl-D-glutamat 115 mg (40 %) Benzyl (S)-4-benzamido-4-[[(3)-1-[[(1S,2S,4S)-1-(cyclohexylmethyl)-2-hydroxy-4-isopropyl-5-hexenyl]carbamoyl]-2-imidazol-4-yläthyl]carbamoyl]butyrat, MS: 714 $(M+H)^+$;

- Aus 100 mg (S)-α-Amino-N-[(1S,2S,4S)-1-(cyclohexylmethyl)-2-hydroxy-4-isopropyl-5-hexenyl]imidazol-4-propionamid und 64 mg (RS)-α-[[(2-Hydroxyäthyl)carbamoyl]methyl]hydrozimtsäure 50 mg (S)-N-[-(1S,2S,4S)-1-(Cyclohexylmethyl)-2-hydroxy-4-isopropyl-5-hexenyl]-α-[(RS)-α-[[(2-(hydroxäthyl)carbamoyl]-methyl]hydrocinnamido]imidazol-4-propionamid als 1:1-Epimerengemisch, MS: 624 $(M+H)^+$;
- Aus 100 mg (S)-α-Amino-N-[(1S,2S,4S)-1-(cyclohexylmethyl)-2-hydroxy-4-isopropyl-5-hexenyl]imidazol-4-propionamid und 80 mg α-[(Phenäthylcarbamoyl)methyl]zimtsäure 65 mg (37 %) (S)-N-[(1S,2S,4S)-1-(Cyclohexylmethyl)-2-hydroxy-4-isopropyl-5-hexenyl]-α-[α-[(phenäthylcarbamoyl)methyl]cinnamamido]-imidazol-4-propionamid, MS: 682 $(M+H)^+$;
- Aus 214 mg (0,55 mMol) (S)-α-Amino-N-[(1S,2S,4S)-1-(cyclohexylmethyl)-2-hydroxy-4-isopropyl-5-hexenyl]imidazol-4-propionamid und 160 mg (0,55 mMol) (RS)-α-[2-(Morpholinocarbonyl)äthyl]-hydrozimtsäure 210 mg (60 %) (S)-N-[(1S,2S,4S)-1-(Cyclohexylmethyl)-2-hydroxy-4-isopropyl-5-hexenyl]-α-[(RS)-α-[2-(morpholinocarbonyl)äthyl]hydrocinnamyl]imidazol-4-propionamid als 1:1-Epimerengemisch, MS: 664 $(M+H)^+$;
- Aus 100 mg (0,25 mMol) (S)-α-Amino-N-[(1S,2S,4S)-1(cyclohexylmethyl)-2-hydroxy-4-isopropyl-5-hexenyl]imidazol-4-propionamid und 100 mg (0,28 mMol) N-[(4-Biphenylmethyl)carbamoyl]-3-phenyl-L-alanin 150 mg (77 %) 1-(4-Biphenylmethyl)-3-[(S)-1[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]harnstoff, MS: 748 $(M+H)^+$

Die jeweils als Ausgangsstoffe eingesetzten Säuren wurden wie folgt hergestellt:

N-Benzyl-3-äthoxycarbonyl-D-alanin

574 mg (2,9 mMol) β-Aethyl-D-asparaginsäure, welche nach der von Pivitti in Gazetta, 18, 480 (1888) für die Herstellung der racemischen Verbindung beschriebenen Methode hergestellt wurde, werden in 2,9 ml 2N Natronlauge gelöst und mit 0,3 ml Benzaldehyd versetzt und anschliessend eine Stunde bei Raumtemperatur kräftig gerührt. Dann gibt man 33 mg Natriumborhydrid zu und rührt eine weitere Stunde bei Raumtemperatur. Danach werden nochmals 33 mg Natriumborhydrid zugegeben und eine weitere Stunde bei Raumtemperatur gerührt. Anschliessend extrahiert man mit Aether und stellt danach die wässrige Phase auf pH 6-7 ein, wobei Kristallisation eintritt. Die ausgefallenen Kristalle werden abfiltriert und im Hochvakuum getrocknet, wobei man 81 mg (10 %) N-Benzyl-3-äthoxycarbonyl-D-alanin erhält, welches man direkt in die nächste Stufe einsetzt.
MS: 206 $(M-COOH)^+$

N-Benzoyl-γ-benzyl-D-glutamat

γ-Benzyl-D-glutamat wird nach der in Arch. Biochem. Biophys. 1952, 35, 176 von Miller und Waelsch beschriebenen Methode zur Benzoylierung von γ Aethyl-L-glutamat benzoyliert, wobei man das erwünschte Produkt in 75 %-iger Ausbeute erhält, MS: 341 $(M)^+$

(RS)-α-[[(2-(Hydroxyäthyl)carbamoyl]methyl]hydrozimtsäure

150 mg (0,8 mMol) Benzalbernsteinsäureanhydrid [S.G. Cohn et al., JACS, 90, 3495 (1968)] in 10 ml Methylenchlorid werden mit 0,048 ml Aethanolamin versetzt und 2 Stunden bei Raumtemperatur gerührt. Die ausgefallenen Kristalle werden abfiltriert und getrocknet, wobei man 155 mg (78 %) α-[[(2-(Hydroxäthyl)carbamoyl]methyl]zimtsäure als farblose Kristalle erhält, MS: 249 $(M)^+$

180 mg der oben genannten Säure in 5 ml Aethanol werden in Gegenwart von 20 ml Palladium auf Kohle 2 Stunden bei Raumtemperatur hydriert. Danach wird der Katalysator abfiltriert, und das Lösungsmittel unter vermindertem Druck entfernt. Der Rückstand wird in Essigester gelöst und mit 0,1N Salzsäure gewaschen. Die organische Phase wird über Natriumsulfat getrocknet und das Lösungsmittel unter vermindertem Druck abgedampft, wobei man 125 mg (70 %) (RS)-α-[[(2-Hydroxyäthyl)carbamoyl]methyl]-hydrozimtsäure erhält, welche direkt in die nächste Stufe eingesetzt wird.

α-[(Phenäthylcarbamoyl)methyl]zimtsäure

226 mg (1,2 mMol) Benzalbernsteinsäureanhydrid werden in 20 ml Methylenchlorid mit 0,15 ml 2-Phenäthylamin versetzt und anschliessend 2 Stunden bei Raumtemperatur gerührt. Das Lösungsmittel wird unter vermindertem Druck entfernt, und der Rückstand an Kieselgel chromatographiert, wobei man 260 mg (70 %) α-[(Phenäthylcarbamoyl)methyl]zimtsäure erhält, MS 309 $(M)^+$

(RS)-α-[2-(Morpholinocarbonyl)äthyl]hydrozimtsäure

2,5 g (10 mMol) Benzylmalonsäurediäthylester und 1,45 ml (10 mMol) Acrylsäure-t-butylester in 5 ml Acetonitril werden mit 1,49 ml DBU versetzt und 6 Stunden bei Raumtemperatur gerührt. Danach giesst man auf verdünnte Salzsäure (pH2), extrahiert mit Aether, wäscht die organische Phase mit wenig Wasser, trocknet über Natriumsulfat und entfernt das Lösungsmittel unter vermindertem Druck. Auf diese Weise erhält man 3,7 g (97 %) 4-t-Butyl 2,2-diäthyl 1-phenyl-2,2,4-butantricarboxylat als hellgelbes Oel, welches direkt in die nächste Stufe eingesetzt wird.

1,9 g (5,4 mMol) 4-t-Butyl 2,2-diäthyl 1-phenyl-2,2,4-butantricarboxylat in 20 ml Aethanol werden mit 27 ml 2N Natronlauge (54 mMol) versetzt und anschliessend 6 Stunden bei 50° gerührt. Danach extrahiert man das Reaktionsgemisch mit Aether, trocknet die Extrakte über Natriumsulfat und dampft das Lösungsmittel unter vermindertem Druck ab. Der Rückstand wird unter Verwendung eines 10:1-Gemisches von Methylenchlorid und Methanol als Eluierungsmittel chromatographiert, wobei man 0,8 g (50 %) 3-Aethyl 2,4-dihydrogen 1-phenyl-2,2,4-butantricarboxylat als Oel erhält, MS: 250 $(M-CO_2)^+$

800 mg 3-Aethyl 2,4-dihydrogen 1-phenyl-2,2,4-butantricarboxylat werden in Hochvakuum 2 Stunden auf 170° erhitzt und anschliessend mittels eines 10:1-Gemisches von Methylenchlorid und Methanol an Kieselgel chromatographiert, wobei man 600 mg (88 %) (RS)-4-(Aethoxycarbonyl)-5-phenylvaleriansäure als Oel erhält, welches direkt in die nächste Stufe eingesetzt wird, MS: 250 $(M^+)$

600 mg (2,4 mMol) (RS)-4-(Aethoxycarbonyl)-5-phenylvaleriansäure in 20 ml Acetonitril werden mit 1,06 g (2,4 mMol) BOP, 310 mg Hünigbase und 0,21 ml Morpholin versetzt und zunächst 1 Stunde bei Raumtemperatur und danach 2 Stunden bei 50° gerührt. Nach üblicher Aufarbeitung und Chromatographie an Kieselgel unter Verwendung eines 10:1-Gemisches von Methanol und Chloroform als Eluierungsmittel erhält man 400 mg (52 %) (RS)-α-[2-(Morpholinocarbonyl)äthyl]hydrozimtsäureäthylester, welcher direkt in die nächste Stufe eingesetzt wird, MS: 329 $(M^+)$

400 mg (1,25 mMol) des oben genannten Esters in 10 ml Aethanol werden mit 5 ml 0,5N Natronlauge versetzt und anschliessend 2 Stunden bei 50° gerührt. Nach üblicher Aufarbeitung und Chromatographie an Kieselgel unter Verwendung eines 10:1-Gemisches von Methylenchlorid und Methanol erhält man 170 mg (47 %) (RS)-α-[2-(Morpholinocarbonyl)äthyl]hydrozimtsäure als Oel, welches direkt in die nächste Stufe eingesetzt wird, MS 291 $(M^+)$

N-[(4-Biphenylmethyl)carbamoyl]-3-phenyl-L-alanin

3,9 g (21,3 mMol) Biphenylmethylamin (A. Kamil et al., Czech. 163, 819 (1976)) und 7,2 ml Hünigbase in 200 ml Toluol werden unter Kühlung in einem Eisbad mit 10, 5 ml Phosgen in Toluol (20 %-ig) versetzt und 1 Stunde bei 0° gerührt. Danach gibt man 9 g L-Phenylalaninbenzylester-4-toluolsulfonat zu und rührt die Lösung 12 Stunden bei 80°. Anschliessend verteilt man zwischen Methylenchlorid und Wasser, trocknet die organische Phase über Natriumsulfat, entfernt das Lösungsmittel unter vermindertem Druck und isoliert das Produkt durch Chromatographie an Kieselgel unter Verwendung eine 10:1-Gemisches von Methylenchlorid und Methanol, wobei man 7,3 g (74 %) N-[(4-Biphenylmethyl)carbamoyl]-3-phenyl-L-alaninbenzylester erhält.

3,2 g (6,7 mMol) des oben genannten Benzylesters in 100 ml Aethanol werden in Gegenwart von 320 mg Palladium auf Kohle bei Raumtemperatur bis zur Aufnahme der theoretischen Wasserstoffmenge hydriert. Danach wird der Katalysator abfiltriert, das Filtrat eingedampft und der Rückstand aus Aethanol/Methylenchlorid/Aether kristallisiert, wobei man 2.1 g (65 %) N-[(4-Biphenylmethyl)carbamoyl]-3-L-alanin in Form weisser Kristalle erhält, Schmelzpunkt 169-170°.

## Beispiel 56

57 mg N-[(1S,2S,4S)-1-(Cyclohexylmethyl)-2-hydroxy-4-isopropyl-5-hexenyl]-α-(3-phenyl-L-alanyl)-

imídazol-4-propionamid, 21 mg t-Butoxycarbonyl-L-azetidin-2-carbonsäure, welche durch t-Butoxycarbonylierung aus Azetidincarbonsäure nach der von O. Keller et al. in Org. Synth. 63, 160, 1985 beschriebenen Methode hergestellt wurde, 47 mg BOP und 0.021 ml Hünigbase in 5 ml Acetonitril werden 8 Stunden bei Raumtemperatur gerührt und danach in üblicher Weise aufgearbeitet. Das Rohprodukt wird durch Chromatographie an Kieselgel mit einem 10:1-Gemisch von Methanol und Chloroform gereinigt, wobei man 69 mg t-Butyl [(S)-2-[(S)-α-[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]phenäthyl]carbamoyl]-1-azetidincarboxylat in Form weisser Kristalle erhält. MS: 721 $(M+H)^+$.

## Beispiel 57

56 mg t-Butyl [(S)-2-[(S)-α-[(S)-1-[[(1S,2S,4S)-1-(Cyclohexylmethyl)-2-hydroxy-4-isopropyl-5-hexenyl]-carbamoyl]-2-imidazol-4-yläthyl]carbamoyl]phenäthyl]carbamoyl]-1-azetidincarboxylat in 5 ml Aethanol werden in Gegenwart von 10 mg Palladium auf Kohle 2 Stunden bei Raumtemperatur hydriert. Danach wird der Katalysator abfiltriert, und das Lösungsmittel unter vermindertem Druck abgedampft, wobei man 47 mg t-Butyl (S)-2-[[(S)-α-[[(S)-1-[[(1S,2S,4S)-1-(Cyclohexylmethyl)-2-hydroxy-4-isopropylhexyl]carbamoyl]-2-imidazol-4-yläthyl]carbamoyl]phenäthyl]carbamoyl]-1-azetidincarboxylat in Form weisser Kristalle erhält. MS: 723 $(M+H)^+$

In analoger Weise wurden durch Hydrierung von 90 mg (S)-N-[(1S,2S,4S)-1-(Cyclohexylmethyl)-2-hydroxy-4-isopropyl-5-hexenyl]-α-[(RS)-α-[(cyclopentylcarbonyl)methyl]hydrocinnamamido]imidazol-4-propionamid 78 mg (S)-N-[(1S,2S,4S)-1-(Cyclohexylmethyl)-2-hydroxy-4-isopropylhexyl]-α-[(RS)-α-[-(cyclopentylcarbonyl)methyl]hydrocinnamamido]imidazol-4-propionamid in Form eines amorphen Festkörpers als 1:1-Epimerengemisch erhalten, MS: 635 $(M+H)^+$

## Beispiel 58

In analoger Weise wie in Beispiel 56 beschrieben wurden die folgenden Verbindungen hergestellt:

- Aus 75 mg N-[(1S,2S,4S)-1-(Cyclohexylmethyl)-2-hydroxy-4-isopropyl-5-hexenyl]-α-(3-phenyl-L-alanyl)-imidazol-4-propionamid und 30 mg (3R,8aR)-Hexahydro-8a-methyl-5-oxo-5H-thiazolo[3,2-a]pyridin-3-carbonsäure (J.E. Baldwin et al., JACS 100, 4597), 64 mg (64 %) (S)-N-[(1S,2S,4S)-1-(Cyclohexylmethyl)-2-hydroxy-4-isopropyl-5-hexenyl]-α-[(RS)-α-[(3R,8aR)-hexahydro-8a-methyl-5-oxo-5H-thiazolo-[3,2-a]pyridin-3-carboxamido]hydrocinnamamido]-imidazol-4-propionamid als weisser Festkörper, MS: 735 $(M+H)^+$;
- Aus 75 mg N-[(1S,2S,4S)-1-(Cyclohexylmethyl)-2-hydroxy-4-isopropyl-5-hexenyl]-α-(3-phenyl-L-alanyl)-imidazol-4-propionamid und 30 mg (3R, 7aR)-Tetrahydro-7a-methylpyrrolo [2,1-b]thiazol-3-carbonsäure (J.E. Baldwin et al., JACS 100, 4597) 86 mg (3R,7aR)-N-[(S)-α-[[(S)-1-[[(1S,2S,4S)-1-(Cyclohexylmethyl)-2-hydroxy-4-isopropyl-5-hexenyl]carbamoyl]-2-imidazol-4-yläthyl]carbamoyl]phenäthyl]hexahydro-5-oxo-pyrrolo[2,1-b]thiazol-3-carboxamid als amorpher Festkörper, MS: 721 $(M+H)^+$;
- Aus 75 mg N-[(1S,2S,4S)-1-(Cyclohexylmethyl)-2-hydroxy-4-isopropyl-5-hexenyl]-α-(3-phenyl-L-alanyl)-imidazol-4-propionamid und 34 mg N-t-Butoxycarbonyl-L-methionin 90 mg t-Butyl [(S)-1-[[(S)-α-[[(S)-1-[[-(1S,2S,4S)-1-(Cyclohexylmethyl)-2-hydroxy-4-isopropyl-5-hexenyl]carbamoyl]-2-imidazol-4-yläthyl]-carbamoyl]phenäthyl]carbamoyl]-2-(methylthio)äthyl]carbamat als amorpher Festkörper, MS: 769 $(M+H)^+$;
- Aus 75 mg N-[(1S,2S,4S)-1-(Cyclohexylmethyl)-2-hydroxy-4-isopropyl-5-hexenyl]-α-(3-phenyl-L-alanyl)-imidazol-4-propionamid und 32 mg N-Isovaleryl-L-methionin, welches seinerseits durch Umsetzen von L-Methioninmethylester mit Isovaleriansäurechlorid und anschliessender Verseifung des Methylesters hergestellt wurde, 61 mg (S)-N-[(1S,2S,4S)-1-(Cyclohexylmethyl)-2-hydroxy-4-isopropyl-5-hexenyl]-α-[(S)-α-[(S)-4-(methylthio)-2-isovaleramidobutyramido]hydrocinnamamido]imidazol-4-propionamid als amorpher Festkörper MS: 753 $(M+H)^+$;

## Beispiel 59

Ein Gemisch von 95 mg (RS)-α-[(2-Morpholinoäthyl)carbamoylmethyl]-1-naphthalinpropionsäure, 100 mg (S)-α-Amino-N-[(1S,2S,4S)-1-(cyclohexylmethyl)-2-hydroxy-4-isopropyl-5-hexenyl]imidazol-4-propionamid und 97 mg HBTU in Acetonitril wird 3,5 Stunden bei Raumtemperatur gerührt und dann in üblicher Weise aufgearbeitet. Der Rückstand wird mit einem 4:1-Gemisch von Methylenchlorid und Methanol als Eluierungsmittel an Kieselgel chromatographiert, wobei man 60 mg N-[(1S,2S,4S)-1-(Cyclohexylmethyl)-2-hydroxy-4-isopropyl-5-hexenyl]-α-[3-[(2-morpholinoäthyl)carbamoyl]-2-(1-naphthylmethyl)propionamido]-imidazol-4-propionamid als Schaum erhält.

MS: 743 $(M+H)^+$

Die als Ausgangsmaterial eingesetzte (RS)-α-[(2-Morpholinoäthyl)carbamoylmethyl]-1-naphthalinpropionsäure wurde wie folgt hergestellt:

Man gibt zu 1,0 g (3,5 mMol) (RS)-3-(Aethoxycarbonyl)-4-(1-naphthyl)buttersäure (EPA 0.181.110) in 50 ml Methylenchlorid 0,72 g DCC und rührt die erhaltene Suspension 2 Stunden bei Raumtemperatur. Anschliessend gibt man 0,46 ml (3,5 mMol) 4-(2-Aminoäthyl)morpholin zu und rührt weitere 18 Stunden bei Raumtemperatur. Nach üblicher Aufarbeitung wird der Rückstand mit einem 20:1-Gemisch von Methylenchlorid und Methanol als Eluierungsmittel an Kieselgel Flash-chromatographiert, wobei man 0,5 g (RS)-α-[(2-Morpholinoäthyl)carbamoylmethyl]-1-naphthalinpropionsäureäthylester in Form eines hellgelben Harzes erhält.

MS: 398 $(M)^+$

0,5 g (1,25 mMol) des oben genannten Aethylesters in Aethanol wird mit 2,5 ml 1N Natronlauge versetzt und bei 80° 6 Stunden gerührt. Danach wird das Reaktionsgemisch auf pH 6 eingestellt, und das Lösungsmittel unter vermindertem Druck entfernt. Der Rückstand wird in einem 1:1-Gemisch von Methylenchlorid und Methanol aufgeschlämmt, die nicht löslichen Salze werden abfiltriert, das Filtrat über Natriumsulfat getrocknet, und das Lösungsmittel unter vermindertem Druck abgedampft. Man erhält auf diese Weise 500 mg (RS)-α-[(2-Morpholinoäthyl)carbamoylmethyl]-1-naphthalinpropionsäure in Form eines gelben Schaums, der direkt in die nächste Stufe eingesetzt wird.

## Beispiel 60

Ein Gemisch von 153 mg (0, 4mMol) (S)-α-Amino-N-[(1S,2S,4S)-1-benzyl -2-hydroxy-4-isopropyl-5-hexenyl]imidazol-4-propionamid 98 mg (R)-α-(Pivaloylmethyl)hydrozimtsäure, 175 mg BOP und 0,14 ml Hünigbase in 10 ml Acetonitril wird 12 Stunden bei Raumtemperatur gerührt und danach in üblicher Weise aufgearbeitet. Chromatographie des Rückstands an Kieselgel unter Verwendung eines 10:1-Gemisches von Methylenchlorid und Methanol als Eluierungsmittel liefert 120 mg (N-[(1S,2S,4S)-1-Benzyl-2-hydroxy-4-isopropyl-5-hexenyl]-α-(α-pivaloylhydrocinnamamido)imidazol-4-propionamid als weisse Kristalle, MS: 615 $(M+H)^+$.

Das als Ausgangsmaterial eingesetzte (S)-α-Amino-N-[(1S,2S,4S)-1-benzyl-2-hydroxy-4-isopropyl-5-hexenyl]imidazol-4-propionamid wurde wie folgt hergestellt:

In analoger Weise zur in Beispiel 1 beschriebenen Herstellung von (αS,βS)-β-t-Butoxycarbonylamino-α-[(S)-2-isopropyl-3-butenyl]cyclohexanpropanol wurden aus 56,6 g (320 mMol) (RS)-2-Isopropyl-3-butenylbromid 7,7 g Magnesium und 24, 2 g N-t-Butoxycarbonyl-L-phenylalanal (Evans et al., J. Org. Chem., 50, 4615 (1985) 15,6 g (49 %) t-Butyl [(1S,2S,4RS)-1-benzyl-2-hydroxy-4-isopropyl-5-hexenyl]carbamat als Epimeren-Gemisch erhalten. Das erwünschte (1S,2S,4S)-Epimere wurde durch Flash-Chromatographie an Kieselgel unter Verwendung eines 9:1-Gemisches von Methylenchlorid und Aether als Eluierungsmittel als weniger polares Isomeres isoliert, MS: 256 $(M\text{-Benzyl})^+$.

2,2 g t-Butyl [(1S,2S,4S)-1-benzyl-2-hydroxy-4-isopropyl-5-hexenyl]carbamat werden in 70 ml 1,8N Chlorwasserstoff in Dioxan 2 Stunden bei Raumtemperatur gerührt. Danach giesst man das Reaktionsgemisch in Natriumbicarbonatlösung und extrahiert bei pH 3 mit Aether. Trocknen und Eindampfen der Aetherextrakte und Chromatographie des Rückstands an Kieselgel mit einem 5:1-Gemisch von Methylenchlorid und Methanol liefert 900 mg (60 %) (2S,3S,5S)-2-Amino-5-isopropyl-1-phenyl-6-hepten-3-ol als Oel, MS: 248 $(M)^+$.

Ein Gemisch von 1,22 g (2,83 mMol) N-α-N-im-Di-t-butoxycarbonyl-L-histidin, 700 mg (2,83 mMol) (2S,3S,5S)-2-Amino-5-isopropyl-1-phenyl-6-hepten-3-ol, 1,25 g BOP und 0,58 ml Hünigbase in 20 ml Acetonitril wird 12 Stunden bei Raumtemperatur gerührt und danach in üblicher Weise aufgearbeitet. Chromatographie des Rückstands an Kieselgel unter Verwendung eines 2:1-Gemisches von Methylenchlorid und Aether liefert 1,0 g (10 %) t-Butyl 4-[(S)-2-[[(1S,2S,4S)-1-benzyl-2-hydroxy-4-isopropyl-5-hexenyl]-carbamoyl]-2-(1-t-butoxyformamido)äthyl]imidazol-1-carboxylat in Form eines Schaums, MS: 585 $(M+H)^+$.

1 g (1,7 mMol) t-Butyl 4-[(S)-2-[[(1S,2S,4S)-1-benzyl-2-hydroxy-4-isopropyl-5-hexenyl]carbamoyl]-2-(1-t-butoxyformamido)äthyl]imidazol-1-carboxylat werden in 7 ml 5N Chlorwasserstoff in Dioxan gelöst und

anschliessend 2 Stunden bei Raumtemperatur gerührt. Danach giesst man das Reaktionsgemisch in Natriumcarbonatlösung und extrahiert mit Essigester. Der organische Extrakt wird getrocknet und eingedampft, und der Rückstand mit einem 3:1-Gemisch von Methylenchlorid und Methanol an Kieselgel chromatographiert, wobei man 340 mg (52 %) (S)-α-Amino-N-[(1S,2S,4S)-1-benzyl-2-hydroxy-4-isopropyl-5-hexenyl]imidazol-4-propionamid als Schaum erhält, MS: 385 $(M+H)^+$.

Beispiel 61

120 mg N-[(1S,2S,4S)-1-Benzyl-2-hydroxy-4-isopropyl-5-hexenyl]-α-(α-pivaloylhydrocinnamamido)-imidazol-4-propionamid in 10 ml Aethanol werden in Gegenwart von 20 mg Palladium auf Kohle 2 Stunden bei Raumtemperatur hydriert. Danach wird der Katalysator abfiltriert, und das Lösungsmittel unter vermindertem Druck abgedampft, wobei man (S)-N-[(1S,2S,4S)-1-Benzyl-2-hydroxy-4-isopropylhexyl]-α-[(R)-α-(3,3-dimethyl-2-oxobutyl)hydrocinnamamido]imidazol-4-propionamid als Schaum erhält.
MS: 617 $(M+H)^+$

Beispiel 62

Ein Gemisch von 256 mg (1,03 mMol) (2S,3S,5S)-2-Amino-5-isopropyl-1-phenyl-6-hepten-3-ol, 414 mg Boc-Phe-His-OH, 0,354 ml Hünigbase und 455 mg BOP in 20 ml Acetonitril wird 12 Stunden bei Raumtemperatur gerührt und dann in üblicher Weise aufgearbeitet. Durch Flash-Chromatographie des Rückstands an Kieselgel unter Verwendung eines 7:1-Gemisches von Methylenchlorid und Methanol erhält man 385 mg (61 %) t-Butyl [(S)-α[[(RS)-1-[[(1S,2S,4S)-1-benzyl-2-hydroxy-4-isopropyl-5-hexenyl]-carbamoyl]-2-imidazol-4-yläthyl]carbamoyl]phenäthyl]carbamat als 4:1-Epimerengemisch.
MS: 632 $(M+H)^+$

Beispiel 63

334 mg (0,53 mMol) t-Butyl [(S)-α-[[(RS)-1-[[(1S,2S,4S)-1-benzyl-2-hydroxy-4-isopropyl-5-hexenyl]-carbamoyl]-2-imidazol-4-yläthyl]carbamoyl]phenäthyl]carbamat werden in 3 ml 5,2N Chlorwasserstoff in Dioxan gelöst und 2 Stunden bei Raumtemperatur gerührt. Nach der üblichen Aufarbeitung löst man 100 mg des erhaltenen Rohproduktes in 10 ml Acetonitril, gibt 40,4 mg (0,188 mMol) t-Butoxycarbonyl-D-prolin, 0,064 ml Hünigbase und 83 mg BOP zu und rührt das Reaktionsgemisch anschliessend 12 Stunden bei Raumtemperatur. Nach üblicher Aufarbeitung chromatographiert man den Rückstand an Kieselgel unter Verwendung eines 10:1-Gemisches von Methylenchlorid und Methanol, wobei man 78 mg (56 %) t-Butyl (R)-2-[[(S)-α-[(RS)-1-[[[(1S,2S,4S)-1-benzyl-2-hydroxy-4-isopropyl-5-hexenyl]carbamoyl]-2-imidazol-4-yläthyl]carbamoyl]phenäthyl]carbamoyl]-1-pyrolidincarboxylat in Form weisser Kristalle als 4:1-Epimerengemisch erhält.
MS: 729 $(M+H)^+$

Beispiel 64

0,65 g (S)-α-Amino-N-[(1S,2S,4RS)-2-hydroxy-1-isobutyl-4-isopropyl-5-hexenyl]imidazol-4-propionamid in 20 ml Acetonitril werden zusammen mit 0,36 ml Aethyldiisopropylamin zu einer Lösung von 0,45 g 2-Benzyl-3-phenylpropionsäure (welche nach der in J. Am. Chem. Soc. 71, 1863 (1949) beschriebenen Methode hergestellt wurde) und 0,8 g BOP in 30 ml Acetonitril gegeben und während 20 Stunden bei Raumtemperatur gerührt. Anschliessend wird das Reaktionsgemisch unter vermindertem Druck eingedampft, der Rückstand in 50 ml Essigester gelöst, und die organische Lösung zweimal mit je 100 ml Wasser gewaschen, über Natriumsulfat getrocknet und eingedampft. Der Rückstand wird durch Flash-Chromatographie an 100 g Kieselgel mit einem 250:15-Gemisch von Methylenchlorid und Methanol als Eluierungsmittel aufgetrennt, wobei man 0,17 g (S)-α-(2,2-Dibenzylacetamido)-N-[(1S,2S,4S)-2-hydroxy-1-isobutyl-4-isopropyl-5-hexenyl]imidazol-4-propionamid als weisses Pulver erhält, Schmelzpunkt 120°. Diese Verbindung entspricht dem weniger polaren Isomeren und besitzt einen Rf-Wert von 0,14.

Das als Ausgangsmaterial verwendete (S)-α-Amino-N-[(1S,2S,4RS)-2-hydroxy-1-isobutyl-4-isopropyl-5-hexenyl]imidazol-4-propionamid wurde wie folgt hergestellt:

0,72 g (4S)-Amino-(5S)-hydroxy-7-isopropyl-2-methyl-8-nonen in 20 ml Dimethylformamid werden zusammen mit 0,7 ml Aethyldiisopropylamin zu einer Lösung von 2 g N-α-N-im-Bis-Fmoc-L-histidin und 1,47 g BOP in 20 ml Dimethylformamid gegeben und 20 Stunden bei Raumtemperatur gerührt. Anschliessend wird das Reaktionsgemisch unter vermindertem Druck eingedampft, der Rückstand in 60 ml Essigester gelöst, und die organische Phase zweimal mit je 200 ml Wasser gewaschen, über Natriumsulfat getrocknet und eingedampft. Das Rohprodukt wird durch Flash-Chromatographie an 100 g Kieselgel mit einem 250:20-Gemisch von Methylenchlorid und Methanol als Eluierungsmittel gereingt, wobei man 1,25 g Fluoren-9-ylmethyl [(S)-1-[[(1S,2S,4RS)-2-hydroxy-1-isobutyl-4-isopropyl-5-hexenyl]carbamoyl]-2-imidazol-4-yläthyl]-carbamat als weisses Pulver erhält, MS: 573 $(M+H)^+$.

1,25 g Fluoren-9-ylmethyl [(S)-1-[[(1S,2S,4RS)-2-hydroxy-1-isobutyl-4-isopropyl-5-hexenyl]carbamoyl]-2-imidazol-4-yläthyl]carbamat werden in 20 ml Piperidin gelöst und 3 Stunden bei Raumtemperatur gerührt. Danach giesst man das Reaktionsgemisch auf 200 ml Eis/Wasser und filtriert. Eindampfen des Filtrates liefert 0,65 g (S)-α-Amino-N-[(1S,2S,4RS)-2-hydroxy-1-isobutyl-4-isopropyl-5-hexenyl]imidazol-4-propionamid als weisses Pulver, MS: 269 $(M-C_4H_5N_2)^+$.

## Beispiel 65

0,3 g (S)-2-[[N-(t-Butoxycarbonyl)-3-phenyl-L-alanyl]oxy]succinamidsäure werden zusammen mit 0,35 g BOP in 20 ml Acetonitril unter Zusatz von 2 ml Dimethylformamid gelöst, mit einer Lösung von 0,17 g (4S)-Amino-(5S)-hydroxy-7-isopropyl-2-methyl-8-nonen und 0,15 ml Aethyldiisopropylamin in 20 ml Acetonitril versetzt und 20 Stunden bei Raumtemperatur gerührt. Anschliessend wird das Reaktionsgemisch unter vermindertem Druck eingedampft, und der Rückstand in 50 ml Methylenchlorid gelöst. Die organische Lösung wird nacheinander mit 50 ml 3N Salzsäure, 50 ml gesättigter Natriumbicarbonatlösung und 50 ml gesättigter Kochsalzlösung gewaschen, über Natriumsulfat getrocknet und eingedampft. Der Rückstand wird durch Flash-Chromatographie an 50 g Kieselgel unter Verwendung eines 10:1-Gemisches von Methylenchlorid und Methanol als Eluierungsmittel gereinigt, und anschlies sen aus Aether/Methylenchlorid kristallisiert. Man erhält auf diese Weise 0,3 g t-Butyl [(S)-α[[(S)-2-carbamoyl-1-[[(1S,2S,4RS)-2-hydroxy-1-isobutyl-4-isopropyl-5-hexenylcarbamoyl]äthoxy]carbonyl]phenäthyl]carbamat als weisses Pulver, Schmelzpunkt 75°.

Die als Ausgangsmaterial verwendete (S)-2-[[N-(t-Butoxycarbonyl)-3-phenyl-L-alanyl]oxy]-succinamidsäure wurde wie folgt hergestellt:

0,8 g t-Butoxycarbonyl-L-phenylalanin werden in 5 ml Pyridin gelöst, bei -5° mit 0,3 ml Oxalylchlorid versetzt und 10 Minuten bei 0-5° gerührt. Nach Zugabe von 0,7 g Benzyl-(S)-3-carbamoyl-2-hydroxypropionsäure (welche nach den Vorschriften in Agr. Biol. Chem. 40, 1651 (1976) und Int. J. Peptide Protein Res. 20, 35 (1982) hergestellt wurde) in 1 ml Pyridin wird zunächst 2 Stunden bei 0-5° und danach 1 Stunde bei Raumtemperatur gerührt. Das Reaktionsgemisch wird dann auf Eis gegossen und zweimal mit je 50 ml Essigester extrahiert. Die vereinigten organischen Extrakte werden mit 50 ml 3N Salzsäure und 50 ml gesättigter Natriumbicarbonatlösung gewaschen, über Natriumsulfat getrocknet und eingedampft. Der Rückstand wird durch Flash-Chromatographie an 50 g Kieselgel unter Verwendung eines 10:1-Gemisches von Methylenchlorid und Methanol als Eluierungsmittel gereinigt, wobei man 0,9 g (S)-2-[[N-(t-Butoxycarbonyl)-3-phenyl-L-alanyl]oxy]succinamidbenzylester als gelbes Pulver erhält, MS: 397 $(M-C_4H_9O)^+$.

0,9 g des obigen Benzylesters werden in 40 ml Aethanol gelöst und in Gegenwart von 0,1 g Palladium auf Kohle (10 %ig) bei Normaldruck hydriert. Nach beendeter Wasserstoffaufnahme wird der Katalysator abfiltriert, und das Filtrat eingedampft. Kristallisation des Rückstands aus Methylenchlorid liefert 0,52 g (S)-2-[[N-(t-Butoxycarbonyl)-3-phenyl-L-alanyl]oxy]succinamidsäure als weisses Pulver, MS: 381 $(M+H)^+$.

## Beispiel 66

Eine Lösung von 200 mg (2S,3S,5S)-2-[Boc-His(3-Bom)-Pro-His-NH]-1-cyclohexyl-5-isopropyl-6-hepten-3-ol wird in einem 4:1-Gemisch von Eisessig und Wasser in Gegenwart von 20 mg Palladium auf Kohle (10

%ig) bei Raumtemperatur erschöpfend hydriert. Nach beendeter Wasserstoffaufnahme (4 Stunden) wird der Katalysator abfiltriert, und das Filtrat zur Trockene eingedampft. Die nach der chromatographischen Reinigung an Kieselgel erhaltenen Kristalle werden aus Essigester/Hexan umkristallisiert, wobei man (2S,3S,5S)-2-(Boc-His-Pro-His-NH)-1-cyclohexyl-5-isopropyl-3-heptanol in 69 %iger Ausbeute erhält, Schmelzpunkt 135-136°.

Die Herstellung des als Ausgangsmaterial eingesetzten (2S,3S,5S)-2-[Boc-His(3-Bom)Pro-His-NH]-1-cyclohexyl-5-isopropyl-6-hepten-3-ols wird in Beispiel 68 beschrieben.

## Beispiel 67

Eine Lösung von 100 mg (S)-α-[[N-(t-Butoxycarbonyl)-β-phenyl-L-alanyl]oxy]imidazol-4-propionsäure, 78 mg (αS,βS)-β-Amino-α-[(S)-2-isopropyl-3-butenyl]cyclohexanpropanol und 55 mg N-Methylmorpholin in 3 ml Dimethylformamid wird unter Rühren und Begasung mit Argon mit 102 mg HBTU versetzt. Nach Stehenlassen über Nacht giesst man die Reaktionslösung in 5 ml konzentrierte wässrige Natriumbicarbonatlösung und extrahiert mit Essigester. Die organischen Extrakte werden getrocknet und unter vermindertem Druck eingedampft, und das erhaltene Rohprodukt durch Chromatographie an Kieselgel mit einem 95:5-Gemisch von Methylenchlorid und Methanol als Eluierungsmittel gereinigt, wobei man t-Butyl [(S)-α-[[(S)-1-[[(1S,2S,4S)-1-(cyclohexylmethyl)-2-hydroxy-4-isopropyl-5-hexenyl]carbamoyl]-2-imidazol-4-yläthoxy]-carbonyl]phenäthyl]carbamat in 22 %iger Ausbeute erhält, Schmelzpunkt 72-75° (Zers.).

Die als Ausgangsmaterial eingesetzte (S)-α-[[N-(t-Butoxycarbonyl)-3-phenyl-L-alanyl]oxy]imidazol-4-propionsäure wurde wie folgt hergestellt:

1,99 g N-t-Butoxycarbonyl-L-phenylalanin und 1,58 g Benzyl (S)-α-Hydroxyimidazolpropionat werden in 25 ml Methylenchlorid suspendiert und gerührt. Nach 15minütigem Rühren bei Raumtemperatur gibt man 305 mg 4-Dimethylaminopyridin zu und kühlt das Reaktionsgemisch auf -15°. Nach tropfenweiser Zugabe von 2 ml N-Methylmorpholin und einer Lösung von 6,6 g Propanphosphorsäureanhydrid in 5 ml Methylenchlorid wird das Reaktionsgemisch noch 2 Stunden bei -15° weitergerührt und danach 6 Tage bei -18° stehengelassen. Nach Zugabe von einigen Tropfen Wasser wird das Reaktionsgemisch unter vermindertem Druck eingedampft, und der ölige Rückstand 2 Stunden mit 100 ml Essigester und 25 ml Wasser verrührt. Die organische Phase wird dann nacheinander mit wässriger Natriumbicarbonatlösung (5 %ig) Wasser und 1,5M Zitronensäure gewaschen. Das nach dem Abdampfen des Lösungsmittels unter vermindertem Druck erhaltene farblose Oel wird an Kieselgel mit einem 95:5-Gemisch von Methylenchlorid und Methanol als Eluierungsmittel chromatographiert, wobei man (S)-α-[[N-(t-Butoxycarbonyl)-3-phenyl-L-alanyl]oxy]imidazol-4-propionsäurebenzylester als gelbes Oel in 13 %iger Ausbeute erhält, MS: 494 $(M+H)^+$.

Eine Lösung von 960 mg des oben beschriebenen Benzylester in 20 ml absolutem Aethanol wird in Gegenwart von 500 mg Palladium auf Kohle (10 %ig) bis zur Aufnahme der theoretischen Menge Wasserstoff hydriert. Danach wird der Katalysator abfiltriert, und das Filtrat unter vermindertem Druck eingedampft, wobei man einen öligen Rückstand erhält, der nach Zugabe von Methanol/Essigester kristallisiert. Auf diese Weise erhält man (S)-α-[[N-(t-Butoxycarbonyl)-3-phenyl-L-alanyl]oxy]imidazol-4-propionsäure in 18 %iger Ausbeute, Schmelzpunkt 130-131°.

## Beispiel 68

In analoger Weise wie in Beispiel 67 beschrieben wurden die folgenden Verbindungen hergestellt:

- Aus (S)-α-Amino-N-[(1S,2S,4S)-1(cyclohexylmethyl)-2-hydroxy-4-isopropyl-5-hexenyl]imidazol-4-propionamid und N-(t-Butoxycarbonyl)-3-(1-naphthyl)-L-alanin in 50 %iger Ausbeute das t-Butyl [(S)-1-[[(S)-1-[[(1S,2S,4S)-1-(cyclohexylmethyl)-2-hydroxy-4-isopropyl-5-hexenyl]carbamoyl]-2-imidazol-4-yläthyl]-carbamoyl]-2-(1-naphthyl)äthyl]carbamat, Schmelzpunkt 141-143° (aus Essigester/Hexan);
- Aus (S)-α-Amino-N-[(1S,2S,4S)-1(cyclohexylmethyl)-2-hydroxy-4-isopropyl-5-hexenyl]imidazol-4-propionamid und N-(t-Butoxycarbonyl)-4-chlorphenylalanin in 41 %iger Ausbeute das t-Butyl [(S)-1-[[(S)-1-[[(1S,2S,4S)-1-(cyclohexylmethyl)-2-hydroxy-4-isopropyl-5-hexenyl]carbamoyl]-2-imidazol-4-yläthyl]-carbamoyl]-2-(4-chlorphenyl)äthyl]carbamat, Schmelzpunkt 220-221° (aus Aethanol/Essigester);
- Aus (S)-α-Amino-N-[(1S,2S,4S)-1-(cyclohexylmethyl)-2-hydroxy-4-isopropyl-5-hexenyl]imidazol-4-propionamid und N-t-Butoxycarbonyl-L-phenylglycin in 39 %iger Ausbeute das t-Butyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]benzyl]-carbamat, Schmelzpunkt 125-126° (aus Essigester/Hexan);
- Aus (S)-α-Amino-N-[(1S,2S,4S)-1-(cyclohexylmethyl)-2-hydroxy-4-isopropyl-5-hexenyl]imidazol-4-propionamid und N-t-Butoxycarbonyl-L-cyclohexylglycin in 54 %iger Ausbeute das t-Butyl [(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]-cyclohexylmethyl]carbamat, Schmelzpunkt 139-140° (aus Essigester/Hexan);
- Aus (S)-α-Amino-N-[(1S,2S,4S)-1-(cyclohexylmethyl)-2-hydroxy-4-isopropyl-5-hexenyl]imidazol-4-propionamid und t-Butoxycarbonyl-D-phenylalanin in 48 %iger Ausbeute das t-Butyl [(R)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]-carbamat, Schmelzpunkt 195° (aus Essigester);
- Aus (S)-α-Amino-N-[(1S,2S,4S)-1-(cyclohexylmethyl)-2-hydroxy-4-isopropyl-5-hexenyl]imidazol-4-propionamid und t-Butoxycarbonyl-L-cyclohexylalanin in 22 %iger Ausbeute das t-Butyl [(S)-2-cyclohexyl-1-[[(S)-1-[[(1S,2S,4S)-1-(cyclohexylmethyl)-2-hydroxy-4-isopropyl-5-hexenyl]carbamoyl]-(2-imidazol-4-yläthyl]-carbamoyl]äthyl]carbamat, Schmelzpunkt 116° (aus Essigester/Diisopropyläther);
- Aus N-[(1S,2S,4S)-1-(Cyclohexylmethyl)-2-hydroxy-4-isopropyl-5-hexenyl]-α-(3-phenyl-L-alanyl)imidazol-4-propionamid und N-[5-(1-t-Butoxyformamido)valeryl]-β-alanin in 40 %iger Ausbeute das t-Butyl [4-[[-2-[[(S)-α-[[(S)-1-[[(1S,2S,4S)-1-(cyclohexylmethyl)-2-hydroxy-4-isopropyl-5-hexenyl]carbamoyl]-2-imidazol-4-yläthyl]carbamoyl]phenäthyl]carbamoyl]äthyl]carbamoyl]butyl]carbamat, Schmelzpunkt 114-115° (aus Essigester);
- Aus (S)-α-Amino-N-[(1S,2S,4S)-1-(cyclohexylmethyl)-2-hydroxy-4-isopropyl-5-hexenyl]imidazol-4-propionamid und Boc-His(3-Bom)-Pro-OH in 51 %iger Ausbeute das (2S,3S,5S)-2-[Boc-His(3-Bom)-Pro-His-NH]-1-cyclohexyl-5-isopropyl-6-hepten-3-ol, Schmelzpunkt 121-122° (aus Essigester/ Hexan).

Die als Ausgangsstoffe eingesetzten Säuren sind entweder bekannt und käuflich oder wurden wie folgt hergestellt:

N-[5-(1-t-Butoxyformamido)valeryl]-β-alanin

Zu einer Suspension von 7,68 g β-Alaninäthylester-hydrochlorid in 50 ml Methylenchlorid werden unter Rühren in der angegebenen Reihenfolge 5,56 g Triäthylamin, 10,86 g N-t-Butoxycarbonyl-5-aminovaleriansäure, gelöst in 50 ml Methylenchlorid, und portionsweise 11,35 g DCC gegeben. Nach beendeter Zugabe wird das Reaktionsgemisch bei Raumtemperatur über Nacht gerührt und danach vom ausgefallenen Harnstoff abfiltriert. Das Filtrat wird nacheinander mit 250 ml Natriumbicarbonatlösung (5 %ig) Wasser, 250 ml 0,1M Zitronensäure und schliesslich mit gesättigter Kochsalzlösung gewaschen. Danach wird das Lösungsmittel unter vermindertem Druck abgedampft, und der ölige Rückstand mittels Säulenchromatographie an Kieselgel gereinigt, wobei man N-[5-(1-t-Butoxyformamido)valeryl]-β-alaninäthylester in 59 %iger Ausbeute erhält, Schmelzpunkt 28° (aus Hexan).

Man löst 3,94 g des oben genannten Esters in 20 ml Methanol und gibt unter Rühren bei 0° zunächst 1 Moläquivalent und nach 30 Minuten weitere 0,2 Moläquivalente 1N Natronlauge zu. Dann rührt man eine Stunde bei Raumtemperatur, gibt tropfenweise 1,2 Moläquivalente 1N Salzsäure (pH 4) zu, dampft das Lösungsmittel unter vermindertem Druck ab und extrahiert die wässrige Phase mehrmals mit Methylenchlorid. Trocknen und Eindampfen der Extrakte liefert einen öligen Rückstand, der durch Zugabe von Hexan kristallisiert. Umkristallisation aus Hexan liefert N-[5-(1-t-Butoxyformamido)valeryl)-β-alanin in 87 %iger Ausbeute, Schmelzpunkt 70°.

## Beispiel 69

Zu 1,06 g (3 mMol) t-Butyl [(1S,2S,4S)-4-(2-furyl)-2-hydroxy-1-isobutyl-5-methylhexyl]carbamat in 8 ml Essigsäure werden 5 ml einer 4-molaren Lösung von Chlorwasser stoff in Dioxan gegeben. Nach 30 Minuten wird das Lösungsmittel unter reduziertem Druck abgedampft, der Rückstand mit Toluol digeriert, und das Lösungsmittel wiederum abgedampft. Der so erhaltene Rückstand wird in 10 ml absolutem Dimethylformamid gelöst, und zur Lösung 1,8 g (3 mMol) N-α-N-im-Bis-Fmoc-L-histidin in 10 ml Tetrahydrofuran, 0,35 ml N-Methylmorpholin und 0,8 g (5,6 mMol) N-Hydrobenzotriazol gegeben. Diese Lösung wird auf -10° abgekühlt und tropfenweise mit 5 ml einer 1-molaren DCC-Lösung in Tetrahydrofuran versetzt. Das Reaktionsgemisch wird über Nacht gerührt und danach abfiltriert. Die Lösung wird mit 5 ml einer 50 %igen Piperidin-Dimethylformamid-Lösung versetzt und nach 30 Minuten im Hochvakuum eingedampft. Der Rückstand wird in Aether gelöst, und die organische Phase nacheinander mit Wasser, einer

Natriumcarbonatlösung und nochmals mit Wasser gewaschen. Danach wird die organische Phase über Natriumsulfat getrocknet und unter vermindertem Druck eingedampft. Dann löst man den Rückstand in 10 ml Dimethylformamid und gibt 0,8 g (3 mMol) N-t-Butoxycarbonyl-phenylalanin in 10 ml Tetrahydrofuran, 0,35 ml N-Methylmorpholin, 0,8 g (5,6 mMol) N-Hydroxybenzotriazol und 5 ml einer 1-molaren DCC-Lösung in Tetrahydrofuran zu. Das Reaktionsgemisch wird über Nacht gerührt und danach abfiltriert. Das Filtrat wird im Hochvakuum eingedampft, und der Rückstand in Aether gelöst. Die organische Lösung wird mit Wasser, einer Natriumcarbonatlösung und nochmals mit Wasser gewaschen, über Natriumsulfat getrocknet und unter vermindertem Druck eingedampft. Der Rückstand wird zur Reinigung mit einem 19:3-Gemisch von Methylenchlorid und Essigester als Eluierungsmittel chromatographiert, wobei man 0,65 g (S,S,S)-1-(Boc-Phe-His-NH)methylbutyl-γ-isopropyl-2-furanpropanol als dickes Oel erhält.

MS: 638 $(M+H)^+$.

Das als Ausgangsmaterial eingesetzte t-Butyl [(1S,2S,4S)-4-(2-furyl)-2-hydroxy-1-isobutyl-5-methylhexyl]carbamat wurde wie folgt hergestellt:

Zu einer Lösung von 217 g (1,67 Mol) 2,5-Dimethoxy-2,5-dihydrofuran, 1500 ml (10 Mol) Malonsäurediäthylester, 167 ml Wasser und 330 ml Eisessig werden auf einmal 340 g (2,5 Mol) Zinkchlorid gegeben, wobei die Temperatur auf 35° ansteigt. Die dunkle Lösung wird über Nacht bei Raumtemperatur gerührt, danach auf Eis gegossen, dreimal mit Diäthyläther extrahiert, nacheinander mit gesättigten Natriumbicarbonatund Kochsalzlösungen gewaschen und schliesslich getrocknet. Nach dem Eindampfen des überschüssigen Malonsäurediäthylesters (70°/13Pa) wird das verbleibende Oel über eine Vigreux-Kolonne destilliert, wobei man 109,2 g (29 %) 2-Furylmalonsäurediäthylester (Reinheit 94 %) erhält, Siedepunkt 100-110°/39Pa.

24 g (1 Mol) Natriumhydrid werden in 100 ml absolutem Tetrahydrofuran suspendiert. Während 30 Minuten wird eine Lösung von 218,4 g (0,97 Mol) 2-Furylmalonsäurediäthylester in 200 ml absolutem Tetrahydrofuran zugetropft, wobei die Temperatur auf 30° ansteigt (blaue Suspension). Nach 30-minütigem Rühren bei 30° werden 340 g (200 ml; 2 Mol) Isopropyljodid innerhalb von 30 Minuten zugetropft. Das Reaktionsgemisch wird über Nacht zum Rückfluss erhitzt, danach unter vermindertem Druck eingedampft und in Aether gelöst. Nach dem Eindampfen des Lösungsmittels wird der verbleibende ölige Rückstand im Hochvakuum destilliert, wobei man 240,6 g (92,5 %) 2-Furyl-2-isopropylmalonsäurediäthylester (Reinheit 93,6 %) in Form eines gelblichen Oels erhält, Siedepunkt 85-88°/13Pa.

240,6 g (0,9 Mol) 2-Furyl-2-isopropylmalonsäurediäthylester werden in 500 ml Dimethylsulfoxyd gelöst, mit 2000 ml 2N Natronlauge versetzt und 2 Stunden zum Rückfluss erhitzt. Nach dem Abkühlen wird das Reaktionsgemisch in verdünnte Schwefelsäure gegossen und mit Aether erschöpfend extrahiert. Die organischen Extrakte werden danach getrocknet, und das Lösungsmittel unter vermindertem Druck eingedampft. Auf diese Weise erhält man 148,7 g (98 %) (±) 2-(2-Furyl)-3-methylbuttersäure in Form eines dicken gelblichen Oels.

161,0 g (0,96 Mol) (±) 2-(2-Furyl)-3-methylbuttersäure werden in 1000 ml Aether gelöst und mit 109 g (0,9 Mol) S(-)-α-Phenyläthylamin in 100 ml Aether versetzt. Die ausgefallenen Kristalle werden abfiltriert und dreimal aus 600 ml Essigester umkristallisiert. Auf diese Weise erhält man 80 g weisse Kristalle, Schmelzpunkt 135-136°, $[\alpha]_D^{20}$ = -14,1° (c = 2 %, Methanol).

Diese Kristalle werden in Essigester suspendiert und unter Eiskühlung mit einer 3N Salzsäurelösung angesäuert. Die organische Lösung wird mit Wasser gewaschen, getrocknet und unter vermindertem Druck eingedampft, wobei man 46, 7 g (+) 2-(2-Furyl)-3-methylbuttersäure als dickflüssiges Oel erhält (Reinheit 98,6 %), $[\alpha]_D^{20}$ = +29,9° (c = 1 %, Essigester).

Die Mutterlaugen aus den obigen Kristallisationen werden zusammengenommen, und die freie Säure wie beschrieben isoliert, in 1000 ml Aether gelöst und mit der entsprechenden Menge R-(+)-α-Phenyläthylamin versetzt. Nach dreimaligem Umkristallisieren aus Essigester erhält man 80,3 g weisse Kristalle vom Schmelzpunkt 135-136°, $[\alpha]_D^{20}$ = +15,0° (c = 1 %, Methanol).

Ueberführen dieses Salzes nach dem oben beschriebenen Verfahren liefert 45,0 g (-) 2-(2-Furyl)-3-methylbuttersäure als dickflüssiges Oel von 99,5 %iger Reinheit, $[\alpha]_D^{20}$ = -31,1° (c = 1 %, Essigester).

10,5 g (0,277 Mol) Lithiumaluminiumhydrid werden in 100 ml absolutem Aether suspendiert und unter Rühren innerhalb von 10 Minuten mit 100 ml absolutem Tetrahydrofuran versetzt. Zu dieser gerührten Suspension werden unter Erhitzen zum Rückfluss 46, 7 g (0,277 Mol) (+) 2-(2-Furyl)-3-methylbuttersäure in 100 ml absolutem Tetrahydrofuran innerhalb von 6-8 Stunden getropft. Das Reaktionsgemisch wird danach abgekühlt und mit einer 3N Salzsäurelösung langsam versetzt. Die organische Phase wird abgetrennt, mit Wasser gewaschen, getrocknet und unter vermindertem Druck eingedampft. Das verbleibende Oel wird im Hochvakuum destilliert, wobei man 32 g (75 %) (R)-(+)-2-(2-Furyl)-3-methyl-1-butanol als farblose Flüssigkeit von 96 %iger Reinheit erhält, Siedepunkt 58°/39Pa, $[\alpha]_D^{20}$ = +2,5° (c = 1 %, Aethanol).

Zu einer Lösung aus 17,8 g (0,1154 Mol) (R)-(+)-2-(2-Furyl)-3-methyl-1-butanol in 150 ml Tetrachlorkohlenstoff werden portionenweise 121,7 g (0,464 Mol) Triphenylphosphin innerhalb von 8 Stunden gegeben. Nach 24-stündigem Rühren des Reaktionsgemisches gibt man 500 ml Pentan zu und filtriert den gebildeten Niederschlag ab. Nach dem Eindampfen des Lösungsmittels wird der Rückstand in Pentan gelöst und durch Chromatographie an Kieselgel gereinigt, wobei man 16, 5 g (92 %) 2-[(S)-(-)-1-(Chlormethyl)-2-methylpropylfuran erhält, welches direkt in die nächste Stufe eingesetzt wird.

Eine Lösung von 14,6 g (0,084 Mol) 2-[(S)-(-)-1-(Chlormethyl)-2-methylpropylfuran in 46 ml Aether werden langsam zu einer Suspension von 3,22 g Magnesium in 12 ml Aether getropft. Das Reaktionsgemisch wird über Nacht bei Raumtemperatur kräftig gerührt und danach auf -70° abgekühlt. Dazu werden 8,3 g (0,038 Mol) t-Butoxycarbonyl-L-leucinal in 40 ml Aether getropft. Nach beendeter Zugabe wird das Reaktionsgemisch noch 1 Stunde bei 0° stehengelassen. Danach gibt man 100 ml einer gesättigten Ammoniumchloridlösung zu, trocknet die organische Phase über Magnesiumsulfat und dampft das Lösungsmittel unter vermindertem Druck ab. Der erhaltene Rückstand (16 g) wird mit einem 1:4-Gemisch von Aether und Hexan an Kieselgel chromatographiert, wobei man 4,8 g t-Butyl [(1S,2S,4S)-4-(2-furyl)-2-hydroxy-1-isobutyl-5-methylhexyl]carbamat erhält, welches direkt in die nächste Stufe eingesetzt wird.

## Beispiel 70

In analoger Weise wie in Beispiel 69 beschrieben wurde aus t-Butyl [(1S,2S,4S)-2-hydroxy-1-isobutyl-5-methyl-4-phenylhexyl]carbamat das [4S,5S,7S]-4-(Boc-Phe-His-NH)-2,8-dimethyl-7-phenyl-5-nonanol in Form eines dicken Oels erhalten.

MS: 648 $(M+H)^+$.

Das als Ausgangsmaterial verwendete t-Butyl [(1S,2S,4S)-2-hydroxy-1-isobutyl-5-methyl-4-phenylhexyl]-carbamat wurde wie folgt hergestellt:

Zu 1500 ml flüssigem Ammoniak werden bei -35° innerhalb von 90 Minuten 23 g Natrium in kleinen Stücken gegeben. Nach beendeter Zugabe werden 150 mg Eisen-(III)-nitrat.$9H_2O$ zugegeben. Zu dieser Suspension werden unter starkem Rühren 150 g (1Mol) Phenylessigsäuremethylester in 500 ml absolutem Tetrahydrofuran und eine halbe Stunde später 100 ml (1 Mol) Isopropyljodid getropft. Nach beendeter Zugabe wird über Nacht der Ammoniak abdestilliert. Zum flüssigen Rückstand wird langsam Wasser gegeben und mit Aether extrahiert. Die organischen Extrakte werden mit Wasser gewaschen, über Natriumsulfat getrocknet und unter vermindertem Druck eingedampft, wobei man 165,8 g (87 %) 2-Phenyl-3-methylbuttersäuremethylester als gelbes Oel erhält, welches direkt in die nächste Stufe eingesetzt wird.

Eine Lösung von 165,8 g (0,87 Mol) 2-Phenyl-3-methylbuttersäuremethylester, 52,0 g (1,30 Mol) Natriumhydroxyd, 1200 ml Methanol und 600 ml Wasser wird während einer Stunde zum Rückfluss erhitzt. Nach dem Abdampfen des Methanols unter vermindertem Druck, Verdünnen mit Wasser und Ansäuern mit konz. Salzsäure wird die wässrige Lösung mit Aether extrahiert. Die ätherischen Extrakte werden mit Wasser gewaschen, über Natriumsulfat getrocknet und unter vermindertem Druck eingedampft, wobei man 146,3 g (95 %) 2-Phenyl-3-methylbuttersäure in Form eines gelben Oels erhält, welches direkt in die nächste Stufe eingesetzt wird.

Eine Lösung von 146,3 g (0,82 Mol) rac. 2-Phenyl-3-methylbuttersäure in 820 ml Acetonitril wird mit 78,5 ml (0,615 Mol) (S)-(-)-α-Phenyläthylamin versetzt. Die dabei gebildeten Kristalle werden abfiltriert und mit Acetonitril gewaschen. Nach dreimaligem Umkristallisieren aus Acetonitril erhält man 88,5 g (+)-2-Phenyl-3-methylbuttersäure als α-Phenyläthylammoniumsalz, Schmelzpunkt 198-200°, $[\alpha]_D^{20}$ = +4,3° (c = 1 %, Methanol). Dieses Salz wird in 1000 ml Essigester suspendiert und mit 3N Salzsäure unter Eiskühlung versetzt. Die Essigesterextrakte werden mit Wasser gewaschen, über Natriumsulfat getrocknet und unter vermindertem Druck eingedampft, wobei man 54,5 g (S)-(+)-2-Phenyl-3-methylbuttersäure als leicht gelbliches Oel erhält, $[\alpha]_D^{20}$ = +61,3° (c = 1 %, Chloroform).

Eine Suspension von 19,5, g (0,51 Mol) Lithiumaluminiumhydrid in 750 ml absolutem Aether wird zum Rückfluss erhitzt. Innerhalb von 4 Stunden wird dazu eine Lösung von 54,5 g (0,307 Mol) (S)-(+)-2-Phenyl-3-methylbuttersäure in 250 ml absolutem Aether getropft. Nach weiterem 2-stündigem Erhitzen zum Rückfluss wird die Suspension sorgfältig mit 1N Salzsäure versetzt, und die Aetherphase mit Wasser, verdünnter Natriumbicarbonatlösung und nochmals mit Wasser gewa schen. Nach dem Trocknen der organischen Phase über Natriumsulfat und Eindampfen unter vermindertem Druck erhält man 53,1 g (S)-(+)-2-Phenyl-3-methylbutanol als dickes Oel, welches direkt in die nächste Stufe eingesetzt wird.

Eine Lösung von 49,3 g (0,3 Mol) (S)-(+)-2-Phenyl-3-methylbutanol in 400 ml absolutem Methylenchlorid wird mit 80 g (0,3 Mol) Triphenylphosphin versetzt. Danach werden unter kräftigem Rühren 53,4 g (0,3 Mol) N-Bromsuccinimid portionsweise zugegeben. Nach 16 Stunden wird das Reaktionsgemisch abfiltriert,

und das Filtrat unter vermindertem Druck eingedampft. Der Rückstand wird in Hexan gelöst und durch Chromatographie an Kieselgel gereinigt, wobei man auf diese Weise 34,9 g (51 %) (S)-(-)-2-Phenyl-3-methylbrombutan als flüchtiges Oel erhält, $[\alpha]_D^{20}$ = -19,6° (c = 1 %, Chloroform).

Eine Lösung von 40,9 g (0,18 Mol) (S)-(-)-2-Phenyl-3-methylbrombutan in 110 ml Tetrahydrofuran und 0,5 ml 1,2-Dibromäthan wird langsam zu einer Suspension von 6,6 g Magnesium in 25 ml Aether getropft, wobei die Temperatur bis auf 50° steigt. Das Reaktionsgemisch wird danach noch 2 Stunden bei 50° weitergerührt und dann auf -50° abgekühlt. Dann wird eine Lösung von 13 g (0,06 Mol) N-t-Butoxycarbonyl-L-leucinal in 40 ml Tetrahydrofuran langsam zugegeben. Nach 16 Stunden bei Raumtemperatur wird das Reaktionsgemisch mit 200 ml einer 20 %igen Ammoniumchloridlösung versetzt. Die organische Phase wird über Magnesiumsulfat getrocknet, und das Lösungsmittel unter vermindertem Druck abgedampft. Der Rückstand (24,3 g) wird mit einem 4:1-Gemisch von Hexan und Aether an Kieselgel chromatographiert, wobei man 9,3 g t-Butyl [(1S,2S,4S)-2-hydroxy-1-isobutyl-5-methyl-4-phenylhexyl]-carbamat erhält, welches nach Kristallisation aus Hexan einen Schmelzpunkt von 95-96° aufweist.

## Beispiel 71

In analoger Weise wie in Beispiel 1 beschrieben wurden die folgenden Verbindungen hergestellt:

-Aus 78 mg (0,20 mMol) (S)-α-Amino-N-[(1S,2S,4S)-1-(cyclohexylmethyl)-2-hydroxy-4-isopropyl-5-hexenyl]-imidazol-4-propionamid und 57 mg (0,24 mMol) Aethoxycarbonyl-L-phenylalanin 57 mg (2S,3S,5S)-1-Cyclohexyl-2-[(äthoxycarbonyl)-Phe-His-NH]-5-isopropyl-6-hepten-3-ol in Form eines Festkörpers, MS: 610 $(M+H)^+$;

- Aus 78 mg (0,20 mMol) (S)-α-Amino-N-[(1S,2S,4S)-1-(cyclohexylmethyl)-2-hydroxy-4-isopropyl-5-hexenyl]-imidazol-4-propionamid und 70,8 mg (0,25 mMol) Phenylacetyl-L-phenylalanin 97 mg (2S,3S,5S)-1-Cyclohexyl-2-[(phenylacetyl)-Phe-His-NH]-5-isopropyl-6-hepten-3-ol in Form eines Festkörpers, MS: 656 $(M+H)^+$;

- Aus 78 mg (0,20 mMol) (S)-α-Amino-N-[(1S,2S,4S)-1-(cyclohexylmethyl)-2-hydroxy-4-isopropyl-5-hexenyl]-imidazol-4-propionamid und 68 mg (0,25 mMol) 2-Pyridylcarbonyl-L-phenylalanin 88 mg 1-Cyclohexyl-5-isopropyl-2-[(2-pyridylcarbonyl)-Phe-His-NH]-6-hepten-3-ol als Festkörper, MS: 643 $(M+H)^+$;

- Aus 78 mg (0,20 mMol) (S)-α-Amino-N-[(1S,2S,4S)-1-(cyclohexylmethyl)-2-hydroxy-4-isopropyl-5-hexenyl]-imidazol-4-propionamid und 75 mg (0,25 mMol) 2-Phenylacetyl-L-tyrosin 110 mg (2S,3S,5S)-1-Cyclohexyl-5-isopropyl-2-[(phenylacetyl)-Tyr-His-NH]-6-hepten-3-ol als Festkörper, MS: 672 $(M+H)^+$;

- Aus 78 mg (0,20 mMol) (S)-α-Amino-N-[(1S,2S,4S)-1-(cyclohexylmethyl)-2-hydroxy-4-isopropyl-5-hexenyl]-imidazol-4-propionamid und 72 mg (0,25 mMol) 2-Pyridylcarbonyl-L-tyrosin 96 mg (2S,3S,5S)-1-Cyclohexyl-5-isopropyl-2-[(2-pyridylcarbonyl)-Tyr-His-NH]-6-hepten-3-ol als Festkörper, MS: 659 $(M+H)^+$;

- Aus 175 mg (0,45 mMol) (S)-α-Amino-N-[(1S,2S,4R)-1-(cyclohexylmethyl)-2-hydroxy-4-isopropyl-5-hexenyl]imidazol-4-propionamid und 145 mg (0,5 mMol) 2-Pyridylcarbonyl-L-tyrosin 150 mg (S)-N-[-(1S,2S,4R)-1-(Cyclohexylmethyl)-2-hydroxy-4-isopropyl-5-hexenyl]-α-[(S)-p-hydroxy-α-picolinamidohydrocinnamido]imidazol-4-propionamid als Festkörper, MS: 659 $(M+H)^+$;

- Aus 175 mg (0,45 mMol) (S)-α-Amino-N-[(1S,2S,4R)-1-(cyclohexylmethyl)-2-hydroxy-4-isopropyl-5-hexenyl]imidazol-4-propionamid und 150 mg (0,5 mMol) Phenylacetyl-L-tyrosin 150 mg (S)-N-[(1S,2S,4R)-1-(Cyclohexylmethyl)-2-hydroxy-4-isopropyl-5-hexenyl]-α-[(S)-p-hydroxy-α-(2-phenylacetamido)-hydrocinnamamido]imidazol-4-propionamid als Festkörper, MS: 672 $(M+H)^+$;

- Aus 175 mg (0,45 mMol) (S)-α-Amino-N-[(1S,2S,4R)-1-(cyclohexylmethyl)-2-hydroxy-4-isopropyl-5-hexenyl]imidazol-4-propionamid und 137 mg (0,5 mMol) 2-Pyridylcarbonyl-L-phenylalanin 150 mg (S)-N-[-(1S,2S,4R)-1-(Cyclohexylmethyl)-2-hydroxy-4-isopropyl-5-hexenyl]-α-[(S)-α-picolinamidohydrocinnamamido]-imidazol-4-propionamid als Festkörper, MS: 643 $(M)^+$;

- Aus 175 mg (0,45 mMol) (S)-α-Amino-N[(1S,2S,4R)-1-(cyclohexylmethyl)-2-hydroxy-4-isopropyl-5-hexenyl]imidazol-4-propionamid und 141 mg (0,50 mMol) Phenylacetyl-L-phenylalanin 150 mg (S)-N-[-(1S,2S,4R)-1-(Cyclohexylmethyl)-2-hydroxy-4-isopropyl-5-hexenyl]-α-[(S)-α-(2-phenylacetamido)-hydrocinnamamido)imidazol-4-propionamid als Festkörper, MS: 656 $(M+H)^+$;

- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 66 mg 1-(Imidazolyl)-3-propionyl-D-prolin 126 mg (S)-N-[-(1S,2S,4S)-1-(Cyclohexylmethyl)-2-hydroxy-4-isopropyl-5-hexenyl]-α-[(S)-α-[(R)-1-[3-(1H-imidazol-1-yl)-propionyl]-2-pyrrolidincarboxamido]hydrocinnamamido]]imidazol-4-propionamid als Festkörper, MS: 757 $(M+H)^+$;

- Aus 111 mg (0,20 mMol) (S)-α-[p-Fluor-L-alanyl)amino]-N-[(1S,2S,4S)-1-(cyclohexylmethyl)-2-hydroxy-4-

isopropyl-5-hexenyl]imidazol-4-propionamid und 47, 8 mg (0,24 mMol) 2-(2-Pyridyl)benzoesäure 120 mg (S)-N-[(1S,2S,4S)-1-(Cyclohexylmethyl)-2-hydroxy-4-isopropyl-5-hexenyl]-α-[(S)-α-[2-(2-pyridyl)benzamido]-p-fluor(hydrocinnamamido)]imidazol-4-propionamid als Festkörper, MS: 737 $(M+H)^+$;

- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 75 mg (0,24 mMol) Boc-D-Pro-Pro-OH 122 mg (2S,5S)-2-(Boc-D-Pro-Pro-Phe-His-NH)-1-cyclohexyl-5-isopropyl-6-hepten-3-ol als Festkörper, MS: 832 $(M+H)^+$.
- Aus 269 mg (0,6 mMol) N-[(1S,2S,4R)-1-(Cyclohexylmethyl)-2-hydroxy-4-isopropyl-5-hexenyl]-α-(3-phenyl-L-alanyl)imidazol-4-propionamid und 187 mg (0,6 mMol) Boc-D-Pro-Pro-OH 400 mg t-Butyl (R)-2-[[(S)-2-[[(S)-α-[[(S)-1-[[(1S,2S,4R)-1-(cyclohexylmethyl)-2-hydroxy-4-isopropyl-5-hexenyl]carbamoyl]-2-imidazol-4-yläthyl]carbamoyl]phenäthyl]carbamoyl]-1-pyrrolidinyl]carbonyl]-1-pyrrolidincarboxylat als Festkörper, MS: 832 $(M+H)^+$.
- Aus 269 mg (0,5 mMol) N-[(1S,2S,4R)-1-(Cyclohexylmethyl)-2-hydroxy-4-isopropyl-5-hexenyl]-α-(3-phenyl-L-alanyl)imidazol-4-propionamid und 260 mg (0,6 mMol) t-Butoxycarbonyl-D-phenylglycin 110 mg t-Butyl [[(R)-α-[[(S)-α-[[(S)-1-[[(1S,2S,4R)-1-(cyclohexylmethyl)-2-hydroxy-4-isopropyl-5-hexenyl]carbamoyl]-2-imidazol-4-yläthyl]carbamoyl]phenäthyl]carbamoyl]benzyl]carbamat als Festkörper, MS 771 $(M+H)^+$.
- Aus 269 mg (0,5 mMol) N-[(1S,2S,4R)-1-(Cyclohexylmethyl)-2-hydroxy-4-isopropyl-5-hexenyl]-α-(3-phenyl-L-alanyl)imidazol-4-propionamid und 119 mg (0,6 mMol) 2-(2-Pyridyl)benzoesäure 290 mg (S)-N-[(2S,2S,4R)-1-(Cyclohexylmethyl)-2-hydroxy-4-isopropyl-5-hexenyl]-α-[(S)-α-[2-(2-pyridyl)benzamido]-hydrocinnamamido]imidazol-4-propionamid als Festkörper, MS: 719 $(M+H)^+$.
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 75 mg (0,24 mMol) Boc-Pro-Pro-OH 137 mg (2S,3S,5S)-2-(Boc-Pro-Pro-Phe-His-NH)-1-cyclohexyl-5-isopropyl-6-hepten-3-ol als Festkörper, MS: 832 $(M+H)^+$;
- Aus 107 mg (0,20 mMol) N-[(1S,2S,4R)-1-(Cyclohexylmethyl)-2-hydroxy-4-isopropyl-5-hexenyl]-α-(3-phenyl-L-alanyl)imidazol-4-propionamid und 71 mg (0,24 mMol) IVA-D-Pro-L-Pro-OH 130 mg (S)-N-[(1S,2S,4R)-1-(Cyclohexylmethyl)-2-hydroxy-4-isopropyl-5-hexenyl]-α-[[N-[1-[(1-isovaleryl-D-prolyl)-L-prolyl]-3-phenyl-L-alanyl]amino]imidazol-4-propionamid als Festkörper, MS: 816 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 67 mg (0,24 mMol) N-t-Butoxycarbonyl-N-methyl-L-phenyl alanin 100 mg t-Butyl [(S)-α-[[(S)-α-[[(S)1-[[(1S,2S,4S)-1-(cyclohexylmethyl)-2-hydroxy-4-isopropyl-5-hexenyl]-carbamoyl]-2-imidazol-4-yläthyl]carbamoyl]phenäthyl]carbamoyl]phenäthyl]methylcarbamat als Festkörper, MS: 799 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 71 mg (0,24 mMol) IVA-D-Pro-Pro-OH 141 mg (2S,3S,5S)-1-Cyclohexyl-2-[(3-methylbutyryl)-D-Pro-Pro-Phe-His-NH]-5-isopropyl-6-hepten-3-ol als Festkörper, MS: 816 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 74 mg (0,24 mMol) Boc-(Δ3-deshydro)-Pro-Pro-OH 125 mg (2S,3S,5S)-2-[[[(S)-1-Boc-3-pyrrolin-2-yl]carbonyl]-Pro-Phe-His-NH]-1-cyclohexyl-5-isopropyl-6-hexen-3-ol als Festkörper, MS: 830 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 75 mg (0,24 mMol) Boc-Pro-D-Pro-OH 135 mg (2S,3S,5S)-2-(Boc-Pro-D-Pro-Phe-His-NH)-1-cyclohexyl-5-isopropyl-6-hepten-3-ol als Festkörper, MS: 832 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 75 mg (0,24 mMol) Boc-D-Pro-D-Pro-OH 133 mg (2S,3S,5S)-2-(Boc-D-Pro-D-Pro-Phe-His-NH)-1-cyclohexyl-5-isopropyl-6-hepten-3-ol als Festkörper, MS: 832 $(M+H)^+$;
- Aus 110 mg (0,20 mMol) (S)-α-(Phe-methylamino)-N-[(1S,2S,4S)-1-(cyclohexylmethyl)-2-hydroxy-4-isopropyl-5-hexenyl]imidazol-4-propionamid und 75 mg (0,24 mMol) Boc-D-Pro-Pro-OH 87 mg (S)-α-[Boc-D-Pro-Pro-Phe-N($CH_3$)]-N-[(1S,2S,4S)-1-(cyclohexylmethyl)-2-hydroxy-4-isopropyl-5-hexenyl]imidazol-4-propionamid als Festkörper, MS: 846 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 55 mg (0,24 mMol) (S)-N-t-Butoxycarbonyl-piperidin-2-carbonsäure 127 mg (2S,3S,5S)-1-[[[(S)-1-Boc-2-piperidinyl]carbonyl]-Phe-His-NH]-1-cyclohexyl-5-isopropyl-6-hepten-3-ol als Festkörper, MS: 749 $(M+H)^+$;
- Aus 54 mg (0,10 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 49 mg (0,12 mMol) Boc-D-Pro-D-Pro-Pro-OH 57 mg (2S,3S,5S)-2-(Boc-D-Pro-D-Pro-Pro-Phe-His-NH)-1-cyclohexyl-5-isopropyl-6-hepten-3-ol als Festkörper, MS: 927 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 56 mg (0,24 mMol) (R)-N-t-Butoxycarbonyl-thiazolidin-2-carbonsäure 126 mg (2S,3S,5S)-2-[[[(R)-3-Boc-4-thiazolidinyl]carbonyl-Phe-His-NH]-1-cyclohexyl-5-isopropyl-6-hepten-3-ol als Festkörper, MS: 753 $(M+H)^+$;

- Aus 80 mg (0,123 mMol) (2S,3S,5S)-1-[[[(S)-2-Piperidinyl]carbonyl]-Phe-His-NH]-1-cyclohexyl-5-isopropyl-6-hepten-3-ol und 64 mg (0,30 mMol) Boc-D-Pro-OH 106 mg (2S,3S,5S)-2-(Boc-D-Pro-Pip-Phe-His-NH)-1-cyclohexyl-5-isopropyl-6-hepten-3-ol als Festkörper, MS: 846 $(M^+)$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 79 mg (0,24 mMol) (R)-N-t-Butoxycarbonyl-thiazolidinyl-1-carbonyl-prolin 163 mg (2S,3S,5S)-2-[[[(R)-3-Boc-4-thiazolidinyl]carbonyl]-Pro-Phe-His-NH]-1-cyclohexyl-5-isopropyl-6-hepten-3-ol als Festkörper, MS: 850 $(M+H)^+$;
- Aus 107 mg (0,20 mMol) N-[(1S,2S,4S)-1-(Cyclohexylmethyl)-2-hydroxy-4-isopropyl-5-hexenyl]-α-(3-phenyl-L-alanyl)imidazol-4-propionamid und 71 mg (0,24 mMol) 2-(2-Pyridyl)benzoesäure 140 mg (2S,3S,5S)-1-Cyclohexyl-5-isopropyl-2-[[2-(2-pyridyl)benzoyl]-Pro-Phe-His-NH]-6-hepten-3-ol in Form des Dihydrochlorids, MS: 816 $(M+H)^+$;
- Aus 107 mg (0,20 mMol) (N-[(1S,2S,4S)-1-(Cyclohexylmethyl)-2-hydroxy-4-isopropyl-5-hexenyl]-α-(3-phenyl-L-alanyl)imidazol-4-propionamid und 57 mg (0,24 mMol) 3-Dimethyl-3-(1-imidazolyl)propionyl-Pro-Pro-OH 160 mg (2S,3S,5S)-1-Cyclohexyl-2-[(3,3-dimethyl-3-imidazol-1-ylpropionyl)-Pro-Pro-Phe-His-NH]-5-isopropyl-6-hepten-3-ol als Festkörper, MS: 882 $(M+H)^+$;
- Aus 107 mg (0,20 mMol) (N-[(1S,2S,4S)-1-(Cyclohexylmethyl)-2-hydroxy-4-isopropyl-5-hexenyl]-α-(3-phenyl-L-alanyl)imidazol-4-propionamid und 77 mg (0,24 mMol) N-(1-Imidazolyl)acetyl-D-Pro-Pro-OH 160 mg (2S,3S,5S)-1-Cyclohexyl-2-[(imidazol-1-ylacetyl)-D-Pro-Pro-Phe-His-NH]-5-isopropyl-6-hepten-3-ol in Form des Dihydrochlorids als Festkörper, MS: 840 $(M+H)^+$;
- Aus 107 mg (0,20 mMol) (N-[(1S,2S,4R)-1-(Cyclohexylmethyl)-2-hydroxy-4-isopropyl-5-hexenyl]-α-(3-phenyl-L-alanyl)imidazol-4-propionamid und 77 mg (0,24 mMol) N-(1-Imidazolyl)acetyl-D-Pro-Pro- OH 148 mg (2S,3S,5R)-1-Cyclohexyl-2-[(imidazol-1-ylacetyl)-D-Pro-Pro-Phe-His-NH]-5-isopropyl-6-hepten-3-ol in Form des Dihydrochlorids, als Festkörper, MS: 840 $(M+H)^+$;
- Aus 107 mg (0,20 mMol) (N-[(1S,2S,4S)-1-(Cyclohexylmethyl)-2-hydroxy-4-isopropyl-5-hexenyl]-α-(3-phenyl-L-alanyl)imidazol-4-propionamid und 83 mg (0,24 mMol) Benzyloxycarbonyl-Pro-Pro-OH 155 mg (2S,3S,5S)-2-(Benzyloxycarbonyl-Pro-Pro-Phe-His-NH)-1-cyclohexyl-5-isopropyl-6-hepten-3-ol als Festkörper, MS: 866 $(M+H)^+$;

Das als Ausgangsmaterial eingesetzte (S)-α-(Phe-methylamino)-N-[(1S,2S,4S)-1-(cyclohexylmethyl)-2-hydroxy-4-isopropyl-5-hexenyl]imidazol-4-propionamid wurde wie folgt hergestellt:

Ein Gemisch von 1,50 g (4 mMol) N-α-N-im-Di-t-butoxycarbonyl-N-α-methylhistidin, 1,0 g (3,4 mMol) (S,S,S)-6-Amino-6-cyclohexylmethyl-4-isopropyl-1-hexen-5-ol-hydrochlorid, 1,33 g (3,5 mMol) HBTU, 7,8 ml N-Methylmorpholin und 20 ml Dimethylformamid wird 4 Stunden bei Raumtemperatur gerührt. Danach wird das Lösungsmittel unter vermindertem Druck abgedampft, und der ölige Rückstand in Aether gelöst. Die organische Lösung wird zunächst mit einer Natriumbicarbonatlösung und danach mit gesättigter Kochsalzlösung gewaschen und nach dem Trocknen über Natriumsulfat unter vermindertem Druck eingedampft, wobei man 2,1 g (S)-3-(t-Butoxycarbonyl)-α-[1-(t-butoxycarbonyl)-N-methylformamido]-N-[(1S,2S,4S)-1-(cyclohexylmethyl)-2-hydroxy-4-isopropyl-5-hexenyl]imidazol-4-propionamid erhält, welches direkt in die nächste Stufe eingesetzt wird.

2,1 g (S)-3-(t-Butoxycarbonyl)-α-[1-(t-butoxycarbonyl)-N-methylformamido]-N-[(1S, 2S,4S)-1-(cyclohexylmethyl)-2-hydroxy-4-isopropyl-5-hexenyl]imidazol-4-propion amid werden in 30 ml 3N Chlorwasserstoff in Dioxan gelöst und 5 Stunden bei Raumtemperatur stehengelassen. Danach werden 500 ml Aether zugegeben, und der gebildete Niederschlag abfiltriert. Dann wird der Niederschlag mit Aether gewaschen und in Wasser gelöst. Zur wässrigen Lösung gibt man festes Natriumbicarbonat zu und extrahiert mehrmals mit Essigester, wobei man 0,85 g (S)-α-(N-Methylformamido)-N-[(1S,2S,4S)-1-(cyclohexylmethyl)-2-hydroxy-4-isopropyl-5-hexenyl]imidazol-4-propionamid erhält, welches direkt in die nächste Stufe eingesetzt wird.

Ein Gemisch von 0,85 g (2,1 mMol) (S)-α-(N-Methylformamido)-N-[(1S,2S,4S)-1-(cyclohexylmethyl)-2-hydroxy-4-isopropyl-5-hexenyl]imidazol-4-propionamid, 0,67 g (2,5 mMol) N-t-Butoxycarbonylphenylalanin, 0,84 g (2,2 mMol) HBTU, 0,3 ml N-Methylmorpholin und 20 ml Dimethylformamid wird 60 Stunden bei Raumtemperatur stehengelassen und danach auf eine gesättigte Natriumbicarbonatlösung gegossen. Der dabei gebildete Niederschlag wird abfiltriert und in Essigester gelöst. Die organische Lösung wird über Magnesiumsulfat getrocknet und unter vermindertem Druck eingedampft. Der verbleibende Rückstand wird mit Hexan verrieben und abfiltriert, wobei man 1,35 g (S)-α-(Boc-Phe-methylamino)-N-[(1S,2S,4S)-1-(cyclohexylmethyl)-2-hydroxy-4-isopropyl-5-hexenyl]imidazol-4-propionamid in Form eines Pulvers erhält, welches, direkt in die nächste Stufe eingesetzt wird.

1,3 g (S)-α-(Boc-Phe-methylamino)-N-[(1S,2S,4S)-1-(cyclohexylmethyl)-2-hydroxy-4-isopropyl-5-hexenyl]imidazol-4-propionamid werden in 30 ml 3N Chlorwasserstoff in Dioxan gelöst und 1 Stunde bei Raumtemperatur stehengelassen. Danach gibt man 200 ml Aether zu und lässt 16 Stunden bei Raumtemperatur rühren. Der gebildete Niederschlag wird abfiltriert, mit Aether gewaschen und in Wasser gelöst. Zur wässrigen Lösung gibt man danach festes Natriumbicarbonat und extrahiert dann mehrmals mit Essigester, wobei man 0,97 g (S)-α-(Phe-methylamino)-N-[(1S,2S,4S)-1-(cyclohexylmethyl)-2-hydroxy-4-isopropyl-5-hexenyl]imidazol-4-propionamid erhält, welches man direkt in die nächste Stufe einsetzt.

Das ebenfalls als Ausgangsmaterial eingesetzte (2S,3S,5S)-1-[[[(S)-2-Piperidinyl]carbonyl]-Phe-His-NH]-1-cyclohexyl-5-isopropyl-6-hepten-3-ol wurde durch Abspaltung der t-Butoxycarbonylgruppe aus (2S,3S,5S)-1-[[[(S)-1-Boc-2-piperidinyl]carbonyl-Phe-His-NH]-1-cyclohexyl-5-isopropyl-6-hepten-3-ol mittels Chlorwasserstoffsäure in Dioxan hergestellt und direkt in die nächste Stufe eingesetzt.

## Beispiel 72

Ein Gemisch von 31 mg (0,21 mMol) N-Cyano-dimethyldithiocarbamat, 107 mg (0,20 mMol) N-[-(1S,2S,4S)-1-(Cyclohexylmethyl]-2-hydroxy-4-isopropyl-5-hexenyl]-α-(3-phenyl-L-alanyl)imidazol-4-propionamid und 1 ml Methanol wird 24 Stunden bei Raumtemperatur stehengelassen. Danach wird das Lösungsmittel unter vermindertem Druck eingedampft, und der Rückstand mit einem 1:1-Gemisch von Hexan und Aether digeriert, wobei man 112 mg (S)-N-[(1S,2S,4S)-1-(Cyclohexylmethyl)-2-hydroxy-4-isopropyl-5-hexenyl]-α-[[N-[(cyanoimino)(methylthio)methyl]-3-phenyl-L-alanyl]amino]imidazol-4-propionamid als Festkörper erhält, MS: 636 $(M+H)^+$.

## Beispiel 73

Man löst 100 mg (2S,5S)-2-(Boc-D-Pro-Pro-Phe-His-NH)-1-cyclohexyl-5-isopropyl-6-hepten-3-ol in 3 ml 3,2N Chlorwasserstoff in Dioxan, lässt die Lösung 1 Stunde stehen und fällt danach das erwünschte Produkt durch Zugabe von Aether aus. Der Niederschlag wird abfiltriert und unter reduziertem Druck getrocknet, wobei man 100 mg [2S,3S,5S]-1-Cyclohexyl-2-(D-Pro-Pro-Phe-His-NH)-5-isopropyl-6-hepten-3-ol in Form des Dihydrochlorids als Festkörper erhält, MS: 732 $(M+H)^+$.

## Beispiel 74

41 mg (0,05 mMol) (2S,3S,5S)-1-Cyclohexyl-2-[(3-methylbutyryl)-D-Pro-Pro-Phe-His-NH]-5-isopropyl-6-hepten-3-ol werden in 5 ml Methanol gelöst und in Gegenwart von 20 mg Palladium auf Kohle (5 %ig) hydriert. Nach beendeter Wasserstoffaufnahme wird der Katalysator abfiltriert, und das Lösungsmittel unter vermindertem Druck abgedampft. Zerreiben des Rückstands mit Hexan und Trocknen liefert 34 mg (2S,3S,5S)-1-Cyclohexyl-5-isopropyl-2-(isovaleryl-D-Pro-Pro-Phe-His-NH)-3-heptanol als Festkörper, MS: 818 $(M+H)^+$.

Man kann die obige Hydrierung auch so lenken, dass man die entsprechende isomere Verbindung erhält, wenn man die Hydrierung unter den folgenden Bedingungen durchführt:

200 mg Ausgangsmaterial in 15 ml Methanol werden in Gegenwart von 50 mg Palladium auf Kohle (5 %ig) hydriert und danach üblicherweise aufgearbeitet, wobei man 184 mg (2S,3S,5R)-1-Cyclohexyl-5-isopropyl-2-(isovaleryl-D-Pro-Pro-Phe-His-NH)-3-heptanol als Festkörper erhält, MS: 818 $(M+H)^+$.

## Beispiel 75

80 mg (0,25 mMol) 3-(1-Imidazolyl)propionyl-Pro-Pro-OH, 110 mg (0,20 mMol) (S)-α-(Phe-methylamino)-N-[(1S,2S,4S)-1-(cyclohexylmethyl)-2-hydroxy-4-isopropyl-5-hexenyl]imidazol-4-propionamid, 84 mg (0,22 mMol) HBTU, 0,03 ml N-Methylmorpholin und 2 ml Dimethylformamid werden während 16 Stunden bei Raumtemperatur stehengelassen. Das Reaktionsgemisch wird danach auf 50 ml einer 5 %igen

Natriumcarbonat lösung gegossen, und die milchige Suspension mehrmals mit Essigester extrahiert. Die organischen Extrakte werden über Magnesiumsulfat getrocknet und unter vermindertem Druck eingedampft. Der Rückstand wird in 10 ml Essigester gelöst, und die Lösung mit 1,5 ml einer 3,0N Lösung von Chlorwasserstoff in Dioxan versetzt. Der dabei gebildete Niederschlag wird abfiltriert und getrocknet, wobei man 143 mg (S)-N-[(1S,2S,4S)-1-(Cyclohexylmethyl)-2-hydroxy-4-isopropyl-5-hexenyl]-α-[[3-(imidazol-1-yl)-propionyl]-D-Pro-Pro-Phe-N-(methyl)]imidazol-4-propionamidhydrochlorid als Festkörper erhält, MS: 868 $(M+H)^+$.

In analoger Weise wie oben beschrieben wurde aus 77 mg (0,23 mMol) 2-(1-Imidazolyl)acetyl-Pro-Pro-OH und 110 mg (S)-α-(Phemethylamino)-N-[(1S,2S,4S)-1-(cyclohexylmethyl)-2-hydroxy-4-isopropyl-5-hexenyl]imidazol-4-propionamid 155 mg (S)-N-[(1S,2S,4S)-1-(Cyclohexylmethyl)-2-hydroxy-4-isopropyl-5-hexenyl]-α-[(imidazol-1-ylacetyl)-D-Pro-Pro-Phe-N-(methyl)]imidazol-4-propionamid-dihydrochlorid als Festkörper hergestellt, MS: 840 $(M+H)^+$.

## Beispiel 76

105 mg (S)-N-[(1S,2S,4S)-1-(Cyclohexylmethyl)-2-hydroxy-4-isopropyl-5-hexenyl]-α-[[3-(imidazol-1-yl)-propionyl]-D-Pro-Pro-Phe-N-(methyl)]imidazol-4-propionamid--hydrochlorid werden in 15 ml Methanol gelöst und in Gegenwart von 50 mg Palladium auf Kohle hydriert. Nach beendeter Wasserstoffaufnahme wird der Katalysator abfiltriert, und das Filtrat zur Trockene eingedampft. Der Rückstand wird mit Aether verrieben und abfiltriert, wobei man 65 mg (2S,3S,5S)-1-Cyclohexyl-2-[(3-imidazol-1-ylpropionyl)-D-Pro-Pro-Phe-His-NH]-5-isopropyl-3-heptanol-dihydrochlorid als Festkörper erhält, MS: 856 $(M+H)^+$.

## Beispiel 77

Eine sterilfiltrierte wässrige Lösung von (S)-α-[(R)-2-Benzyl-5,5-dimethyl-4-oxohexanamido]-N-[-(1S,2S,4S)-1-(cyclohexylmethyl)-2-hydroxy-4-isopropyl-5-hexenyl]imidazol-4-propionamid wird unter Erwärmen mit einer sterilen Gelatinelösung, welche als Konservierungsmittel Phenol enthält, unter aseptischen Bedinungen so vermischt, dass 1,0 ml Lösung folgende Zusammensetzung hat:

| | |
|---|---|
| (S)-α-[(R)-2-Benzyl-5,5-dimethyl-4-oxohexanamido]-N-[(1S,2S,4S)-1-(cyclohexylmethyl)-2-hydroxy-4-isopropyl-5-hexenyl]imidazol-4-propionamid | 3,0 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel 78

Man löst 5 mg (S)-α-[(R)-2-Benzyl-5,5-dimethyl-4-oxohexanamido]-N-[(1S,2S,4S)-1-(cyclohexylmethyl)-2-hydroxy-4-isopropyl-5-hexenyl]imidazol-4-propionamid in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 79

In einer Mischung von 3,5 ml Myglyol 812 und 0,08 g Benzylalkohol werden 500 mg fein gemahlenes (5,0 μm) (S)-α-[(R)-2-Benzyl-5,5-dimethyl-4-oxohexanamido]-N-[(1S,2S,4S)-1-(cyclohexylmethyl)-2-hydroxy-4-isopropyl-5-hexenyl]imidazol-4-propionamid suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

## Ansprüche

1. Aminosäurederivate der allgemeinen Formel

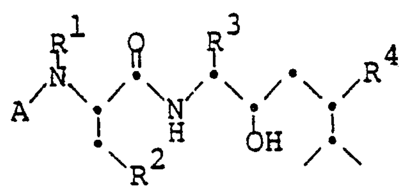

I

worin $R^1$ Wasserstoff oder Methyl, $R^2$ Aethyl, Propyl oder Imidazol-4-yl, $R^3$ Isobutyl, Cyclohexylmethyl oder Benzyl, $R^4$ Phenyl, Furyl, Vinyl, Aethyl oder 1,2-Dihydroxyäthyl und A über die Carboxylgruppe gebundene Dibenzsuberanessigsäure, 2-(2-Pyridyl)benzosäure, ß-Naphthylbernsteinsäuremonoäthylester, 2-Indolylessigsäure oder Dihydrozimtsäure oder eine der Gruppen

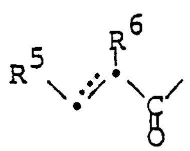

und

$-Y-Z$

(a) (b)

bedeuten, worin die gestrichelte Linie eine zusätzliche Bindung bedeuten kann, $R^5$ Phenyl, Naphthyl, Indolyl, Pyrazolyl, Imidazolyl oder Pyridylmethyl und $R^6$ Wasserstoff, Alkyl, Arylalkyl, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminocarbonyl, substituiertes Aminocarbonyl, Alkoxycarbonyl, Alkylcarbonyloxy, Arylalkylcarbonyloxy, Alkylaminocarbonyloxy, Alkoxycarbonylamino, Cyan oder 2,5-Dimethylpyrrol-1-yl bedeuten, mit der Massgabe dass $R^4$ nicht Alkoxycarbonylamino bedeuten kann, wenn $R^3$ Phenyl oder α-Naph thyl bedeutet, und Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, α-Naphthylalanin, Homophenylalanin, Asparaginsäure-

äthylester, Glutaminsäure-t-butylester oder Glutaminsäurebenzylester und Z Wasserstoff, Acyl oder eine der Gruppen

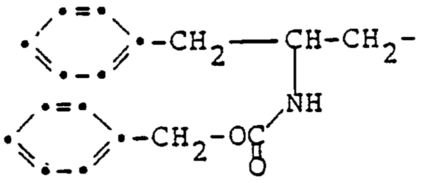

(c)

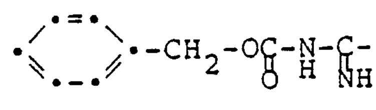

(d)

und

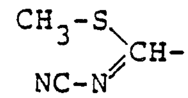

(e)

bedeuten,
in Form von optisch reinen Diastereomeren, Diastereomerengemischen, diastereomeren Racematen oder Gemischen von diastereomeren Racematen sowie pharmazeutisch verwendbare Salze dieser Verbindungen.

2. Verbindungen gemäss Anspruch 1, worin $R^1$ Wasserstoff, $R^2$ Imidazol-4-yl, $R^3$ Cyclohexylmethyl und $R^4$ Vinyl oder Aethyl bedeuten.

3. Verbindungen gemäss Anspruch 1 oder 2, worin A die Gruppe (a) oder (b) bedeutet, worin $R^5$ Phenyl oder Naphthyl, $R^6$ Alkylcarbonylalkyl, Cycloalkylcarbonylalkyl, Heterocycloalkylcarbonylalkyl, Aminocarbonylalkyl oder substituiertes Aminocarbonylalkyl, Y Phenylalanin, Cyclohexylalanin, 4-Chlorphenylalanin oder α-Naphthylalanin und Z Acyl bedeuten.

4. Verbindungen gemäss Anspruch 3, worin $R^5$ Phenyl, $R^6$ $C_1$-$C_4$-Alkylcarbonylmethyl, $C_5$-$C_6$-Cycloalkylcarbonylmethyl, N-t-Butoxycarbonylpyrrolidinyl carbonylmethyl oder Aminocarbonylalkyl, welches durch $C_1$-$C_4$-Alkyl oder gegebenenfalls durch ein Sauerstoffatom unterbrochenes $C_4$-$C_5$-Alkylen disubstituiert oder durch Alkoxycarbonylalkyl oder Alkoxycarbonyl mono- oder di-substituiertes Aminoalkyl monosubstituiert ist und Y Phenylalanin bedeuten und Z den C-terminal mit Y verbundenen Rest einer N- und/oder α-methylierten, gegebenenfalls durch die Gruppe $R^b$-CO- oder $R^a$-O-CO- N-substituierten, natürlichen Aminosäure mit der L-Konfiguration oder eines Epimeren einer solchen Aminosäure mit der D-Konfiguration oder eines Dipeptids aus zwei solchen Aminosäuren oder die Gruppe $R^b$-CO- oder $R^a$-O-CO- bedeutet, worin $R^a$ einen gegebenenfalls substituierten, gesättigten oder ungesättigten aliphatischen, cycloaliphatischen, cycloaliphatisch-aliphatischen Kohlenwasserstoffrest mit bis zu 18 Kohlenstoffatomen, einen gegebenenfalls substituierten aromatischen, heteraromatischen, aromatisch-aliphatischen oder heteroaromatisch-aliphatischen Kohlenwasserstoffrest mit bis zu 18 Kohlenstoffatomen oder einen gegebenenfalls substituierten, gesättigten 5- oder 6-gliedrigen Heterocyclus bedeutet und $R^b$ Wasserstoff bedeutet oder die Bedeutung von $R^a$ besitzt, vorzugsweise den C-terminal mit Y verbundenen Rest von Prolin, Prolylprolin oder Histidinylprolin bedeutet, wobei die Aminogruppe jeweils durch t-Butoxycarbonyl, Benzoxycarbonyl, Isovaleryl oder die Gruppe $R^b$-CO- substituiert ist, worin $R^b$ einen heteroaromatisch-aliphatischen Kohlenwasserstoffrest mit bis zu 10 Kohlenwasserstoffatomen oder einen gesättigten 5- oder 6-gliedrigen Heterocyclus bedeutet.

5. Verbindungen gemäss einem der Ansprüche 1-4, worin $R^1$ Wasserstoff, $R^2$ Imidazol-4-yl, $R^3$ Cyclohexylmethyl, $R^4$ Vinyl oder Aethyl und $R^5$ Phenyl und $R^6$ $C_1$-$C_4$-Alkylcarbonylmethyl, $C_5$-$C_6$-Cycloalkylcarbonylmethyl, N-t-Butoxycarbonylpyrrolidinylcarbonylmethyl oder Aminocarbonylalkyl, welches durch $C_1$-$C_4$-Alkyl oder gegebenenfalls durch ein Sauerstoffatom unterbrochenes $C_4$-$C_5$- Alkylen disubstituiert oder durch Alkoxycarbonylalkyl oder Alkoxycarbonyl mono- oder di-substituiertes Aminoalkyl monosubstituiert ist, oder Y den N-terminal mit Z verbundenen, bivalenten Rest von Phenylalanin und Z den C-terminal mit Y verbundenen Rest von Prolin, Prolylprolin oder Histidinylprolin bedeuten, wobei die Aminogruppe jeweils durch t-Butoxycarbonyl, Benzoxycarbonyl, Isovaleryl oder die Gruppe $R^b$-CO- substituiert ist, worin $R^b$ einen heteroaromatisch-aliphatischen Kohlenwasserstoffrest mit bis zu 10 Kohlenstoffatomen oder einen gesättigten 5- oder 6-gliedrigen Heterocyclus bedeutet.

6. t-Butyl (R)-2-[[(S)-α-[[(S)-1-[(1S,2S,4S)-1-(cyclohexylmethyl-2-hydroxy-4-isopropylhexyl]-2-imidazol-4-yläthyl]carbamoyl]phenäthyl]carbamoyl]-1-pyrrolidincarboxylat.

7. (2S,3S,5S)-2-(Boc-D-Pro-Phe-His-NH)-1-cyclohexyl-5-isopropyl-6-hepten-3-ol.

8. t-Butyl (S)-2-[[(R)-α-[[(S)-1-[[(1S,2S,4S)-1-(cyclohexylmethyl)-2-hydroxy-4-isopropylhexyl]carbamoyl]-2-imidazol-4-yläthyl]carbamoyl]phenäthyl]acetyl]-1-pyrrolidincarboxylat.

9. N-(S)-1-[[(1S,2S,4S)-1-(Cyclohexylmethyl)-2-hydroxy-4-isopropylhexyl]carbamoyl]-2-imidazol-4-yläthyl]-γ-oxo-α-(1-naphthylmethyl)-4-morpholinbutyramid.

10. (2S,5S)-2-(Boc-D-Pro-Pro-Phe-His-NH)-1-cyclohexyl-5-isopropyl-6-hepten-3-ol.

11. N-(S)-[(1S,2S,4S)-1-(Cyclohexylmethyl-2-hydroxy-4-isopropyl-5-hexenyl]-α-[(S)-α-3-methylbutyramido]imidazol-4-propionamid,
t-Butyl[(S)-α-[[(S)-1-[[(1S,2S,4S)-1-cyclohexylmethyl-2-hydroxy-4-isopropyl-5-hexenyl]carbamoyl]-2-imidazol-4-yläthyl]carbamoyl]phenäthyl]-carbamat,
t-Butyl [(S)-α-[[(S)-1-[[(1S,2S,4S)-1-cyclohexylmethyl-2-hydroxy-4-isopropylhexyl]carbamoyl]-2-imidazol-4-yläthyl] carbamoyl]phenäthyl]carbamat,
(S)-N-[(1S,2S,4S)-1-(Cyclohexylmethyl)-2-hydroxy-4-isopropylhexyl]-α-[[[[(R)-α-2-hydroxy-1-(hydroxymethyl)-1-methyläthyl]carbamoyl]methyl]hydrocinnamamido]imidazol-4-propionamid,
t-Butoxycarbonyl [2-[(R und S)-3-[[(S)-1-[[(1S,2S,4S)-1-(cyclohexylmethyl)-2-hydroxy-4-isopropyl-5-hexenyl]-carbamoyl]-2-imidazol-4-yläthyl]carbamoyl]-4-phenylbutyramido]äthyl]glycin-t-butylester,
(S)-N-[(1S,2S,4S)-1-(Cyclohexylmethyl)-2-hydroxy-4-isopropyl-5-hexenyl]-α-[(R)-α-[[[2-hydroxy-1-(hydroxymethyl)-1-methyläthyl]carbamoyl]methyl]hydrocinnamamido]imidazol-4-propionamid,
(S)-α-[(R)-2-Benzyl-5,5-dimethyl-4-oxohexanamido]-N-[(1S,2S,4S)-1-(cyclohexylmethyl)-2-hydroxy-4-isopropyl-5-hexenyl]imidazol-4-propionamid,
(S)-N-[(1S,2S,4S)-1-(Cyclohexylmethyl)-2-hydroxy-4-isopropyl-5-hexenyl]-α-2-[N-(morpholinocarbamoyl)-3-phenyl-L-alanyl]amino]imidazol-4-propionamid,
(S)-α-[(R)-α-(Carbamoylmethyl)hydrocinnamamido]-N-[(1S,2S,4S)-1-(cyclohexylmethyl)-2-hydroxy-4-isopropyl-5-hexenyl]imidazol-4-propionamid,
(S)-N-[(1S,2S,4S)-1-(Cyclohexylmethyl)-2-hydroxy-4-isopropyl-5-hexenyl]-α-[(R)-α-[(dimethylcarbamoyl)-methyl]hydrocinnamamido]imidazol-4-propionamid,
(S)-N-[(1S,2S,4S)-1-(Cyclohexylmethyl)-2-hydroxy-4-isopropyl-5-hexenyl]-α-[(RS)-α-[(cylcopentylcarbonyl)-methyl]hydrocinnamamido]imidazol-4-propionamid oder
(S)-N-[(1S,2S,4S)-1-(Cyclohexylmethyl)-2-hydroxy-4-isopropylhexyl]-α-[(RS)-α-[(cyclopentylcarbonyl)-methyl]hydrocinnamamido]imidazol-4-propionamid.

12. Verbindungen der allgemeinen Formeln

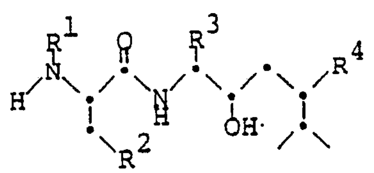 II, 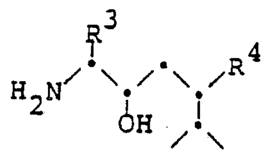 III und 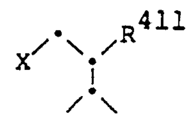 VI'

worin $R^1$ Wasserstoff oder Methyl, $R^2$ Aethyl, Propyl oder Imidazol-4-yl, $R^3$ Isobutyl, Cyclohexylmethyl oder Benzyl, $R^4$ Phenyl, Furyl, Vinyl, Aethyl oder 1,2-Dihydroxyäthyl, X Chlor, Brom oder Jod und $R^{411}$ Phenyl oder Furyl bedeuten.

13. Aminosäurederivate gemäss einem der Ansprüche 1-11 zur Anwendung als therapeutische Wirkstoffe.

14. Aminosäurederivate gemäss einem der Ansprüche 1-11 zur Anwendung bei der Bekämpfung bzw. Verhütung von Bluthochdruck und Herzinsuffizienz.

15. Verfahren zur Herstellung einer Verbindung gemäss einem der Ansprüche 1-11, dadurch gekennzeichnet, dass man

a) eine Verbindung der allgemeinen Formel

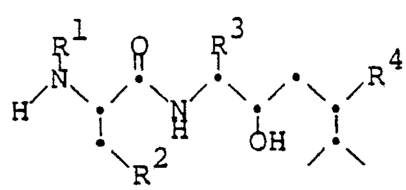 II

worin $R^1$, $R^2$, $R^3$ und $R^4$ die in Anspruch 1 angegebene Bedeutung besitzen, mit einem die Gruppe

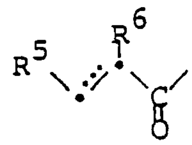

oder

-Y-Z

(a) (b)

worin $R^5$, $R^6$, Y, Z und die gestrichelte Linie die in Anspruch 1 angegebene Bedeutung besitzen, abgebenden Acylierungsmittel umsetzt, oder

b) eine Verbindung der allgemeinen Formel

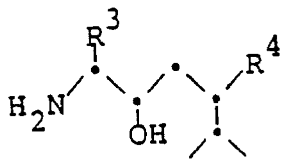

III

worin $R^3$ und $R^4$ die in Anspruch 1 angegebene Bedeutung besitzen,
mit einer Verbindung der allgemeinen Formel

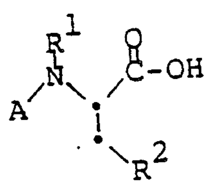

IV

worin $R^1$, $R^2$ und A die in Anspruch 1 angegebene Bedeutung besitzen,
oder einem aktivierten Derivat davon umsetzt, oder

c) zur Herstellung einer Verbindung der Formel I, worin $R^4$ Aethyl bedeutet und die übrigen Symbole die in Anspruch 1 angegebene Bedeutung besitzen, eine Verbindung der Formel I, worin $R^4$ Vinyl bedeutet und die übrigen Symbole die in Anspruch 1 angegebene Bedeutung besitzen, hydriert, oder

d) zur Herstellung einer Verbindung der Formel I, worin $R^4$ 1,2-Dihydroxyäthyl bedeutet und die übrigen Symbole die in Anspruch 1 angegebene Bedeutung besitzen, eine Verbindung der Formel I, worin $R^4$ Vinyl bedeutet und die übrigen Symbole die in Anspruch 1 angegebene Bedeutung besitzen, oxidiert, oder

e) zur Herstellung einer Verbindung der Formel I, worin A eine freie Aminogruppe enthält, aus einer entsprechenden Verbindung der Formel I, in der A eine N-geschützte Aminogruppe enthält, die N-Schutzgruppe abspaltet, und/oder

f) erwünschtenfalls ein Gemisch diastereomerer Racemate in die diastereomeren Racemate oder optisch reinen Diastereomeren auftrennt, und/oder

g) erwünschtenfalls ein Diastereomerengemisch in die optisch reinen Diastereomeren auftrennt, und/oder

h) erwünschtenfalls eine erhaltene Verbindung in ein pharmazeutisch verwendbares Salz überführt.

16. Arzneimittel, enthaltend ein Aminosäurederivat gemäss einem der Ansprüche 1-11 und ein therapeutisch inertes Excipiens.

17. Mittel zur Bekämpfung bzw. Verhütung von Bluthockdruck und Herzinsuffizienz, enthaltend ein Aminosäurederivat gemäss einem der Ansprüche 1-11 und ein therapeutisch inertes Excipiens.

18. Verwendung eines Aminosäurederivates gemäss einem der Ansprüche 1-11 bei der Bekämpfung bzw. Verhütung von Krankheiten.

19. Verwendung eines Aminosäurederivates gemäss einem der Ansprüche 1-11 bei der Bekämpfung bzw., Verhütung von Bluthochdruck und Herzinsuffizienz.

20. Verwendung eines Aminosäurederivates gemäss einem der Ansprüche 1-11 zur Herstellung von Mitteln gegen Bluthochdruck und/oder Herzinsuffizienz.

Patentansprüche für folgende Vertragsstaaten: GR, ES

1. Verfahren zur Herstellung von Aminosäurederivaten der allgemeinen Formel

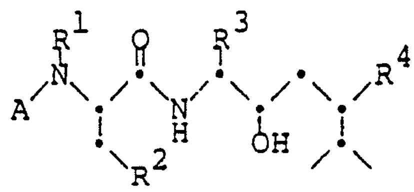

I

worin $R^1$ Wasserstoff oder Methyl, $R^2$ Aethyl, Propyl oder Imidazol-4-yl, $R^3$ Isobutyl, Cyclohexylmethyl oder Benzyl, $R^4$ Phenyl, Furyl, Vinyl, Aethyl oder 1,2-Dihydroxyäthyl und A über die Carboxylgruppe gebundene Dibenzsuberanessigsäure, 2-(2-Pyridyl)benzosäure, ß--Naphthylbernsteinsäuremonoäthylester, 2-Indolylessigsäure oder Dihydrozimtsäure oder eine der Gruppen

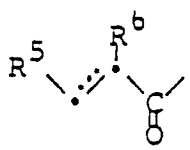

und

-Y-Z

(a) (b)

bedeuten, worin die gestrichelte Linie eine zusätzliche Bindung bedeuten kann, $R^5$ Phenyl, Naphthyl, Indolyl, Pyrazolyl, Imidazolyl oder Pyridylmethyl und $R^6$ Wasserstoff, Alkyl, Arylalkyl, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl,Aminocarbonyl, substituiertes Aminocarbonyl, Alkoxycarbonyl, Alkylcarbonyloxy, Arylalkylcarbonyloxy, Alkylaminocarbonyloxy, Alkoxycarbonylamino, Cyan oder 2,5-Dimethylpyrrol-1-yl bedeuten, mit der Massgabe dass $R^4$ nicht Alkoxycarbonylamino bedeuten kann, wenn $R^3$ Phenyl oder α-Naphthyl bedeutet, und Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, α-Naphthylalanin, Homophenylalanin, Asparaginsäureäthylester, Glutaminsäure-t-butylester oder Glutaminsäurebenzylester und Z Wasserstoff, Acyl oder eine der Gruppen

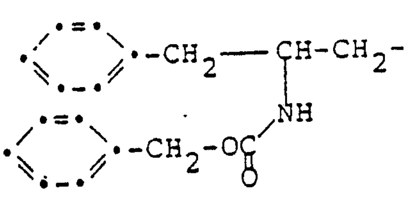

(c)

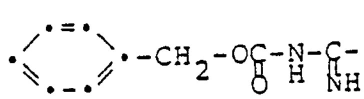

(d)

und

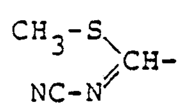

(e)

bedeuten,
in Form von optisch reinen Diastereomeren, Diastereomerengemischen, diastereomeren Racematen oder Gemischen von diastereomeren Racematen sowie von pharmazeutisch verwendbaren Salzen dieser Verbindungen, dadurch gekennzeichnet, dass man

a) eine Verbindung der allgemeinen Formel

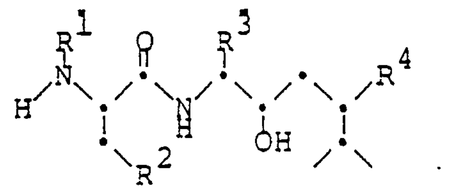

II

worin $R^1$, $R^2$, $R^3$ und $R^4$ die oben angegebene Bedeutung besitzen, mit einem die Gruppe

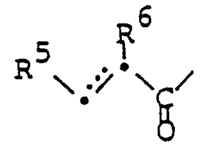

oder

-Y-Z

(a) (b)

worin $R^5$, $R^6$, Y, Z und die gestrichelte Linie die oben angegebene Bedeutung besitzen, abgebenden Acylierungsmittel umsetzt, oder

b) eine Verbindung der allgemeinen Formel

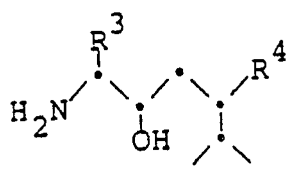

III

worin $R^3$ und $R^4$ die oben angegebene Bedeutung besitzen, mit einer Verbindung der allgemeinen Formel

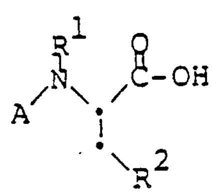

IV

worin $R^1$, $R^2$ und A die oben angegebene Bedeutung besitzen, oder einem aktivierten Derivat davon umsetzt, oder

c) zur Herstellung einer Verbindung der Formel I, worin $R^4$ Aethyl bedeutet und die übrigen Symbole die oben angegebene Bedeutung besitzen, eine Verbindung der Formel I, worin $R^4$ Vinyl bedeutet und die übrigen Symbole die oben angegebene Bedeutung besitzen, hydriert, oder

d) zur Herstellung einer Verbindung der Formel I, worin $R^4$ 1,2-Dihydroxyäthyl bedeutet und die übrigen Symbole die oben angegebene Bedeutung besitzen, eine Verbindung der Formel I, worin $R^4$ Vinyl bedeutet und die übrigen Symbole die oben angegebene Bedeutung besitzen, oxidiert, oder

e) zur Herstellung einer Verbindung der Formel I, worin A eine freie Aminogruppe enthält, aus einer entsprechenden Verbindung der Formel I, in der A eine N-geschützte Aminogruppe enthält, die N-Schutzgruppe abspaltet, und/oder

f) erwünschtenfalls ein Gemisch diastereomerer Racemate in die diastereomeren Racemate oder optisch reinen Diastereomeren auftrennt, und/oder

g) erwünschtenfalls ein Diastereomerengemisch in die optisch reinen Diastereomeren auftrennt, und/oder

h) erwünschtenfalls eine erhaltene Verbindung in ein pharmazeutisch verwendbares Salz überführt.

2. Verfahren gemäss Anspruch 1, worin $R^1$ Wasserstoff, $R^2$ Imidazol-4-yl, $R^3$ Cyclohexylmethyl und $R^4$ Vinyl oder Aethyl bedeuten.

3. Verfahren gemäss Anspruch 1 oder 2, worin A die Gruppe (a) oder (b) bedeutet, worin $R^5$ Phenyl oder Naphthyl, $R^6$ Alkylcarbonylalkyl, Cycloalkylcarbonylalkyl, Heterocycloalkylcarbonylalkyl, Aminocarbonylalkyl oder substituiertes Aminocarbonylalkyl, Y Phenylalanin, Cyclohexylalanin, 4-Chlorphenylalanin oder α-Naphthylalanin und Z Acyl bedeuten.

4. Verfahren gemäss Anspruch 3, worin $R^5$ Phenyl, $R^6$ $C_1$-$C_4$-Alkylcarbonylmethyl, $C_5$-$C_6$-Cycloalkylcarbonylmethyl, N-t-Butoxycarbonylpyrrolidinylcarbonylmethyl oder Aminocarbonylalkyl, welches durch $C_1$-$C_4$-Alkyl oder gegebenenfalls durch ein Sauerstoffatom unterbrochenes $C_4$-$C_5$-Alkylen disubstituiert oder durch Alkoxycarbonylalkyl oder Alkoxycarbonyl mono- oder di-substituiertes Aminoalkyl monosubstituiert ist und Y Phenylalanin bedeuten und Z den C-terminal mit Y verbundenen Rest einer N- und/oder α-methylierten, gegebenenfalls durch die Gruppe $R^b$-CO- oder $R^a$-O-CO- N-substituierten, natürlichen Aminosäure mit der L-Konfiguration oder eines Epimeren einer solchen Aminosäure mit der D-Konfiguration oder eines Dipeptids aus zwei solchen Aminosäuren oder die Gruppe $R^b$-CO- oder $R^a$-O-CO- bedeutet, worin $R^a$ einen gegebenenfalls substituierten, gesättigten oder ungesättigten aliphatischen, cycloaliphatischen, cycloaliphatisch-aliphatischen Kohlenwasserstoffrest mit bis zu 18 Kohlenstoffatomen, einen gegebenenfalls substituierten aromatischen, heteraromatischen, aromatisch-aliphatischen oder heteroaromatisch-aliphatischen Kohlenwasserstoffrest mit bis zu 18 Kohlenstoffatomen oder einen gegebenenfalls substituierten, gesättigten 5- oder 6-gliedrigen Heterocyclus bedeutet und $R^b$ Wasserstoff bedeutet oder die Bedeutung von $R^a$ besitzt, vorzugsweise den C-terminal mit Y verbundenen Rest von Prolin, Prolylprolin oder Histidinylprolin bedeutet, wobei die Aminogruppe jeweils durch t-Butoxycarbonyl, Benzoxycarbonyl, Isovaleryl oder die Gruppe $R^b$-CO- substituiert ist, worin $R^b$ einen heteroaromatisch-aliphatischen Kohlenwasserstoffrest mit bis zu 10 Kohlenwasserstoffatomen oder einen gesättigten 5- oder 6-gliedrigen Heterocyclus bedeutet.

5. Verfahren gemäss einem der Ansprüche 1-4, worin $R^1$ Wasserstoff, $R^2$ Imidazol-4-yl, $R^3$ Cyclohexylmethyl, $R^4$ Vinyl oder Aethyl und $R^5$ Phenyl und $R^6$ $C_1$-$C_4$-Alkylcarbonylmethyl, $C_5$-$C_6$-Cycloalkylcarbonylmethyl, N-t-Butoxycarbonylpyrrolidinylcarbonylmethyl oder Aminocarbonylalkyl, welches durch $C_1$-$C_4$-Alkyl oder gegebenenfalls durch ein Sauerstoffatom unterbrochenes $C_4$-$C_5$-Alkylen disubstituiert oder durch Alkoxycarbonylalkyl oder Alkoxycarbonyl mono- oder di-substituiertes Aminoalkyl monosubstituiert ist, oder Y den N-terminal mit Z verbundenen, bivalenten Rest von Phenylalanin und Z den C-terminal mit Y verbundenen Rest von Prolin, Prolylprolin oder Histidinylprolin bedeuten, wobei die Aminogruppe jeweils durch t-Butoxycarbonyl, Benzoxycarbonyl, Isovaleryl oder die Gruppe $R^b$-CO- substituiert ist, worin $R^b$ einen heteroaromatisch-aliphatischen Kohlenwasserstoffrest mit bis zu 10 Kohlenstoffatomen oder einen gesättigten 5- oder 6-gliedrigen Heterocyclus bedeutet.

6. Verfahren gemäss Anspruch 1, dadurch gekennzeichnet, dass man t-Butyl (R)-2-[[(S)-α-[[(S)-1-[(1S,2S,4S)-1-(cyclohexylmethyl-2-hydroxy-4-isopropylhexyl]-2-imidazol-4-yläthyl]carbamoyl]phenäthyl]-carbamoyl]-1-pyrrolidincarboxylat herstellt.

7. Verfahren gemäss Anspruch 1, dadurch gekennzeichnet, dass man (2S,3S,5S)-2-(Boc-D-Pro-Phe-His-NH)-1-cyclohexyl-5-isopropyl-6-hepten-3-ol herstellt.

8. Verfahren gemäss Anspruch 1, dadurch gekennzeichnet, dass man t-Butyl (S)-2-[[(R)-α-[[(S)-1-[[(1S,2S,4S)-1-(cyclohexylmethyl)-2-hydroxy-4-isopropylhexyl]carbamoyl]-2-imidazol-4-yläthyl]carbamoyl]-phenäthyl]acetyl]-1-pyrrolidincarboxylat herstellt.

9. Verfahren gemäss Anspruch 1, dadurch gekennzeichnet, dass man N-(S)-1-[[(1S,2S,4S)-1-(Cyclohexylmethyl)-2-hydro xy-4-isopropylhexyl]carbamoyl]-2-imidazol-4-yläthyl]-γ-oxo-α-(1-naphthylmethyl)-4-morpholinbutyramid herstellt.

10. Verfahren gemäss Anspruch 1, dadurch gekennzeichnet, dass man (2S,5S)-2-(Boc-D-Pro-Pro-Phe-His-NH)-1-cyclohexyl-5-isopropyl-6-hepten-3-ol herstellt.

11. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der in Anspruch 1 angegebenen Formel I in Form eines optisch reinen Diastereomeren, Diastereomerengemisches, diastereomeren Reacemates oder Gemisches von diastereomeren Racematen oder ein pharmazeutisch verwendbares Salz davon in eine galenische Darreichungsform bringt.

12. Verwendung eines Aminosäurederivates der in Anspruch 1 angegebenen Formel I in Form eines optisch reinen Diastereomeren, Diastereomerengemisches, diastereomeren Reacemates oder Gemisches von diastereomeren Racematen oder eines pharmazeutisch verwendbaren Salzes davon zur Herstellung von Mitteln gegen Bluthochdruck und/oder Herzinsuffizienz.

13. Verbindungen der allgemeinen Formeln

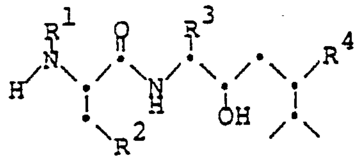 II, 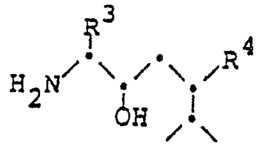 III und 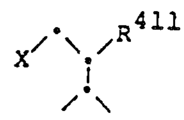 VI'

worin $R^1$ Wasserstoff oder Methyl, $R^2$ Aethyl, Propyl oder Imidazol-4-yl, $R^3$ Isobutyl, Cyclohexylmethyl oder Benzyl, $R^4$ Phenyl, Furyl, Vinyl, Aethyl oder 1,2-Dihydroxyäthyl, X Chlor, Brom oder Jod und $R^{411}$ Phenyl oder Furyl bedeuten.